# EUROPEAN PATENT APPLICATION

(11) **EP 2 135 960 A1**
(43) Date of publication of application: **23.12.2009**
(21) Application number: 09170787.7
(22) Date of filing: 19.09.2002
(51) Int. Cl.: C12Q 1/68

(54) **Genetic analysis for stratification of cancer risk by determining the allelic profile of the VDR-ApaI and CYP11B2 genes**

(30) Priority: 19.09.2001 US 323510 P
(62) Divisional of application: 02773461.5
(71) Applicant: Intergenetics Incorporated, Oklahoma City, OK 73104 (US); Oklahoma Medical Research Foundation, Oklahoma City, OK 73104 (US)
(72) Inventor: Ralph, David, Edmund, OK 73034 (US); Aston, Christopher, Edmond, OK 73003 (US); Jupe, Eldon, Norman, OK 73071 (US)
(74) Representative: Dehmel, Albrecht

(57) **Abstract**

The present invention provides a method for (i) assessing a female subject's risk for developing breast cancer, (ii) determining the need for routine diagnostic testing of a female subject for breast cancer, or (iii) determining the need of a female subject for prophylactic anti-breast cancer therapy, the method comprising the step of determining, in a sample from said subject, the allelic profile of the VDR-ApaI and CYP11B2 genes.

## Description

### BACKGROUND OF THE INVENTION

### 1. Field of the Invention

The present invention relates generally to the fields of oncology and genetics. More particularly, it concerns use of a multivariate analysis of genetic alleles to determine which combinations of alleles are associated with low, intermediate and high risk of particular cancers. These risk alleles, when used in combination to screen patient samples, provide a means to direct patients towards their most effective prediagnostic cancer risk management. This provides a method for evaluation of incremental and lifetime risk of developing cancer.

### 2. Description of Related Art

For patients with cancer, early diagnosis and treatment are the keys to better outcomes. In 2001, there are expected to be 1.25 million persons diagnosed with cancer in the US. Tragically, in 2001, over 550,000 people are expected to die of cancer. To a very large extent, the difference between life and death for a cancer patient is determined by the stage of the cancer when the disease is first detected and treated. For those patients whose tumors are detected when they are relatively small and confined, the outcomes are usually very good. Conversely, if a patient's cancer has spread from its organ of origin to distant sites throughout the body, the patient's prognosis is very poor regardless of treatment. The problem is that tumors that are small and confined usually do not cause symptoms. Therefore, to detect these early stage cancers, it is necessary to screen or examine people without symptoms of illness. In such apparently healthy people, cancers are actually quite rare. Therefore it is necessary to screen a large number of people to detect a small number of cancers. As a result, cancer-screening tests are relatively expensive to administer in terms of the number of cancers detected per unit of healthcare expenditure.

A related problem in cancer screening is derived from the reality that no screening test is completely accurate. All tests deliver, at some rate, results that are either falsely positive (indicate that there is cancer when there is no cancer present) or falsely negative (indicate that no cancer is present when there really is a tumor present). Falsely positive cancer screening test results create needless healthcare costs because such results demand that patients receive follow-up examinations, frequently including biopsies, to confirm that a cancer is actually present. For each falsely positive result, the costs of such follow-up examinations are typically many times the costs of the original cancer-screening test. In addition, there are intangible or indirect costs associated with falsely positive screening test results derived from patient discomfort, anxiety and lost productivity. Falsely negative results also have associated costs. Obviously, a falsely negative result puts a patient at higher risk of dying of cancer by delaying treatment. To counter this effect, it might be reasonable to increase the rate at which patients are repeatedly screened for cancer. This, however, would add direct costs of screening and indirect costs from additional falsely positive results. In reality, the decision on whether or not to offer a cancer screening test hinges on a cost-benefit analysis in which the benefits of early detection and treatment are weighed against the costs of administering the screening tests to a largely disease free population and the associated costs of falsely positive results.

Another related problem concerns the use of chemopreventative drugs for cancer. Basically, chemopreventatives are drugs that are administered to prevent a patient from developing cancer. While some chemopreventative drugs may be effective, such drugs are not appropriate for all persons because the drugs have associated costs and possible adverse side effects (Reddy & Chow, 2000). Some of these adverse side effects may be life threatening. Therefore, decisions on whether to administer chemopreventative drugs are also based on a cost-benefit analysis. The central question is whether the benefits of reduced cancer risk outweigh the costs and associated risks of the chemopreventative treatment.

Currently, an individual's age is the most important factor in determining if a particular cancer-screening test should be offered to a patient. Truly, cancer is a rare disease in the young and a fairly common ailment in the elderly. The problem arises in screening and preventing cancers in the middle years of life when cancer can have its greatest negative impact on life expectancy and productivity. In the middle years of life, cancer is still fairly uncommon. Therefore, the costs of cancer screening and prevention can still be very high relative to the number of cancers that are detected or prevented. Decisions on when to begin screening also may be influenced by personal history or family history measures. Unfortunately, appropriate informatic tools to support such decision making are not yet available for most cancers.

A common strategy to increase the effectiveness and economic efficiency of cancer screening and chemoprevention in the middle years of life is to stratify individuals' cancer risk and focus the delivery of screening and prevention resources on the high-risk segments of the population. Two such tools to stratify risk for breast cancer are termed the Gail Model and the Claus Model (Costantino *et al.,* 1999, McTiernan *et al.,* 2001). The Gail model is used as the "Breast Cancer Risk-Assessment Tool" software provided by the National Cancer Institute of the National Institutes of Health on their web site. Neither of these breast cancer models utilize genetic markers as part of their inputs. Furthermore, while both models are steps in the right direction, neither the Claus nor Gail models have the desired predictive power or discriminatory accuracy to truly optimize the delivery of breast cancer screening or chemopreventative therapies.

These issues and problems could be reduced in scope or even eliminated if it were possible to stratify or differentiate a given individual's risk from cancer more accurately than is now possible. If a precise measure of actual risk could be accurately determined, it would be possible to concentrate cancer screening and chemopreventative efforts in that segment of the population that is at highest risk. With accurate stratification of risk and concentration of effort in the high-risk population, fewer screening tests would be required to detect a greater number of cancers at an earlier and more treatable stage. Fewer screening tests would mean lower test administrative costs and fewer falsely positive results. A greater number of cancers detected would mean a greater net benefit to patients and other concerned parties such as health care providers. Similarly, chemopreventative drugs would have a greater positive impact by focussing the administration of these drugs to a population that receives the greatest net benefit.

### SUMMARY OF THE INVENTION

Thus, in accordance with the present invention, there is provided a method for assessing a female subject's risk for developing breast cancer comprising determining, in a sample from the subject, the allelic profile of two or more genes selected from the group consisting of prohibitin (PRO), progesterone receptor (PROGINS), steroid 17,20 lyase (CYP17), catechol o-methyltransferase (COMT), epidermal growth factor receptor 2 (HER2), 5α-reductase (SRD5α), glutathione S-transferase P1 (GSTP1), phenol sulphotransferase (SULF1A1), cytochrome p450-1B1 (CYP1B1), tumor suppressor p53 (p53 72), methylenetetrahydrofolate reductase (MTHFR), vitamin D receptor Apa I polymorphism (VDR/ApaI), vitamin D receptor Taq^{α}I polymorphism (VDR/TaqI), vitamin D receptor Fok I polymorphism (VDR/FokI), cytochrome P450 1A1 (CYP1A1), human aldosterone synthase or steroid 18-hydroxylase (CYP11B2), cyclin D1 (CYC D1), homo sapiens DNA repair protein (XRCC 1), human cytochrome P450IIE1 (ethanol-inducable) (CYP2E1), and microsomal epoxide hydrolase (EPHX).

In a more particular embodiment, a gene pair selected from the group consisting of Pro and CYP17; Pro and COMT; Pro and GSTP1; Pro and SULF1A1; Pro and HER2; Pro and p53 72; Pro and CYP1B1; Pro and PROGINS; Pro and SRD5A2_2; Pro and MTHFR; Pro and VDR/ApaI; Pro and VDR/TaqI; Pro and VDR/FokI; Pro and CYP2E1; Pro and EPHX; Pro and CYP11B2; Pro and CYC D1; Pro and XRCC1; CYP17 and COMT; CYP17 and GSTP1; CYP17 and SULF1A1; CYP17 and HER2; CYP17 and p53 72; CYP17 and CYP1B1; CYP17 and PROGINS; CYP17 and SRD5A2_2; CYP17 and MTHFR; CYP17 and VDR/ApaI; CYP17 and VDR/TaqI; CYP17 and VDR/FokI; CYP17 and CYP2E1; CYP17 and EPHX; CYP17 and CYP1A1; CYP17 and CYP11B2; CYP17 and CYC D1; CYP17 and XRCC1; COMT and GSTP1; COMT and SULF1A1; COMT and HER2; COMT and p53 72; COMT and CYP1B1; COMT and PROGINS; COMT and SRD5A2_2; COMT and MTHFR; COMT and VDR/ApaI; COMT and VDR/TaqI; COMT and VDR/FokI; COMT and CYP2E1; COMT and EPHX; COMT and CYP1A1; COMT and CYP11B2; COMT and CYC D1; COMT and XRCC 1; GSTP1 and SULF1A1; GSTP1 and HER2; GSTP1 and p53 72; GSTP1 and CYP1B1; GSTP1 and PROGINS; GSTP1 and SRD5A2_2; GSTP1 and MTHFR; GSTP1 and VDR/ApaI; GSTP1 and VDR/TaqI; GSTP1 and VDR/FokI; GSTP1 and CYP2E1; GSTP1 and EPHX; GSTP1 and CYP1A1; GSTP1 and CYP11B2; GSTP1 and CYC D1; GSTP1 and XRCC1; SULF1A1 and HER2; SULF1A1 and p53 72; SULF1A1 and CYP1B1; SULF1A1 and PROGINS; SULF1A1 and SRD5A2_2; SULF1A1 and MTHFR; SULF1A1 and VDR/ApaI; SULF1A1 and VDR/TaqI; SULF1A1 and VDR/FokI; SULF1A1 and CYP2E1; SULF1A1 and EPHX; SULF1A1 and CYP1A1; SULF1A1 and CYP11B2; SULF1A1 and CYC D1; SULF1A1 and XRCC 1; HER2 and p53 72; HER2 and CYP1B1; HER2 and PROGINS; HER2 and SRD5A2_2; HER2 and MTHFR; HER2 and VDR/ApaI; HER2 and VDR/TaqI; HER2 and VDR/FokI; HER2 and CYP2E1; HER2 and EPHX; HER2 and CYP1A1; HER2 and CYP11B2; HER2 and CYC D1; HER2 and XRCC 1; p53 72 and CYP1B1; p53 72 and PROGINS; p53 72 and SRDA2_2; p53 72 and MTHFR; p53 72 and VDR/ApaI; p53 72 and VDR/TaqI; p53 72 and VDR/FokI; p53 72 and CYP2E1; p53 72 and EPHX; p53 72 and CYP1A1; p 53 72 and CYP11B2; p53 72 and CYC D1; p53 72 and XRCC1; CYP1B1 and PROGINS; CYP1B1 and SRD5A2_2; CYP1B1 and MTHFR; CYP1B1 and VDR/ApaI; CYP1B1 and VDR/TaqI; CYP1B1 and VDR/FokI; CYP1B1 and CYP2E1; CYP1B1 and EPHX; CYP1B1 and CYP1A1; CYP1B1 and CYP11B2; CYP1B1 and CYC D1; CYP1B1 and XRCC 1; PROGINS and SRD5A2_2; PROGINS and MTHFR; PROGINS and VDR/ApaI; PROGINS and VDR/TaqI; PROGINS and VDR/FokI; PROGINS and CYP2E1; PROGINS and EPHX; PROGINS and CYP1A1; PROGINS and CYP11B2; PROGINS and CYC D1; PROGINS and XRCC 1; SRD5A2_2 and MTHFR; SRD5A2_2 and VDR/ApaI; SRD5A2_2 and VDR/TaqI; SRD5A2_2 and VDR/FokI; SRD5A2_2 and CYP2E1; SRD5A2_2 and EPHX; SRD5A2_2 and CYP1A1; SRD5A2_2 and CYP11B2; SRD5A2_2 and CYC D1;SRD5A2_2 and XRCC 1; MTHFR and VDR/ApaI; MTHFR and VDR/TaqI; MTHFR and VDR/FokI; MTHFR and CYP2E1; MTHFR and EPHX; MTHFR and CYP1A1; MTHFR and CYP11B2; MTHFR and CYC D1; MTHFR and XRCC1; VDR/ApaI and VDR/TaqI; VDR/ApaI and VDR/FokI; VDR/ApaI and CYP2E1; VDR/ApaI and EPHX; VDR/ApaI and CYP1A1; VDR/ApaI and CYP11B2; VDR/ApaI and CYC D1; VDR/ApaI and XRCC 1; VDR/TaqI and VDR/FokI; VDR/TaqI and CYP2E1; VDR/TaqI and EPHX; VDR/TaqI and CYP1A1; VDR/TaqI and CYP11B2; VDR/TaqI and CYC D1; VDR/Taq1 and XRCC 1; VDR/FokI and CYP2E1; VDR/FokI and EPHX; VDR/FokI and CYP1A1; VDR/FokI and CYP11B2; VDR/FokI and CYC D1; VDR/FokI and XRCC 1; CYP2E1 and EPHX; CYP2E1 and CYP1A1; CYP2E1 and CYP11B2; CYP2E1 and CYC D1; CYP2E1 and XRCC 1; EPHX and CYP1A1; EPHX and CYP11B2; EPHX and CYC D1; EPHX and XRCC 1; CYP1A1 and CYP11B2; CYP1A1 and CYC D1; CYP1A1 and XRCC 1; CYP11B2 and CYC D1; CYP11B2 and XRCC 1; CYC D1 and XRCC 1; and p52 72 and CYC D1 may be examined.

The method may further comprise determining the allelic profile of at least a third or fourth gene. Specifically, each of the foregoing two gene combinations may be combined with one or two of the remaining 18 single genes.

The method may further comprise assessing one or more aspects of the subject's personal history, such as age, ethnicity, reproductive history, menstruation history, use of oral contraceptives, body mass index, alcohol consumption history, smoking history, exercise history, diet, family history of breast cancer or other cancer including the age of the reltive at the time of their cancer diagnosis, and a personal history of breast cancer, breast biopsy or DCIS, LCIS, or atypical hyperplasia.

In a particular embodiment, the subject is stratified by age, specifically below age 54. In a parallel embodiment, the subject is stratified by age 54 or greater.

The method may comprise determining the allelic profile by amplification of nucleic acid from the sample, for example, using PCR. Primers for amplification may be located on a chip. Such primers may be specific for alleles of said genes. The method may further comprise cleaving amplified nucleic acid. The sample may be derived from oral tissue or blood. The method may further comprise making a decision on the timing and/or frequency of cancer diagnostic testing for the subject. The method may further comprise making a decision on the timing and/or frequency of prophylactic cancer treatment for the subject.

The specific alleles examined may be are either a C or T at base 729 (GB# U49725) for Pro, either plus or minus an Alu insert at base 956 (GB# Z49816) for PROGINS, either a T or C at base 1805 (GB# M19489) for CYP17, either a G or A at position 1947 (GB# Z26491) for COMT, either a G or A at base 77,829 (GB# AC040933) for HER2, either an 18 or 38 insert at base 2333 (GB# L03843) for SRD5α, either a G or A at base 2628 (GB# M24485) for GSTP1, either a G or A at base 4742 (GB# U54701) for SULF1A1, either a G or C at base 1294 (GB# U56438)for CYP1B1, either a G or C at base 640 (GB# AF136270) for p53, either a C or T at base 1024 (GB# AH007464) for MTHFR, either a C or T at base 46,083 (GB# AC004466) for VDR/ApaI, a polymorphism at base 46,360-46,363 (GB# AC004466) for VDR/TaqI, a polymorphism at base 12,019-12024 (GB# AC004466) for VDR/FokI, either a C or T at base 133 (GB# D12525) for CYP1A1, either a C or T at the Hae III Site (GGCC) at base 335-338 (GB# D13752) for CYP11B2, either a G or A at codon 330 in exon 4 at base 8429 (GB# AF511593) for CYC D1, either a G or A at codon 399, exon 10 at base 28152 (GB# L34079) for XRCC 1, Intron 6 Dra I polymorphism at base 10454-10459 for CYP2E1, and a Tyr/His change in exon 3 (GB# AF253417) for EPHX.

In a separate embodiment, there is provided a nucleic acid microarray comprising nucleic acid sequences corresponding to genes for prohibitin (Pro), progesterone receptor (PROGINS), steroid 17,20 lyase (CYP17), catechol o-methyltransferase (COMT), epidermal growth factor receptor 2 (HER2), 5α-reductase (SRD5α), glutathione S-transferase P1 (GSTP1), phenol sulphotransferase (SULF1A1), cytochrome p450-1B1 (CYP1B1), tumor suppressor p53 (p53 72), methylenetetrahydrofolate reductase (MTHFR), vitamin D receptor Apa I polymorphism (VDR/ApaI), vitamin D receptor Taq^{α}I polymorphism (VDR/TaqI), vitamin D receptor Fok I polymorphism (VDR/FokI), cytochrome P450 1A1 (CYP1A1), human aldosterone synthase or steroid 18-hydroxylase (CYP11B2), cyclin D1 (CYC D1), homo sapiens DNA repair protein (XRCC 1), human cytochrome P450IIE1 (ethanol-inducable) (CYP2E1), and microsomal epoxide hydrolase (EPHX). The nucleic acid sequences may comprise sequence for at least two different alleles for each of the genes.

In yet another embodiment, there is provided a method for determining the need for routine diagnostic testing of a female subject for breast cancer comprising determining, in a sample from the subject, the allelic profile of two or more genes selected from the group consisting of prohibitin (Pro), progesterone receptor (PROGINS), steroid 17,20 lyase (CYP17), catechol o-methyltransferase (COMT), epidermal growth factor receptor 2 (HER2), 5α-reductase (SRD5α), glutathione S-transferase P1 (GSTP1), phenol sulphotransferase (SULF1A1), cytochrome p450-1B1 (CYP1B1), tumor suppressor p53 (p53 72), methylenetetrahydrofolate reductase (MTHFR), vitamin D receptor Apa I polymorphism (VDR/ApaI), vitamin D receptor Taq^{α}I polymorphism (VDR/TaqI), vitamin D receptor Fok I polymorphism (VDR/FokI), cytochrome P450 1A1 (CYP1A1), human aldosterone synthase or steroid 18-hydroxylase (CYP11B2), cyclin D1 (CYC D1), homo sapiens DNA repair protein (XRCC 1), human cytochrome P450IIE1 (ethanol-inducable) (CYP2E1), and microsomal epoxide hydrolase (EPHX). Each of the preceeding specific embodiments may be applied here as well.

In still yet another embodiment, there is provided a method for determining the need of a female subject for prophylactic anti-breast cancer therapy comprising determining, in a sample from the subject, the allelic profile of two or more genes selected from the group consisting of prohibitin (Pro), progesterone receptor (PROGINS), steroid 17,20 lyase (CYP17), catechol o-methyltransferase (COMT), epidermal growth factor receptor 2 (HER2), 5α-reductase (SRD5α), glutathione S-transferase P1 (GSTP1), phenol sulphotransferase (SULF1A1), cytochrome p450-1B1 (CYP1B1), tumor suppressor p53 (p53 72), methylenetetrahydrofolate reductase (MTHFR), vitamin D receptor Apa I polymorphism (VDR/ApaI), vitamin D receptor Taq^{α}I polymorphism (VDR/TaqI), vitamin D receptor Fok I polymorphism (VDR/FokI), cytochrome P450 1A1 (CYP1A1), human aldosterone synthase or steroid 18-hydroxylase (CYP11B2), cyclin D1 (CYC D1), homo sapiens DNA repair protein (XRCC 1), human cytochrome P450IIE1 (ethanol-inducable) (CYP2E1), and microsomal epoxide hydrolase (EPHX).

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1** **- Predicted Actual Distribution of Breast Cancer Risk in All Women.** Median risk is less than mean risk because relatively uncommon high risk groups skew average risk towards the high risk categories.
**FIG. 2** **- Stratification of Risk Directs Choices of Medical Protocols for All Women.** Breast cancer screening and chemoprevention protocols would vary and be offered to women according to risk appropriate criteria.

### DESCRIPTION OF ILLUSTRATIVE EMBODIMENTS

Despite considerable progress in cancer therapy, cancer mortality rates continue to be high. Generally, the poor prognosis of many cancer patients derives from the failure to identify the disease at an early stage, *i.e.,* before metastasis has occurred. While not trivial, treatment of organ confined primary tumors is far more likely to be successful than any treatment for advanced, disseminated malignancies.

In order to effect early diagnosis of cancer, at a time when patients still appear healthy, it is necessary to screen large numbers of individuals. However, the costs associated with such testing, and the unnecessary follow ups occasioned by false positive results, are prohibitive. Thus, it is necessary to find better ways of assessing cancer risk in the general population.

### I. The Present Invention

In accordance with the present invention, the inventors have identified combinations of alleles for Single Nucleotide Polymorphisms (SNPs) and other genetic variations that are associated with varying levels of risk for a diagnosis of breast cancer. A SNP is the smallest unit of genetic variation. It represents a position in a genome where individuals of the same species may have different nucleotides inserted into their DNA sequences. It could be said that our genes make us human, but our SNPs make us unique individuals. An allele is a particular variant of a gene. For example, some individuals may have the DNA sequence, AAGT**C**CG, in some arbitrary gene. Other individuals may have the sequence, AAGT**T**CG, at the same position in the same gene. Notice that these DNA sequences are the same except at the underlined position where some people have a "C" nucleotide while others have a "T" nucleotide. This is the site of a SNP. It is said that some people carry the C allele of this SNP, while others carry the T allele.

Except for those genes on the sex chromosomes and on the mitochondrial genome, there are two copies of every gene in every cell in the body. A child inherits one copy of each gene from each parent. A person could have two C alleles of the fictitious SNP described above. This person would carry the genotype C/C at this SNP. Alternatively, a person could have the genotype T/T at this SNP. As in both of these examples, if someone carries two identical copies of a portion of a potentially variant portion of a gene, they are referred to as homozygous for this gene or portion of a gene. Obviously, some people will carry two different alleles of this gene having the genotype, C/T or T/C, and will be termed heterozygous for this SNP. Lastly, some genetic variation may involve more than one nucleotide position. Common examples of such variation, and ones that are relevant to this invention, are polymorphisms where there have been insertions or deletions of one or more nucleotides in one allele of a gene relative to the alternative allele(s).

In addition to genetic variation, the inventors have examined the interaction between age and genetic variation to better estimate risk of breast cancer. They have also begun to examine ethnic affiliation and family history of cancer as additional variables to better estimate breast cancer risk. Age, gender, ethnic affiliation and family medical history are all examples of personal history measures. Other examples of personal history measures include reproductive history, menstruation history, use of oral contraceptives, body mass index, smoking and alcohol consumption history, and exercise and diet.

In the experiments disclosed herein, the inventors report the examination of alleles of 20 genetic polymorphisms. Polymorphisms were assayed by standard techniques to detect Restriction Fragment Length Polymorphisms (RFLPs) or simple length polymorphisms in gene specific PCR products. All of the polymorphisms examined have been described previously in the peer reviewed scientific literature. In fact, all of the polymorphisms examined have previously had at least some association with cancer risk.

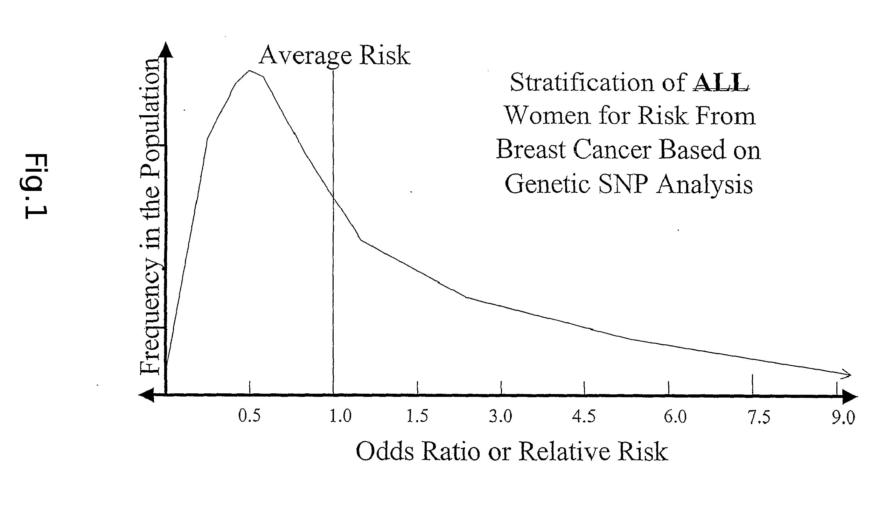

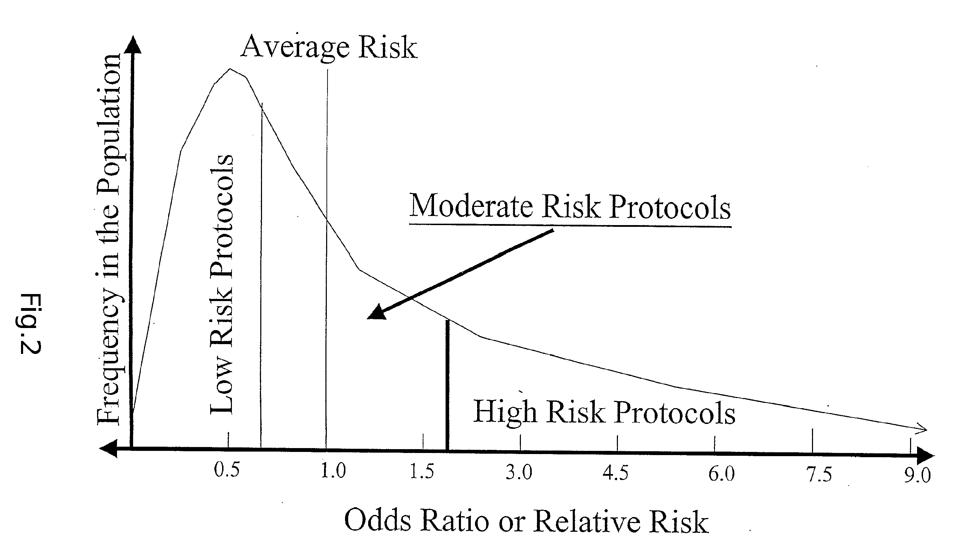

The inventors' hypothesis was that by examining these polymorphisms in combination, one would find combinations that were much more informative for predicting cancer risk than could have been predicted by examining each gene polymorphism separately. In fact, it now has been determined that there are combinations of alleles for certain polymorphisms (SNPs and insertion/deletion polymorphisms) that are associated with extraordinary risk of breast cancer. So high is the genetically inherited risk of breast cancer in individuals carrying certain combinations of alleles, that their risk distorts the apparent breast cancer risk in the population at large. Thus, surprisingly, the large majority of women are actually at much less than "average" risk from breast cancer (FIG. 1). Such dramatic findings were unexpected even by the inventors when these experiments were designed. These results provide a means of reallocating breast cancer screening and chemoprevention resources to concentrate on a relatively small portion of the total population at highest risk of breast cancer, thus facilitating better patient outcomes at lower overall healthcare costs (FIG. 2).

### II. Target Genes and Alleles

Table 1 provides a listing of the genes, the specific genetic polymorphisms examined in the present study, and a literature citation. The letters in parentheses are abbreviations for this polymorphisms that will be used throughout the remainder of this text.

**Table 1, List of SNPS**

| **PROGINS, 11q22** | | |
|---|---|---|
| | **Polymorphism:** The polymorphism is the existence or non-existence of a 306-bp Alu insert in intron7. | |
| | **Primary Reference:** | Dunning *et al.,* 1999. |
| | | |

| **SRD5A2,2p23** | | |
|---|---|---|
| reported | **Polymorphism:** The polymorphism appears in the 3'UTR in the form of TA repeats. It is as 0-bp inserts, 18-bp inserts, or 36-bp inserts. | |
| | **Primary Reference:** | Bharaj *et al.,* 2000. |
| | | |

| **COMT**, **22q11.2** | | |
|---|---|---|
| | **Polymorphism:** G > A Polymorphism Val>Met | |
| | **Primary Reference:** | Thompson *et al.,* 1998. |
| | | |

| **CYP17, 10q24.3** | | |
|---|---|---|
| | **Polymorphism:** T > C Polymorphism in the 5'UTR. | |
| | **Primary Reference:** | Feigelson *et al.,* 2001. |
| | | |

| **SULT1A1,16p12.1-p11.2** | | |
|---|---|---|
| | **Polymorphism:** G > A Polymorphism in exon 7 at codon 213, Arg > His. | |
| | **Primary References:** | 1. Zheng *et al.,* 2001. |
| | | 2. Coughtrie *et al.,* 1999. |
| | | |

| **CYP1B1, 2p22-p21** | | |
|---|---|---|
| | **Polymorphism:** A G → C (guanine to cytosine) transition in exon 3 resulting in a Valine → Leucine substitution in codon 432. | |
| | **Primary Reference:** | Zheng *et al.,* 2000. |

| **GSTP1, 11q13** | | |
|---|---|---|
| | **Polymorphism:** A G → A (guanine to adenine) transition resulting in an Isoleucine to Valine substitution in codon 105. | |
| | **Primary Reference:** | Dunning *et al.,* 1999. |

| **MTHFR, 1p36.3** | | |
|---|---|---|
| | **Polymorphism:** C > | T Polymorphism at nucleotide 677. |
| | **Primary Reference:** | Stern *et al.,* 2000. |
| | | |

| **VDR (Apa I), 12q12-q14** | | |
|---|---|---|
| | **Polymorphism:** | Polymorphism in the 3'UTR. |
| | **Primary Reference:** | Curran *et al.,* 1999. |
| | | |

| **VDR (Taq^{α}I), 12q12-q14** | | |
|---|---|---|
| | **Polymorphism:** | Polymorphism in the 3'UTR. |
| | **Primary Reference:** | Curran *et al.,* 1999. |
| | | |

| **VDR (Fok I), 12q12-q14** | | |
|---|---|---|
| | **Polymorphism:** | Polymorphism in the Initiation Codon variant in the 5' end of the gene. |
| | **Primary Reference:** | Curran *et al.,* 1999. |
| | | |

| **CYP1A1, 15q22-q24** | | |
|---|---|---|
| | **Polymorphism:** | Polymorphism at Ile462Val. |
| | **Primary Reference:** | 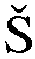 armanová *et al.,* 2001. |
| | | |

| **CYP11B2,8q21** | | |
|---|---|---|
| | **Polymorphism:** The polymorphism is located in the promoter of CYP11B2 344 nucleotides before the start of the coding sequence. It is either a cytosine or thymidine in this position. | |
| | **Primary Reference:** | Kupari *et al.,* 1998. |
| **CycD1, 11q13** | | |
| | **Polymorphism:** A G → A (guanine to adenine) polymorphism at codon 330 in exon 4. | |
| | **Primary Reference:** | Kong *et al.,* 2001. |
| | | |

| **XRCC1, 19q13.2** | | |
|---|---|---|
| | **Polymorphism:** G > A Polymorphism in codon 399, exon 10. | |
| | **Primary Reference:** | Sturgis *et al.,* 1999. |
| | | |

| PROHIBITIN, **17q21** | | |
|---|---|---|
| | **Polymorphism:** C > T Polymorphism on the 3' UTR. | |
| | **Primary Reference:** | Jupe *et al.,* 2001. |
| | | |

| **p53, 17p13.1** | | |
|---|---|---|
| | **Polymorphism:** G > C Polymorphism in codon 72; Arg72Pro. | |
| | **Primary Reference:** | Pierce *et al.,* 2000. |
| | | |

| **HER2, 17q21.1** | | |
|---|---|---|
| | **Polymorphism:** G > A Polymorphism in exon 4 Val>Ile. | |
| | **Primary Reference:** | Wang *et al.,* 2002. |

| **CYP2E1, 10q24.3-qter** | | |
|---|---|---|
| | **Polymorphism:** Intron 6. | |
| | **Primary References:** | 1. 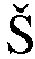 armanová *et al.,* 2001. |
| | | 2. Hirvonen *et al.,* 1993. |
| | | |

| **EPHX** | | |
|---|---|---|
| | **Polymorphism:** Tyr/His change in exon 3. | |
| | **Primary References:** | 1. Hassett *et al.,* 1994a. |
| | | 2. Hassett *et al.,* 1994b. |
| | | 3. Hassett *et al.,* 2000. |

Some of these polymorphisms have been discussed in the literature in depth in perhaps dozens of scientific publications. While the scientific literature suggests that many of these polymorphisms may be associated with very modest changes in cancer risk, or are associated with larger variations in risk within a small subset of the population, many of these polymorphisms are controversial in the scientific literature, with some studies finding no associated change in relative cancer risk. Formally, in genetic terms, these common alleles individually have low penetrance for the breast cancer phenotype, but when occurring together create complex genotypes with very high penetrance for the breast cancer phenotype. The inventors note that their hypothesis for cancer predisposition is consistent with that of a complex multi-gene phenomenon, as has been discussed by others (Lander & Schork, 1994), and correlates well with the long-standing observation that cancers in general, and breast cancer in particular, cluster in some families. However, these particular gene combinations have not previously been identified as being associated with risk of breast or any other cancer.

It should be noted that this is not intended to be an exhaustive list of all polymorphisms that correlate with cancer risk or susceptibility. Other polymorphisms may be found that improve upon this set of breast cancer determinants, and certainly for other cancers, there will be additional marker and marker combinations. This is merely the first set of ten genetic markers for which initial evaluation has been completed.

### III. Sample Obtention and Processing

### A. Sampling

In order to assess the genetic make-up of an individual, it is necessary to obtain a nucleic acid-containing sample. Suitable tissues include almost any nucleic acid containing tissue, but those most convenient include oral tissue or blood. For those DNA specimens isolated from peripheral blood specimens, blood was collected in heparinized syringes or other appropriate vessel following venipuncture with a hypodermic needle. Oral tissue may advantagenously be obtained from a mouth rinse. Oral tissue or buccal cells may be collected with oral rinses, e.g., with "Original Mint" flavor Scope™ mouthwash. Typically, a volunteer participant would vigorously swish 10-15 ml of mouthwash in their mouth for 10-15 seconds. The volunteer would then spit the mouthwash into a 50 ml conical centrifuge tube (for example Fisherbrand disposable centrifuge tubes with plug seal caps (catalog # 05-539-6)) or other appropriate container.

### B. Processing of Nucleic Acids

Genomic DNA was isolated and purified from the samples collected as described below using the PUREGENE™ DNA isolation kit that is manufactured by Gentra Systems of Minneapolis, MN. For the peripheral blood specimens, red blood cells were lysed using the RBC lysis solution provided in the kit. After centrifugation at 2000 X g for 10 minutes the supernatant was discarded and the resulting cell pellet was lysed in a cell lysis solution. The lysate was digested with RNase A and proteins were precipitated. Finally, the genomic DNA was precipitated with isopropanol followed by washing with 70% ethanol. The resulting purified genomic DNA was resuspended in aqueous solution before gene specific PCR and SNP analysis.

In another embodiment, the inventors isolate the large majority of the DNA specimens from buccal cells obtained through the mouthwash procedure. The following is the standard operating procedure (SOP) used to isolate genomic DNA from buccal cells using the Gentra Systems kit.

Genomic DNA is isolated from individual buccal cell samples. Using Polymerase Chain Reaction (PCR) device, target genomic sequences are amplifed. The resulting PCR products are analyzed by gel electrophoresis or by digestion with an appropriate restriction endonuclease followed by gel electrophoresis to obtain a specific genotype for the buccal cell samples.

A number of different materials are used in accordance with the present invention. These include primary solutions used in DNA Extraction (Cell Lysis Solution, Gentra Systems Puregene, and Cat. # D-50K2, 1 Liter; Protein Precipitation Solution, Gentra Systems Puregene, Cat. # D-50K3, 350 ml; DNA Hydration Solution, Gentra Systems Puregene, Cat. # D-500H, 100ml) and secondary solutions used in DNA Extraction (Proteinase K enzyme, Fisher Biotech, Cat. # BP1700, 100mg powder; RNase A enzyme, Amresco, Cat. # 0675, 500mg powder; Glycogen, Fisher Biotech, Cat. # BP676, 5gm powder, 2-propanol (isopropanol), Fisher Scientific, Cat. # A451, 1 Liter; TE Buffer Solution pH 8.0, Amresco, Cat. # E112, 100ml; 95% Ethyl Alcohol, AAPER Alcohol & Chemical Co., 5 Liters).

The exemplified DNA extraction procedure involves five basic steps, as discuss below:
**Preliminary Procedures.** Buccal samples should be processed within 7 days of collection. The DNA is stable in mouthwash at room temperature, but may degrade if left longer than a week before processing.
**Cell Lysis and RNase A Treatment.** Samples are centrifuged (50 ml centrifuge tube containing the buccal cell sample) at 3000 rpm (or 2000 x g) for 10 minutes using a large capacity (holds 20-50 ml or 40-15ml centrifuge tubes) refrigerated centrifuge. Immediately pour off the supernatant into a waste bottle, leaving behind roughly 100 µl of residual liquid and the buccal cell pellet at the bottom of the 50 ml tube. Be aware that loose pellets will result if samples are left too long after centrifugation before discarding the liquid. Vortex (using a Vortex Genie at high speed) for 5 seconds to resuspend the cells in the residual supernatant. This greatly facilitates cell lysis (below). Pipette (use a pipette aide and a 10 ml pipette) 1.5 ml of Cell Lysis Solution into the 50 ml tube to resuspended the cells, and then vortex for 5 seconds to maximize contact between cells and cell lysis solution. (If necessary, new samples may need to be stored longer than a week before finishing the whole DNA extraction process. If so, one needs to process the samples to the point of adding Cell Lysis Solution and store the samples in a cold room at 4°C. The samples will easily be kept viable for months. Do not store unprocessed samples in the cold room, as this has been shown to prevent the preparation of DNA that produces an easily executed PCR.) Using a 20 µl Pipetman and 250 µl pipettes, add 15 µl of Proteinase K (10mg/ml) enzyme into each sample tube, releasing Proteinase K directly into the cell lysate solution of each tube. No part of the Pipetman should touch sample tube - only the pipette tips. Change pipette tip with each sample tube. Vortex briefly to mix. Incubate the cell lysate in the 50 ml tube at 55°C for 1 hour. (The enzyme will not activate until around 55°C, so make sure incubator is near that temperature before starting. It is permissible to incubate longer if needed, even overnight.) Pipette 5 µl of RNase A (5 mg/ml) enzyme directly into the cell lysate solution of each 50 ml sample tube. This is required because of the relatively small volume of the enzyme. Change pipette tips for every new sample. Mix the sample by inverting the tube gently 25 times, and then incubate in the water bath at 37°C for 15 minutes.
**Protein Precipitation.** The sample should be cooled to room temperature. At this point, sample may sit for an hour if needed. Using the pipette aide and 5 ml pipettes, add 0.5 ml of Protein Precipitation Solution to each 50 ml sample tube of cell lysate. Vortex samples for 20 seconds to mix the Protein Precipitation Solution uniformly with the cell lysate. Place 50 ml sample tube in an ice bath for a minimum of 15 minutes, preferably longer. This ensures that the cell protein will form a tight pellet when you centrifuge (next step). Centrifuge at 3000 rpm (2000 x g) for 10 minutes, having the centrifuge refrigerated to 4°C. The precipitated proteins should form a tight, white or green pellet (it may appear green if mint mouthwash was used to collect the buccal samples).
**DNA Precipitation.** While waiting for the centrifuge to finish, prepare enough sterile 15 ml centrifuge tubes to accommodate your samples. Add 5 µl of glycogen (10 mg/ml) to each tube, forming a bead of liquid near the top. Then add 1.5 ml of 100% 2-propanol to each tube. Carefully pour the supernatant containing the DNA into the prepared 15 ml tubes, leaving behind the precipitated protein pellet in the 50 ml tube. If the pellet is loose you may have to pipette the supernatant out, getting as much clear liquid as possible. Pellet may be loose because the sample was not chilled long enough or may need to be centrifuged longer. Nothing but clear greenish liquid should go into the new 15 ml tube. Be careful that the protein pellet does not break loose as you pour. Record on new tube the correct sample number as was on the 50 ml tube. Discard the 50 ml tube. Mix the 15 ml sample tube by inverting gently 50 times. Rough handling may shear DNA strands. Clean white strands clumping together should be observed. Keep at room temperature for at least 5 minutes. Centrifuge at 3000 rpm (2000 x g) for 10 minutes. The DNA may or may not be visible as a small white pellet, depending on yield. (If the pellet is any other color, the sample has contamination. If there is apparent high yield, it may also point to contamination.) Pour off the supernatant into a waste bottle, being careful not to let the DNA dislodge and slide out with the liquid. Invert the open 15ml sample tubes over a clean absorbent paper towel to drain out remaining liquid. Let sit for 5 minutes. Invert tubes right side back up, put caps back on and set them in holding tray (Styrofoam tray the 15 ml tubes were shipped in) with numbered side facing away. Add 1.5ml of 70% ethanol to each tube. Invert the tubes several times to wash the DNA pellet. Centrifuge at 3000 rpm (2000 x g) for 3 minutes. Carefully pour off the ethanol. Invert the sample tube onto a paper towel and let air dry no longer than 15 minutes before resuspending the DNA using a hydration solution. If the DNA is allowed to dry out completely, it will increase the difficulty of rehydrating it. **DNA Hydration.** Depending on the size of the resulting DNA pellet, add between 50-200 µl of DNA Hydration Solution to the 15 ml sample tube. If the tube appears to have no DNA, use 50 µl. If it appears to have some, but not a lot, use 100 µl. With a good-sized pellet, 150-200 µl can be used. This is important because the concentration of DNA affects the results of the PCR experiment, and one does not want to dilute the DNA too much. The optimal concentration of DNA is around 100 ng/µl. Allow the DNA to hydrate by incubating at room temperature overnight or at 65°C for 1 hour. Tap the tube periodically or place on a rotator to aid in dispersing DNA (this helps if the DNA was allowed to dry out completely, but normally it is not required). For storage, sample should be centrifuged briefly and transferred to a cross-linked or UV radiated 1.5 ml centrifuge tube (that was previously autoclaved). Store genomic DNA sample at 4°C. For long term storage, store at -20°C.

While suitable substitute procedures may suffice, following the preceding protocol will ensure the fidelity of the results.

### C. cDNA Production

In one aspect of the invention, it may be useful to prepare a cDNA population for subsequent analysis. In typical cDNA production, mRNA molecules with poly(A) tails are potential templates and will each produce, when treated with a reverse transcriptase, a cDNA in the form of a single-stranded molecule bound to the mRNA (cDNA:mRNA hybrid). The cDNA is then converted into a double-stranded DNA by DNA polymerases such as DNA Pol I (Klenow fragment). Klenow polymerase is used to avoid degradation of the newly synthesized cDNAs. To produce the template for the polymerase, the mRNA must be removed from the cDNA:mRNA hybrid. This is achieved either by boiling or by alkaline treatment (see lecture notes on the properties of nucleic acids). The resulting single stranded cDNA is used as the template to produce the second DNA strand. As with other polymerases, a double-stranded primer sequence is needed and this is fortuitously provided during the reverse transcriptase synthesis which produces a short complementary tail at the 5' end of the cDNA. This tail loops back onto the ss cDNA template (the so-called "hairpin loop") and provides the primer for the polymerase to start the synthesis of the new DNA strand producing a double stranded cDNA (ds cDNA). A consequence of this method of cDNA synthesis is that the two complementary cDNA strands are covalently joined through the hairpin loop. The hairpin loop is removed by use of a single strand specific nuclease (e.g., S1 nuclease from *Aspergillus oryzae*).

Kits for cDNA synthesis (SMART RACE cDNA Amplification Kit; Clontech, Palo Alto, CA). It also is possible to couple cDNA with PCR^{™}, into what is referred to as RT-PCR^{™}. PCR^{™} is discussed in greater detail below.

### IV. Detection Methods

Once the sample has been properly processed, detection of sequence variation is required. Perhaps the most direct method is to actually determine the sequence of either genomic DNA or cDNA and compare these to the known alleles. This can be a fairly expensive and time-consuming process. Nevertheless, this is the lead technology of numerous bioinformatics companies with interests in SNPs including such firms as Celera, Curagen, Incyte, Variagenics and Genaissance, and the technology is available to do fairly high volume sequencing of samples. A variation on the direct sequence determination method is the Gene Chip™ method as advanced by Affymatrix. Such chips are discussed in greater detail below.

Alternatively, more clinically robust and less expensive ways of detecting DNA sequence variation are being developed. For example, Perkin Elmer adapted its TAQman™ Assay to detect sequence variation several years ago.

Orchid BioSciences has a method called SNP-IT™ (SNP-Identification Technology) that uses primer extension with labeled nucleotide analogs to determine which nucleotide occurs at the position immediately 3' of an oligonucleotide probe.

Sequenom uses a hybridization capture technology plus MALDI-TOF (Matrix Assisted Laser Desorption/Ionization - Time-of-Flight mass spectrometry) to detect sequence variation with their MassARRAY™ system.

Promega has the READIT™ SNP/Genotyping System (U.S. Patent 6,159,693). In this method, DNA or RNA probes are hybridized to target nucleic acid sequences. Probes that are complementary to the target sequence at each base are depolymerized with a proprietary mixture of enzymes, while probes which differ from the target at the interrogation position remain intact. The method uses pyrophosphorylation chemistry in combination with luciferase detection to provide a highly sensitive and adaptable SNP scoring system.

Finally, Third Wave Technologies has the Invader OS™ method that uses their proprietary Cleavase® enzymes, which recognize and cut only the specific structure formed during the Invader process The Invader OS relies on linear amplification of the signal generated by the Invader process, rather than on exponential amplification of the target. The Invader OS assay does not utilize PCR in any part of the assay.

Finally, there are a number of forensic DNA testing labs and many research labs that use gene-specific PCR, followed by restriction endonuclease digestion and gel electrophoresis (or other size separation technology) to detect RFLPs in much the same way that the inventors have. The point is that, how one detects sequence variation (SNPs) is not important in the estimation of cancer risk. The key is the genes and polymorphisms that one examines.

The following materials and methodologies relate to the present invention, and are therefore described in some detail.

### A. Chips

As discussed above, one convenient approach to detecting variation involves the use of nucleic acid arrays placed on chips. This technology has been widely exploited by companies such as Affymetrix, and a large number of patented technologies are available. Specifically contemplated are chip-based DNA technologies such as those described by Hacia *et al.* (1996) and Shoemaker *et al.* (1996). These techniques involve quantitative methods for analyzing large numbers of sequences rapidly and accurately. The technology capitalizes on the complementary binding properties of single stranded DNA to screen DNA samples by hybridization. Pease *et al.* (1994); Fodor *et al.* (1991).

Basically, a DNA array or gene chip consists of a solid substrate upon which an array of single-stranded DNA molecules have been attached. For screening, the chip or array is contacted with a single-stranded DNA sample, which is allowed to hybridize under stringent conditions. The chip or array is then scanned to determine which probes have hybridized. In a particular embodiment of the instant invention, a gene chip or DNA array would comprise probes specific for chromosomal changes evidencing the predisposition towards the development of a neoplastic or preneoplastic phenotype. In the context of this embodiment, such probes could include PCR products amplified from patient DNA synthesized oligonucleotides, cDNA, genomic DNA, yeast artificial chromosomes (YACs), bacterial artificial chromosomes (BACs), chromosomal markers or other constructs a person of ordinary skill would recognize as adequate to demonstrate a genetic change.

A variety of gene chip or DNA array formats are described in the art, for example U.S. Patents 5,861,242 and 5,578,832 which are expressly incorporated herein by reference. A means for applying the disclosed methods to the construction of such a chip or array would be clear to one of ordinary skill in the art. In brief, the basic structure of a gene chip or array comprises: (1) an excitation source; (2) an array of probes; (3) a sampling element; (4) a detector; and (5) a signal amplification/treatment system. A chip may also include a support for immobilizing the probe.

In particular embodiments, a target nucleic acid may be tagged or labeled with a substance that emits a detectable signal, for example, luminescence. The target nucleic acid may be immobilized onto the integrated microchip that also supports a phototransducer and related detection circuitry. Alternatively, a gene probe may be immobilized onto a membrane or filter which is then attached to the microchip or to the detector surface itself. In a further embodiment, the immobilized probe may be tagged or labeled with a substance that emits a detectable or altered signal when combined with the target nucleic acid. The tagged or labeled species may be fluorescent, phosphorescent, or otherwise luminescent, or it may emit Raman energy or it may absorb energy. When the probes selectively bind to a targeted species, a signal is generated that is detected by the chip. The signal may then be processed in several ways, depending on the nature of the signal.

The DNA probes may be directly or indirectly immobilized onto a transducer detection surface to ensure optimal contact and maximum detection. The ability to directly synthesize on or attach polynucleotide probes to solid substrates is well known in the art. See U.S. Patents 5,837,832 and 5,837,860, both of which are expressly incorporated by reference. A variety of methods have been utilized to either permanently or removably attach the probes to the substrate. Exemplary methods include: the immobilization of biotinylated nucleic acid molecules to avidin/streptavidin coated supports (Holmstrom, 1993), the direct covalent attachment of short, 5'-phosphorylated primers to chemically modified polystyrene plates (Rasmussen *et al.,* 1991), or the precoating of the polystyrene or glass solid phases with poly-L-Lys or poly L-Lys, Phe, followed by the covalent attachment of either amino- or sulfhydryl-modified oligonucleotides using bifunctional crosslinking reagents (Running *et al.,* 1990; Newton *et al.,* 1993). When immobilized onto a substrate, the probes are stabilized and therefore may be used repeatedly. In general terms, hybridization is performed on an immobilized nucleic acid target or a probe molecule is attached to a solid surface such as nitrocellulose, nylon membrane or glass. Numerous other matrix materials may be used, including reinforced nitrocellulose membrane, activated quartz, activated glass, polyvinylidene difluoride (PVDF) membrane, polystyrene substrates, polyacrylamide-based substrate, other polymers such as poly(vinyl chloride), poly(methyl methacrylate), poly(dimethyl siloxane), and photopolymers (which contain photoreactive species such as nitrenes, carbenes and ketyl radicals) capable of forming covalent links with target molecules.

Binding of the probe to a selected support may be accomplished by any of several means. For example, DNA is commonly bound to glass by first silanizing the glass surface, then activating with carbodimide or glutaraldehyde. Alternative procedures may use reagents such as 3-glycidoxypropyltrimethoxysilane (GOP) or aminopropyltrimethoxysilane (APTS) with DNA linked via amino linkers incorporated either at the 3' or 5' end of the molecule during DNA synthesis. DNA may be bound directly to membranes using ultraviolet radiation. With nitrocellose membranes, the DNA probes are spotted onto the membranes. A UV light source (Stratalinker,™ Stratagene, La Jolla, CA) is used to irradiate DNA spots and induce cross-linking. An alternative method for cross-linking involves baking the spotted membranes at 80°C for two hours in vacuum.

Specific DNA probes may first be immobilized onto a membrane and then attached to a membrane in contact with a transducer detection surface. This method avoids binding the probe onto the transducer and may be desirable for large-scale production. Membranes particularly suitable for this application include nitrocellulose membrane (e.g., from BioRad, Hercules, CA) or polyvinylidene difluoride (PVDF) (BioRad, Hercules, CA) or nylon membrane (Zeta-Probe, BioRad) or polystyrene base substrates (DNA.BIND™ Costar, Cambridge, MA).

### B. Nucleic Acid Amplification Procedures

A useful technique in working with nucleic acids involves amplification. Amplifications are usually template-dependent, meaning that they rely on the existence of a template strand to make additional copies of the template. Primers, short nucleic acids that are capable of priming the synthesis of a nascent nucleic acid in a template-dependent process, are hybridized to the template strand. Typically, primers are from ten to thirty base pairs in length, but longer sequences can be employed. Primers may be provided in double-stranded and/or single-stranded form, although the single-stranded form generally is preferred.

Often, pairs of primers are designed to selectively hybridize to distinct regions of a template nucleic acid, and are contacted with the template DNA 'under conditions that permit selective hybridization. Depending upon the desired application, high stringency hybridization conditions may be selected that will only allow hybridization to sequences that are completely complementary to the primers. In other embodiments, hybridization may occur under reduced stringency to allow for amplification of nucleic acids containing one or more mismatches with the primer sequences. Once hybridized, the template-primer complex is contacted with one or more enzymes that facilitate template-dependent nucleic acid synthesis. Multiple rounds of amplification, also referred to as "cycles," are conducted until a sufficient amount of amplification product is produced.

**PCR:** A number of template dependent processes are available to amplify the oligonucleotide sequences present in a given template sample. One of the best known amplification methods is the polymerase chain reaction (referred to as PCR™) which is described in detail in U.S. Patents 4,683,195, 4,683,202 and 4,800,159, and in Innis *et al.,* 1988, each of which is incorporated herein by reference in their entirety. In PCR™, pairs of primers that selectively hybridize to nucleic acids are used under conditions that permit selective hybridization. The term primer, as used herein, encompasses any nucleic acid that is capable of priming the synthesis of a nascent nucleic acid in a template-dependent process. Primers may be provided in double-stranded or single-stranded form, although the single-stranded form is preferred.

The primers are used in any one of a number of template dependent processes to amplify the target gene sequences present in a given template sample. One of the best known amplification methods is PCR™ which is described in detail in U.S. Patents 4,683,195, 4,683,202 and 4,800,159, each incorporated herein by reference.

In PCR™, two primer sequences are prepared which are complementary to regions on opposite complementary strands of the target-gene(s) sequence. The primers will hybridize to form a nucleic-acid:primer complex if the target-gene(s) sequence is present in a sample. An excess of deoxyribonucleoside triphosphates is added to a reaction mixture along with a DNA polymerase, *e.g., Taq* polymerase, that facilitates template-dependent nucleic acid synthesis.

If the target-gene(s) sequence:primer complex has been formed, the polymerase will cause the primers to be extended along the target-gene(s) sequence by adding on nucleotides. By raising and lowering the temperature of the reaction mixture, the extended primers will dissociate from the target-gene(s) to form reaction products, excess primers will bind to the target-gene(s) and to the reaction products and the process is repeated. These multiple rounds of amplification, referred to as "cycles", are conducted until a sufficient amount of amplification product is produced.

A reverse transcriptase PCR™ amplification procedure may be performed in order to quantify the amount of mRNA amplified. Methods of reverse transcribing RNA into cDNA are well known and described in Sambrook *et al.,* 1989. Alternative methods for reverse transcription utilize thermostable DNA polymerases. These methods are described in WO 90/07641, filed December 21, 1990.

**LCR:** Another method for amplification is the ligase chain reaction ("LCR"), disclosed in European Patent Application No. 320,308, incorporated herein by reference. In LCR, two complementary probe pairs are prepared, and in the presence of the target sequence, each pair will bind to opposite complementary strands of the target such that they abut. In the presence of a ligase, the two probe pairs will link to form a single unit. By temperature cycling, as in PCR™, bound ligated units dissociate from the target and then serve as "target sequences" for ligation of excess probe pairs. U.S. Patent 4,883,750, incorporated herein by reference, describes a method similar to LCR for binding probe pairs to a target sequence.

**Qbeta Replicase:** Qbeta Replicase, described in PCT Patent Application No. PCT/US87/00880, also may be used as still another amplification method in the present invention. In this method, a replicative sequence of RNA which has a region complementary to that of a target is added to a sample in the presence of an RNA polymerase. The polymerase will copy the replicative sequence which can then be detected.

**Isothermal Amplification:** An isothermal amplification method, in which restriction endonucleases and ligases are used to achieve the amplification of target molecules that contain nucleotide 5'-[α-thio]-triphosphates in one strand of a restriction site also may be useful in the amplification of nucleic acids in the present invention. Such an amplification method is described by Walker *et al.* 1992, incorporated herein by reference.

**Strand Displacement Amplification:** Strand Displacement Amplification (SDA) is another method of carrying out isothermal amplification of nucleic acids which involves multiple rounds of strand displacement and synthesis, *i.e*., nick translation. A similar method, called Repair Chain Reaction (RCR), involves annealing several probes throughout a region targeted for amplification, followed by a repair reaction in which only two of the four bases are present. The other two bases can be added as biotinylated derivatives for easy detection. A similar approach is used in SDA.

**Cyclic Probe Reaction:** Target specific sequences can also be detected using a cyclic probe reaction (CPR). In CPR, a probe having 3' and 5' sequences of non-specific DNA and a middle sequence of specific RNA is hybridized to DNA which is present in a sample. Upon hybridization, the reaction is treated with RNase H, and the products of the probe identified as distinctive products which are released after digestion. The original template is annealed to another cycling probe and the reaction is repeated.

**Transcription-Based Amplification:** Other nucleic acid amplification procedures include transcription-based amplification systems (TAS), including nucleic acid sequence based amplification (NASBA) and 3SR, Kwoh *et al.* (1989); PCT Application WO 88/10315, 1989, (each incorporated herein by reference).

In NASBA, the nucleic acids can be prepared for amplification by standard phenol/chloroform extraction, heat denaturation of a clinical sample, treatment with lysis buffer and minispin columns for isolation of DNA and RNA or guanidinium chloride extraction of RNA. These amplification techniques involve annealing a primer, which has target specific sequences. Following polymerization, DNA/RNA hybrids are digested with RNase H while double stranded DNA molecules are heat denatured again. In either case the single stranded DNA is made fully double stranded by addition of second target specific primer, followed by polymerization. The double-stranded DNA molecules are then multiply transcribed by a polymerase such as T7 or SP6. In an isothermal cyclic reaction, the RNA's are reverse transcribed into double stranded DNA, and transcribed once against with a polymerase such as T7 or SP6. The resulting products, whether truncated or complete, indicate target specific sequences.

**Other Amplification Methods:** Other amplification methods, as described in British Patent Application No. GB 2,202,328, and in PCT Application No. PCT/US89/01025, each incorporated herein by reference, may be used in accordance with the present invention. In the former application, "modified" primers are used in a PCR™ like, template and enzyme dependent synthesis. The primers may be modified by labeling with a capture moiety (e.g., biotin) and/or a detector moiety (e.g., enzyme). In the latter application, an excess of labeled probes are added to a sample. In the presence of the target sequence, the probe binds and is cleaved catalytically. After cleavage, the target sequence is released intact to be bound by excess probe. Cleavage of the labeled probe signals the presence of the target sequence.

Davey *et al.,* European Patent Application No. 329 822 (incorporated herein by reference) disclose a nucleic acid amplification process involving cyclically synthesizing single-stranded RNA ("ssRNA"), ssDNA, and double-stranded DNA (dsDNA), which may be used in accordance with the present invention.

The ssRNA is a first template for a first primer oligonucleotide, which is elongated by reverse transcriptase (RNA-dependent DNA polymerase). The RNA is then removed from the resulting DNA:RNA duplex by the action of ribonuclease H (RNase H, an RNase specific for RNA in duplex with either DNA or RNA). The resultant ssDNA is a second template for a second primer, which also includes the sequences of an RNA polymerase promoter (exemplified by T7 RNA polymerase) 5' to its homology to the template. This primer is then extended by DNA polymerase (exemplified by the large "Klenow" fragment of E. *coli* DNA polymerase I), resulting in a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

Miller et al., PCT Patent Application WO 89/06700 (incorporated herein by reference) disclose a nucleic acid sequence amplification scheme based on the hybridization of a promoter/primer sequence to a target single-stranded DNA ("ssDNA") followed by transcription of many RNA copies of the sequence. This scheme is not cyclic, *i.e*., new templates are not produced from the resultant RNA transcripts.

Other suitable amplification methods include "race" and "one-sided PCR™" (Frohman, 1990; Ohara *et al.,* 1989, each herein incorporated by reference). Methods based on ligation of two (or more) oligonucleotides in the presence of nucleic acid having the sequence of the resulting "di-oligonucleotide", thereby amplifying the di-oligonucleotide, also may be used in the amplification step of the present invention, Wu *et al.,* 1989, incorporated herein by reference).

### C. Methods for Nucleic Acid Separation

It may be desirable to separate nucleic acid products from other materials, such as template and excess primer. In one embodiment, amplification products are separated by agarose, agarose-acrylamide or polyacrylamide gel electrophoresis using standard methods (Sambrook *et al.,* 1989). Separated amplification products may be cut out and eluted from the gel for further manipulation. Using low melting point agarose gels, the separated band may be removed by heating the gel, followed by extraction of the nucleic acid.

Separation of nucleic acids may also be effected by chromatographic techniques known in art. There are many kinds of chromatography which may be used in the practice of the present invention, including adsorption, partition, ion-exchange, hydroxylapatite, molecular sieve, reverse-phase, column, paper, thin-layer, and gas chromatography as well as HPLC.

In certain embodiments, the amplification products are visualized. A typical visualization method involves staining of a gel with ethidium bromide and visualization of bands under UV light. Alternatively, if the amplification products are integrally labeled with radio- or fluorometrically-labeled nucleotides, the separated amplification products can be exposed to x-ray film or visualized with light exhibiting the appropriate excitatory spectra.

### V. Personal History Measures

In addition to use of the genetic analysis disclosed herein, the present invention makes use of additional factors in gauging an individual's risk for developing cancer. In particular, one will examine multiple factors including age, ethnicity, reproductive history, menstruation history, use of oral contraceptives, body mass index, alcohol consumption history, smoking history, exercise history, and diet to improve the predictive accuracy of the present methods. A history of cancer in a relative and the age at which the relative was diagnosed with cancer are also important personal history measures. The inclusion of personal history measures with genetic data in an analysis to predict a phenotype, cancer in this case, is grounded in the realization that almost all phenotypes are derived from a dynamic interaction between an individual's genes and the environment in which these genes act. For example, fair skin may predispose an individual to melanoma but only if the individual is exposed to prolonged unshielded exposure to the sun's ultraviolet radiation. The inventors include personal history measures in their analysis because they are possible modifiers of the penetrance of the cancer phenotype for any genotype examined. Those skilled in the art will realize that the personal history measures listed in this paragraph are unlikely to be the only such environmental factors that affect the penetrance of the cancer phenotype.

### VI. Kits

The present invention also contemplates the preparation of kits for use in accordance with the present invention. Suitable kits include various reagents for use in accordance with the present invention in suitable containers and packaging materials, including tubes, vials, and shrink-wrapped and blow-molded packages.

Materials suitable for inclusion in a kit in accordance with the present invention comprises one or more of the following:
gene specific PCR primer pairs (oligonucleotides) that anneal to DNA or cDNA sequence domains that flank the genetic polymorphisms of interest (for example, those listed in Table 1); reagents capable of amplifying a specific sequence domain in either genomic DNA or cDNA without the requirement of performing PCR;
reagents required to discriminate between the various possible alleles in the sequence domains amplified by PCR or non-PCR amplification (e.g., restriction endonucleases, oligonucleotides that anneal preferentially to one allele of the polymorphism, including those modified to contain enzymes or fluorescent chemical groups that amplify the signal from the oligonucleotide and make discrimination of alleles most robust);
reagents required to physically separate products derived from the various alleles (e.g., agarose or polyacrylamide and a buffer to be used in electrophoresis, HPLC columns, SSCP gels, formamide gels or a matrix support for MALDI-TOF).

### VII. Cancer Prophylaxis

In one aspect of the invention, there is an improved ability to identify candidates for prophylactic cancer treatments due to being assessed as at high risk of developing breast cancer. The primary drugs for use in breast cancer prophylaxis are tamoxifen and raloxifene, discussed further below.

### A. Tamoxifen

Tamoxifen (NOLVADEX®) a nonsteroidal antiestrogen, is provided as tamoxifen citrate. Tamoxifen citrate tablets are available as 10 mg or 20 mg tablets. Each 10 mg tablet contains 15.2 mg of tamoxifen citrate which is equivalent to 10 mg of tamoxifen. Inactive ingredients include carboxymethylcellulose calcium, magnesium stearate, mannitol and starch. Tamoxifen citrate is the trans-isomer of a triphenylethylene derivative. The chemical name is (Z)2-[4-(1,2-diphenyl-1-butenyl) phenoxy]-N, N-dimethylethanamine 2-hydroxy-1,2,3- propanetricarboxylate (1:1). Tamoxifen citrate has a molecular weight of 563.62, the pKa' is 8.85, the equilibrium solubility in water at 37°C is 0.5 mg/mL and in 0.02 N HCl at 37°C, it is 0.2 mg/mL.

Tamoxifen citrate has potent antiestrogenic properties in animal test systems. While the precise mechanism of action is unknown, the antiestrogenic effects may be related to its ability to compete with estrogen for binding sites in target tissues such as breast. Tamoxifen inhibits the induction of rat mammary carcinoma induced by dimethylbenzanthracene (DMBA) and causes the regression of DMBA-induced tumors *in situ* in rats. In this model, tamoxifen appears to exert its antitumor effects by binding the estrogen receptors.

Tamoxifen is extensively metabolized after oral administration. Studies in women receiving 20 mg of radiolabeled (¹⁴C) tamoxifen have shown that approximately 65% of the administered dose is excreted from the body over a period of 2 weeks (mostly by fecal route). N-desmethyl tamoxifen is the major metabolite found in patients' plasma. The biological activity of N-desmethyl tamoxifen appears to be similar to that of tamoxifen. 4-hydroxytamoxifen, as well as a side chain primary alcohol derivative of tamoxifen, have been identified as minor metabolites in plasma.

Following a single oral dose of 20 mg, an average peak plasma concentration of 40 ng/mL (range 35 to 45 ng/mL) occurred approximately 5 hours after dosing. The decline in plasma concentrations of tamoxifen is biphasic, with a terminal elimination half-life of about 5 to 7 days. The average peak plasma concentration of N-desmethyl tamoxifen is 15 ng/mL (range 10 to 20 ng/mL). Chronic administration of 10 mg tamoxifen given twice daily for 3 months to patients results in average steady-state plasma concentrations of 120 ng/mL (range 67-183 ng/mL) for tamoxifen and 336 ng/mL (range 148-654 ng/mL) for N-desmethyl tamoxifen. The average steady-state plasma concentrations of tamoxifen and N-desmethyl tamoxifen after administration of 20 mg tamoxifen once daily for 3 months are 122 ng/mL (range 71-183 ng/mL) and 353 ng/mL (range 152-706 ng/mL), respectively. After initiation of therapy, steady state concentrations for tamoxifen are achieved in about 4 weeks and steady state concentrations for N-desmethyl tamoxifen are achieved in about 8 weeks, suggesting a half-life of approximately 14 days for this metabolite.

For patients with breast cancer, the recommended daily dose is 20-40 mg. Dosages greater than 20 mg per day should be given in divided doses (morning and evening). Prophylactic doses may be lower, however.

### B. Raloxifene

Raloxifene hydrochloride (EVISTA®) is a selective estrogen receptor modulator (SERM) that belongs to the benzothiophene class of compounds. The chemical designation is methanone, [6-hydroxy-2-(4-hydroxyphenyl)benzo[b]thien-3-yl]-[4-[2-(1-piperidinyl) ethoxy] phenyl]-hydrochloride. Raloxifene hydrochloride (HCl) has the empirical formula C₂₈H₂₇NO₄S • HCl, which corresponds to a molecular weight of 510.05. Raloxifene HCl is an off-white to pale-yellow solid that is very slightly soluble in water.

Raloxifene HCl is supplied in a tablet dosage form for oral administration. Each tablet contains 60 mg of raloxifene HCl, which is the molar equivalent of 55.71 mg of free base. Inactive ingredients include anhydrous lactose, carnuba wax, crospovidone, FD& C Blue No. 2 aluminum lake, hydroxypropyl methylcellulose, lactose monohydrate, magnesium stearate, modified pharmaceutical glaze, polyethylene glycol, polysorbate 80, povidone, propylene glycol, and titanium dioxide.

Raloxifene's biological actions, like those of estrogen, are mediated through binding to estrogen receptors. Preclinical data demonstrate that raloxifene is an estrogen antagonist in uterine and breast tissues. Preliminary clinical data (through 30 months) suggest EVISTA® lacks estrogen-like effects on uterus and breast tissue.

Raloxifene is absorbed rapidly after oral administration. Approximately 60% of an oral dose is absorbed, but presystemic glucuronide conjugation is extensive. Absolute bioavailability of raloxifene is 2.0%. The time to reach average maximum plasma concentration and bioavailability are functions of systemic interconversion and enterohepatic cycling of raloxifene and its glucuronide metabolites.

Following oral administration of single doses ranging from 30 to 150 mg of raloxifene HCl, the apparent volume of distribution is 2.348 L/kg and is not dose dependent. Biotransformation and disposition of raloxifene in humans have been determined following oral administration of ¹⁴C-labeled raloxifene. Raloxifene undergoes extensive first-pass metabolism to the glucuronide conjugates: raloxifene-4'-glucuronide, raloxifene-6-glucuronide, and raloxifene-6, 4'-diglucuronide. No other metabolites have been detected, providing strong evidence that raloxifene is not metabolized by cytochrome P450 pathways. Unconjugated raloxifene comprises less than 1% of the total radiolabeled material in plasma. The terminal log-linear portions of the plasma concentration curves for raloxifene and the glucuronides are generally parallel. This is consistent with interconversion of raloxifene and the glucuronide metabolites.

Following intravenous administration, raloxifene is cleared at a rate approximating hepatic blood flow. Apparent oral clearance is 44.1 L/kg per hour. Raloxifene and its glucuronide conjugates are interconverted by reversible systemic metabolism and enterohepatic cycling, thereby prolonging its plasma elimination half-life to 27.7 hours after oral dosing. Results from single oral doses of raloxifene predict multiple-dose pharmacokinetics. Following chronic dosing, clearance ranges from 40 to 60 L/kg per hour. Increasing doses of raloxifene HCl (ranging from 30 to 150 mg) result in slightly less than a proportional increase in the area under the plasma time concentration curve (AUC). Raloxifene is primarily excreted in feces, and less than 0.2% is excreted unchanged in urine. Less than 6% of the raloxifene dose is eliminated in urine as glucuronide conjugates.

The recommended dosage is one 60-mg tablet daily which may be administered any time of day without regard to meals. Supplemental calcium is recommended if dietary intake is inadequate.

### C. STAR

More than 400 centers across the U.S., Canada and Puerto Rico are currently participating in a clinical trial for tamoxifen and raloxifene, known as STAR. It is one of the largest breast cancer prevention trials ever undertaken. STAR is also the first trial to compare a drug proven to reduce the chance of developing breast cancer with another drug that has the potential to reduce breast cancer risk. All participants receive one or the other drug for five years. At least 22,000 postmenopausal women at high-risk of breast cancer will participate in STAR. All races and ethnic groups are encouraged to participate in STAR.

Tamoxifen (NOLVADEX®) was proven in the Breast Cancer Prevention Trial to reduce breast cancer incidence by 49 percent in women at increased risk of the disease. The U.S. Food and Drug Administration (FDA) approved the use of tamoxifen to reduce the incidence of breast cancer in women at increased risk of the disease in October 1998. Tamoxifen has been approved by the FDA to treat women with breast cancer for more than 20 years and has been in clinical trials for about 30 years.

Raloxifene (trade name EVISTA®) was shown to reduce the incidence of breast cancer in a large study of its use to prevent and treat osteoporosis. This drug was approved by the FDA to prevent osteoporosis in postmenopausal women in December 1997 and has been under study for about five years.

The study is a randomized double-blinded clinical trial to compare the effectiveness of raloxifene with that of tamoxifen in preventing breast cancer in postmenopausal women. Women must be at least 35 years old, have gone no more than one year since undergoing mammography with no evidence of cancer, have no previous mastectomy to prevent breast cancer, have no previous invasive breast cancer or intraductal carcinoma *in situ,* have not had hormone therapy in at least three months, and have no previous radiation therapy to the breast.

Patients will be randomly assigned to one of two groups. Patients in group one will receive raloxifene plus a placebo by mouth once a day. Patients in group two will receive tamoxifen plus a placebo by mouth once a day. Treatment will continue for 5 years. Quality of life will be assessed at the beginning of the study and then every 6 months for 5 years. Patients will then receive follow-up evaluations once a year.

Those skilled in the art will realize that there are other chemopreventative drugs currently under development. The disclosed invention is expected to facilitate more appropriate and effective application of these new drugs also when and if they become commercially available.

### VIII. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1 - METHODS

DNA specimens from individuals who had been diagnosed with breast cancer or from cancer free controls were arrayed on 96-well PCR plates. Operators were blinded as to information about whether individual specimens were from cancer patients or controls. Also, included among specimens arrayed on the 96-well plates were DNA specimens of known genotypes and no template control blank wells. PCR was performed with gene specific primer pairs that flanked the sites of the genetic polymorphisms that were assayed. The gene specific PCR products were typically digested with restriction endonucleases that recognize and cleave one allele of the SNPs but not the other. Restriction digested PCR products were then displayed by electrophoresis and scored as restriction fragment length polymorphisms (RFLPs). For those genetic polymorphisms that were caused by insertions or deletions of DNA sequences in one allele of a polymorphism relative to the other allele, the polymorphisms were assayed directly as length polymorphisms by display of the gene specific PCR products by gel electrophoresis without prior digestion with a restriction endonuclease. The genotypes of the individual DNA specimens were determined by examining images of the electrophoretic gels upon which the digested or undigested gene specific PCR products were displayed. About one quarter of DNA specimens were subjected to repeat analysis to internally replicate and validate the results of the various assays. The data from the genotyping assays were provided to a biostatistician who broke the numerical code and assigned the various genotypes to the appropriate category, breast cancer patients or cancer free controls. Statistical analysis was performed to determine the association of various genotypes with breast cancer cases relative to the controls.

The results of the analysis are presented in Tables 2A-4B. Tables 2A-C show the associations between combinations of polymorphic alleles with breast cancer risk in the unstratified population of all white women. Tables 3A-B show the associations only for women less than 54 years of age. Tables 4A-B discloses results for women with breast cancer diagnosed when they were over 54 years of age.

The tables are divided into two or three parts, parts A and B or A-C. Part A shows the association of risk with polymorphisms in the twenty examined genes when they are examined one at a time. Part B shows the highest associated risk correlations of these same genes when examined in combinations of two at a time. Part C shows the highest associated risk correlations when genes are examined three at a time.

### EXAMPLE 2 - RESULTS: NOT AGE STRATIFIED

The inventors examined the association of various genetic polymorphisms with breast cancer. The data set used in this analysis consisted of nearly 600 women that have been diagnosed with breast cancer and approximately 1400 women who had never been diagnosed with any cancer. Twenty different genetic polymorphisms were examined. The DNA specimens were assigned numbers for reference but were otherwise stripped of personal identifiers. The results presented in Tables 2A-C are a synthesis of a complex bootstrap analysis performed many different ways. In general, when examined singly (one at a time), the polymorphism were weakly associated with risk of a breast cancer diagnosis. As a group, they may be termed common risk alleles with low penetrance or no penetrance for the breast cancer phenotype. The surprising observation made during this study was that when examined in combination (in pairs or in combinations of three or more), complex genotypes were defined that exhibited higher risk or higher penetrance for a breast cancer diagnosis. Table 2A, presents results for these polymorphisms examined singly. Tables 2B and 2C present results for analysis of these same polymorphisms examined in pairs or in combinations of three respectively. The inventors found that examining these polymorphisms in combinations of two (pairs) is more informative for estimating breast cancer risk than examining polymorphisms alone. These gene pairs can then form the building blocks for identifying complex genotypes involving three or more polymorphic genes with greatly improved discriminatory accuracy for stratifying a woman's individual risk of being diagnosed with breast cancer. The inventors show that by starting with these building blocks of gene pairs, and then adding information from other genes, one can stratify an individual's risk of being diagnosed with breast cancer over a wide range of relative risk. This range extends from less than average risk to many times average risk.

The tables show the gene or genes being examined and the genotypes of these genes being compared. The tables also show the mean Odds Ratio (OR) or relative risk with the respective genotypes compared to an individual with average risk. The OR is shown as a mean because it is the composite or aggregate result of many analyses and represents the best estimate of the true OR in the general population. An OR less than one represents less than average risk while an OR greater than one represents greater than average risk. The mean p value is a measure statistical significance based on an average of Chi² tests of the ORs in used to determine the mean OR. By convention, p values ≤ 0.05 are considered to be statistically significant. It is a general observation of this analysis that the ORs and associated p values for the genetic polymorphisms examined by themselves have very limited ability to stratify risk and have p values typically greater than 0.05. In pairs, the polymorphisms stratify risk over a greater range and have much lower (more significant) p values. This trend continues as additional genes are added to the pairs with even greater stratification of risk and much smaller p values.

**Table 2A - White Women of All Ages (Polymorphisms of Genes examined Singly)**

| >>> Prohibitin | | |
|---|---|---|
| Genotype | Mean OR | Mean p |
| C/C | 0.82 ± 0.03 | 0.1023 |
| C/T | 1.28 ± 0.05 | 0.0425 |
| */T | 1.22 ± 0.05 | 0.1023 |

| >>> CYP17 | | |
|---|---|---|
| Genotype | Mean OR | Mean p |
| C/C | 1.04 ± 0.06 | 0.7376 |
| C/T | 0.96 ± 0.04 | 0.6698 |
| T/T | 1.03 ± 0.04 | 0.7309 |

| >>> COMT | | |
|---|---|---|
| Genotype | Mean OR | Mean p |
| A/A | 0.86 ± 0.04 | 0.2260 |
| G/A | 1.22 ± 0.05 | 0.0829 |
| G/G | 0.90 ± 0.04 | 0.4307 |

| >>> GSTP1 | | |
|---|---|---|
| Genotype | Mean OR | Mean p |
| A/A | 0.96 ± 0.04 | 0.6826 |
| G/A | 0.97 ± 0.04 | 0.7181 |
| G/G | 1.17 ± 0.07 | 0.3713 |

| >>> SULF1A1 | | |
|---|---|---|
| Genotype | Mean OR | Mean p |
| A/A | 1.24 ± 0.07 | 0.1962 |
| G/A | 0.85 ± 0.03 | 0.1507 |
| G/G | 1.06 ± 0.04 | 0.5804 |

| >>> HER2 | | |
|---|---|---|
| Genotype | Mean OR | Mean p |
| A/A | 1.05 ± 0.04 | 0.1855 |
| G/A | 0.97 ± 0.04 | 0.7359 |
| G/G | 0.93 ± 0.08 | 0.7097 |

| >>> p53 72 | | |
|---|---|---|
| Genotype | Mean OR | Mean p |
| C/C | 1.26 ± 0.09 | 0.2814 |
| C/G | 1.07 ± 0.04 | 0.5559 |
| G/G | 0.88 ± 0.03 | 0.2621 |

| >>> CYP1B1 | | |
|---|---|---|
| Genotype | Mean OR | Mean p |
| C/C | 1.12 ± 0.04 | 0.3675 |
| C/G | 0.88 ± 0.03 | 0.2744 |
| G/G | 1.05 ± 0.05 | 0.6783 |

| >>> PROGINS | | |
|---|---|---|
| Genotype | Mean OR | Mean p |
| I/I | 1.04 ± 0.14 | 0.7613 |
| I/N | 0.95 ± 0.04 | 0.6663 |
| N/N | 1.04 ± 0.05 | 0.6868 |

| >>> SRD5A2_2 | | |
|---|---|---|
| Genotype | Mean OR | Mean p |
| O/O | 1.08 ± 0.05 | 0.5706 |
| O/A | 0.97 ± 0.05 | 0.7280 |
| A/A | 0.53 ± 0.11 | 0.2667 |

| >>> MTHFR | | |
|---|---|---|
| Genotype | Mean OR | Mean p |
| C/C | 1.06 ± 0.05 | 0.6656 |
| C/T | 0.99 ± 0.05 | 0.7823 |
| T/T | 0.89 ± 0.07 | 0.6085 |

| >>> VDR/ApaI | | |
|---|---|---|
| Genotype | Mean OR | Mean p |
| A/A | 1.22 ± 0.06 | 0.1209 |
| A/a | 0.87 ± 0.04 | 0.2560 |
| a/a | 0.95 ± 0.04 | 0.6873 |

| >>> VDR/TaqI | | |
|---|---|---|
| Genotype | Mean OR | Mean p |
| T/T | 0.97 ± 0.04 | 0.7214 |
| T/t | 0.94 ± 0.04 | 0.5922 |
| t/t | 1.17 ± 0.06 | 0.3102 |

| >>> VDR/FokI | | |
|---|---|---|
| Genotype | Mean OR | Mean p |
| F/F | 1.15 ± 0.05 | 0.3302 |
| F/f | 0.92 ± 0.04 | 0.5363 |
| f/f | 0.91 ± 0.06 | 0.6103 |

| >>> CYP2E1 | | |
|---|---|---|
| Genotype | Mean OR | Mean p |
| D/D | 0.76 ± 0.05 | 0.1699 |
| D/C | 1.52 ± 0.11 | 0.0571 |
| C/C | 0.51 ± 0.12 | 0.2877 |

| >>> EPHX | | |
|---|---|---|
| Genotype | Mean OR | Mean p |
| Y/Y | 0.87 ± 0.06 | 0.4822 |
| Y/H | 1.20 ± 0.09 | 0.3897 |
| H/H | 0.96 ± 0.09 | 0.7543 |

| >>> CYP1A1 | | |
|---|---|---|
| Genotype | Mean OR | Mean p |
| M/M | 1.02 ± 0.05 | 0.7644 |
| M/m | 0.99 ± 0.05 | 0.7724 |
| m/m | 0.90 ± 0.17 | 0.7447 |

| >>> CYP11B2 | | |
|---|---|---|
| Genotype | Mean OR | Mean p |
| C/C | 0.93 ± 0.05 | 0.5958 |
| C/T | 1.13 ± 0.04 | 0.2977 |
| T/T | 0.92 ± 0.04 | 0.4929 |

| >>> CYC D1 | | |
|---|---|---|
| Genotype | Mean OR | Mean p |
| A/A | 1.86 ± 0.14 | 0.0120 |
| G/A | 0.82 ± 0.05 | 0.3245 |
| G/G | 0.76 ± 0.05 | 0.2110 |

| >>> XRCC 1 | | |
|---|---|---|
| Genotype | Mean OR | Mean p |
| A/A | 1.19 ± 0.12 | 0.5723 |
| G/A | 0.74 ± 0.05 | 0.1366 |
| G/G | 1.27 ± 0.08 | 0.2510 |

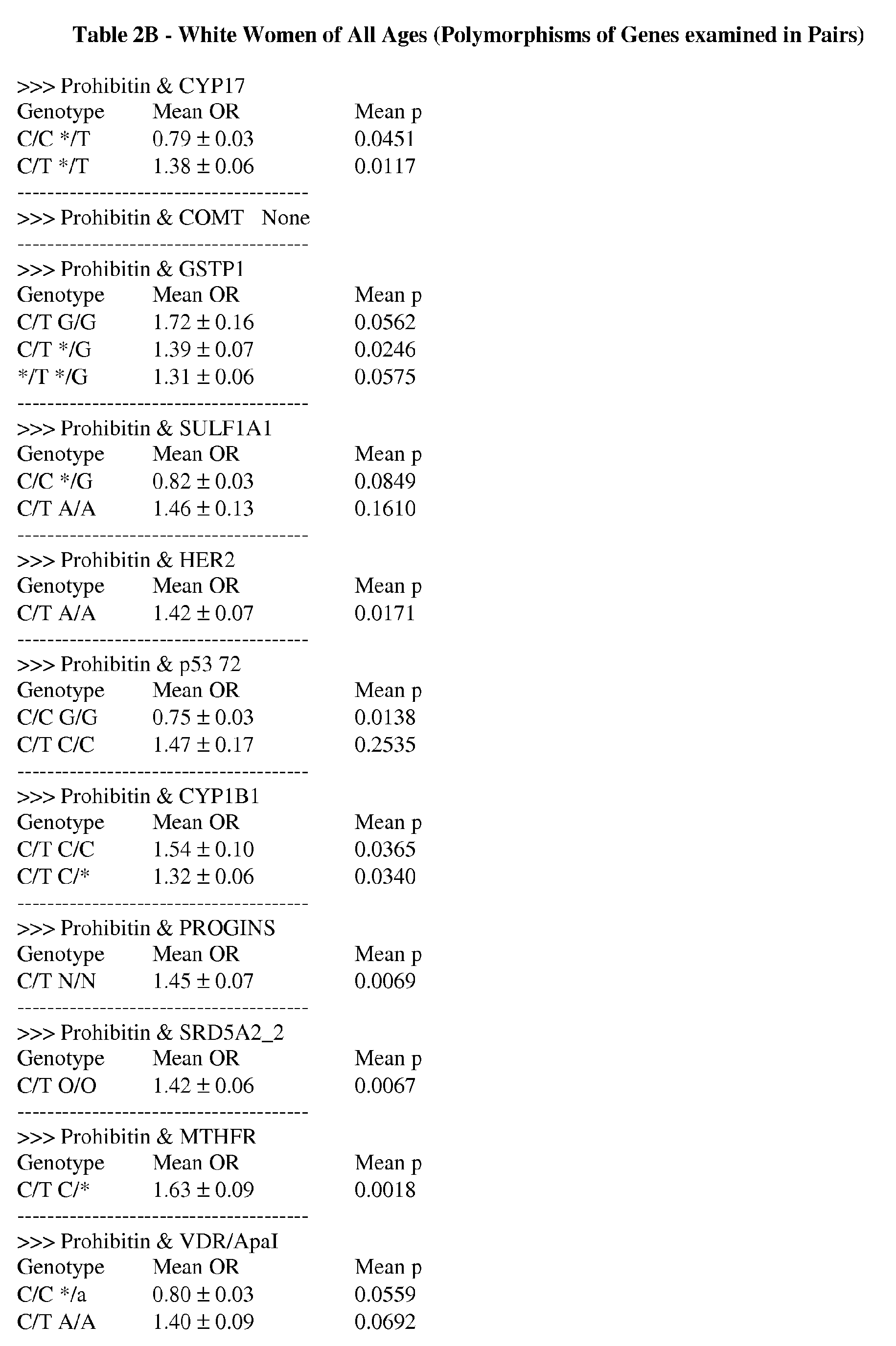

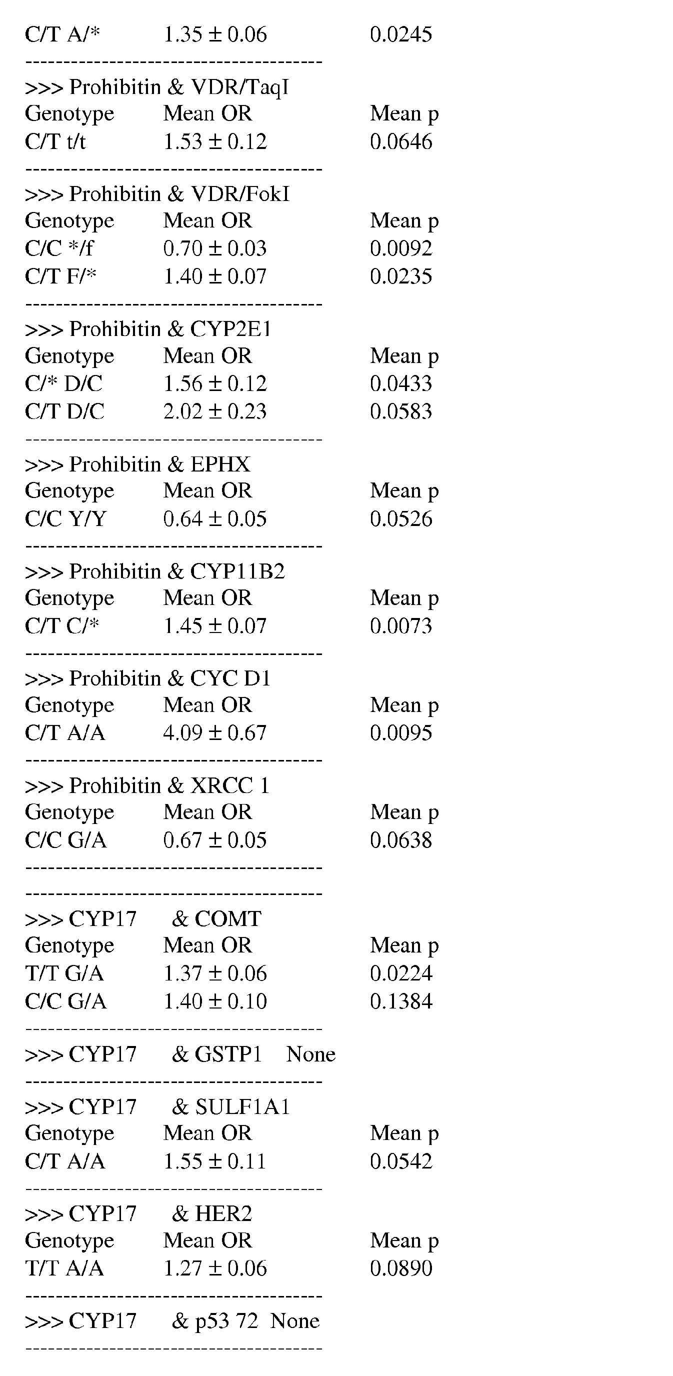

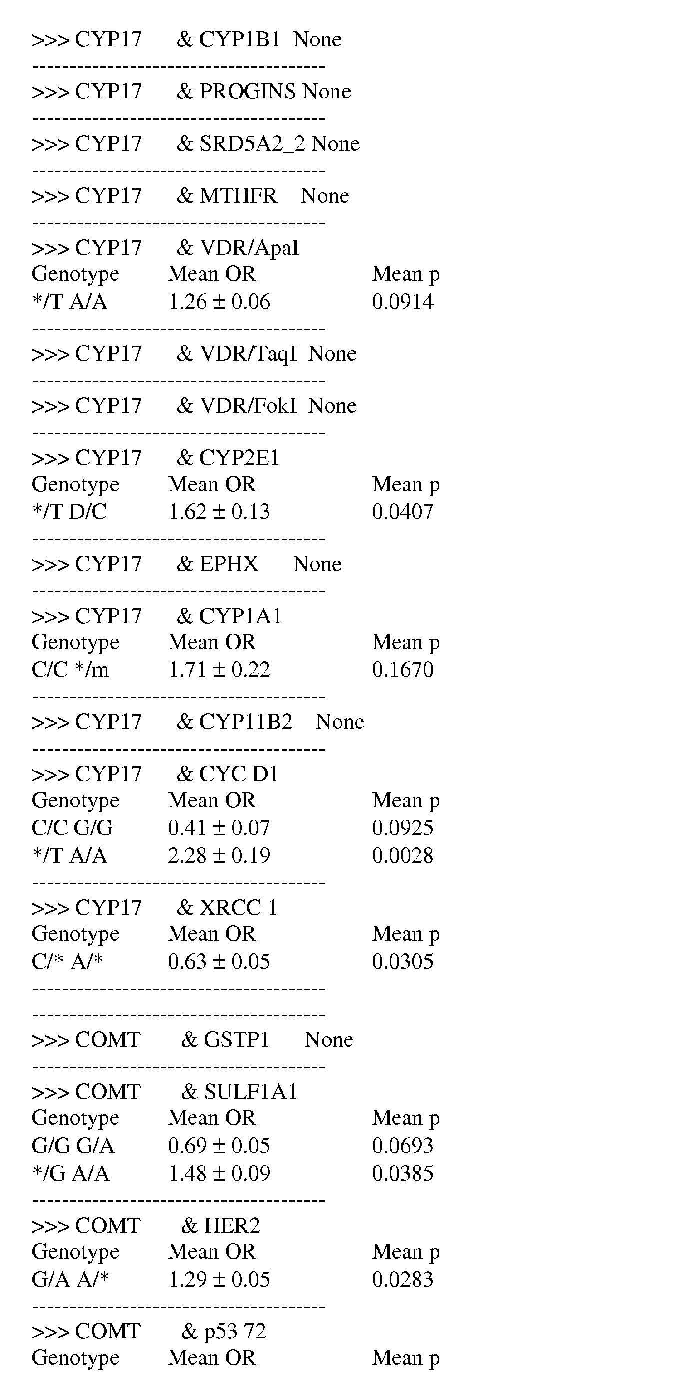

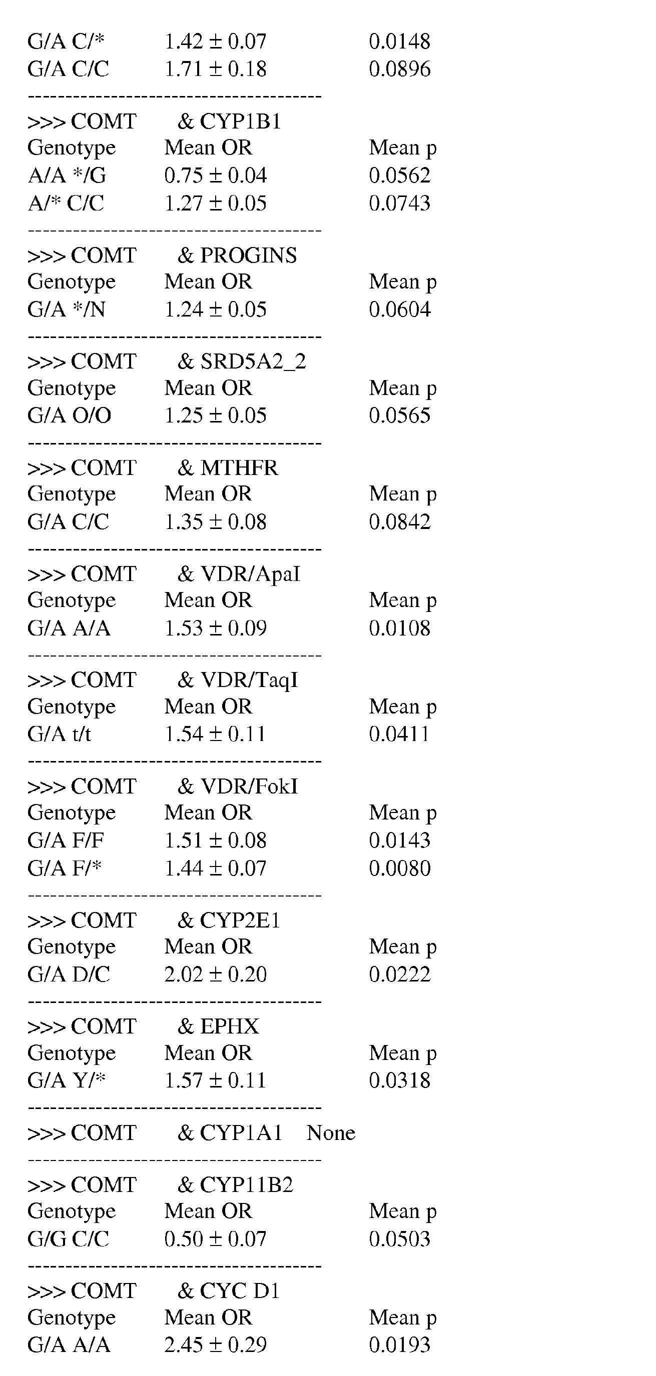

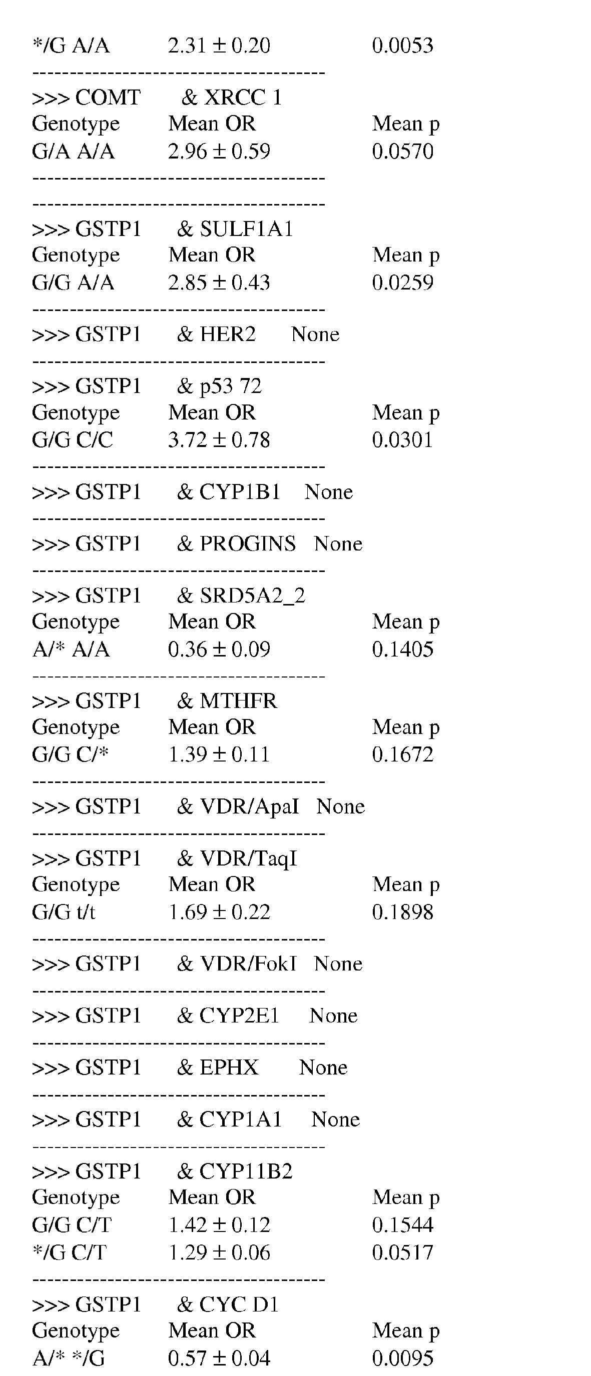

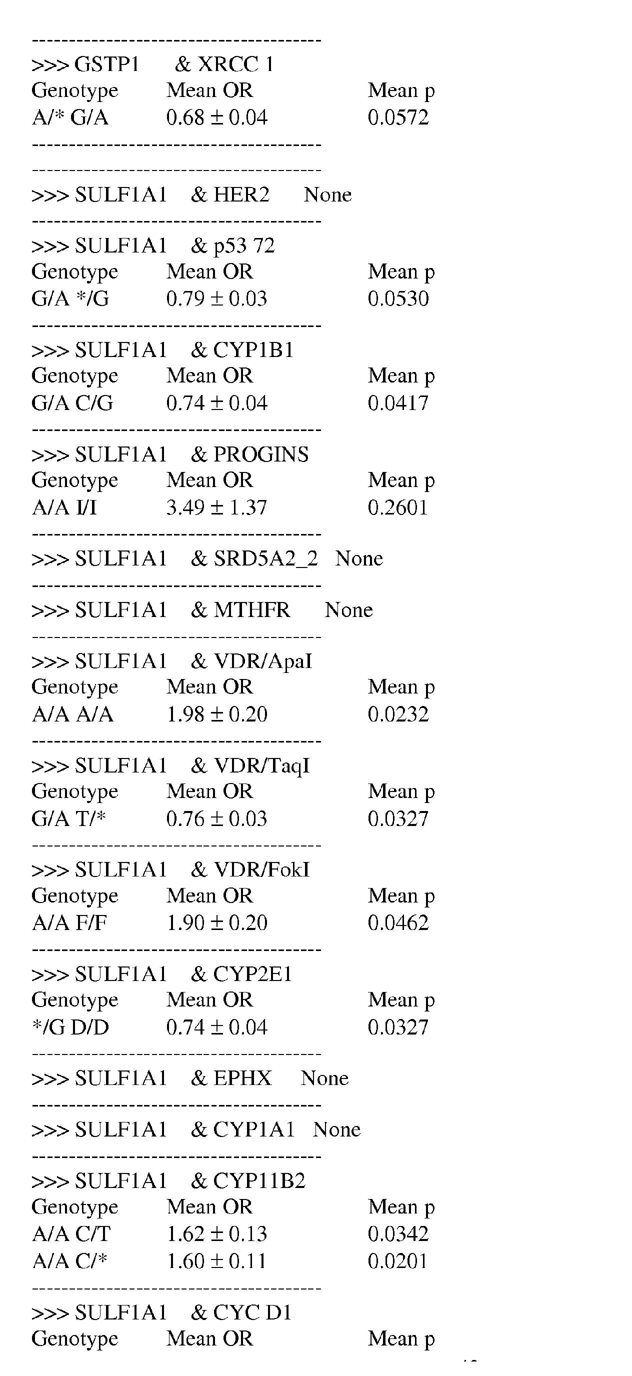

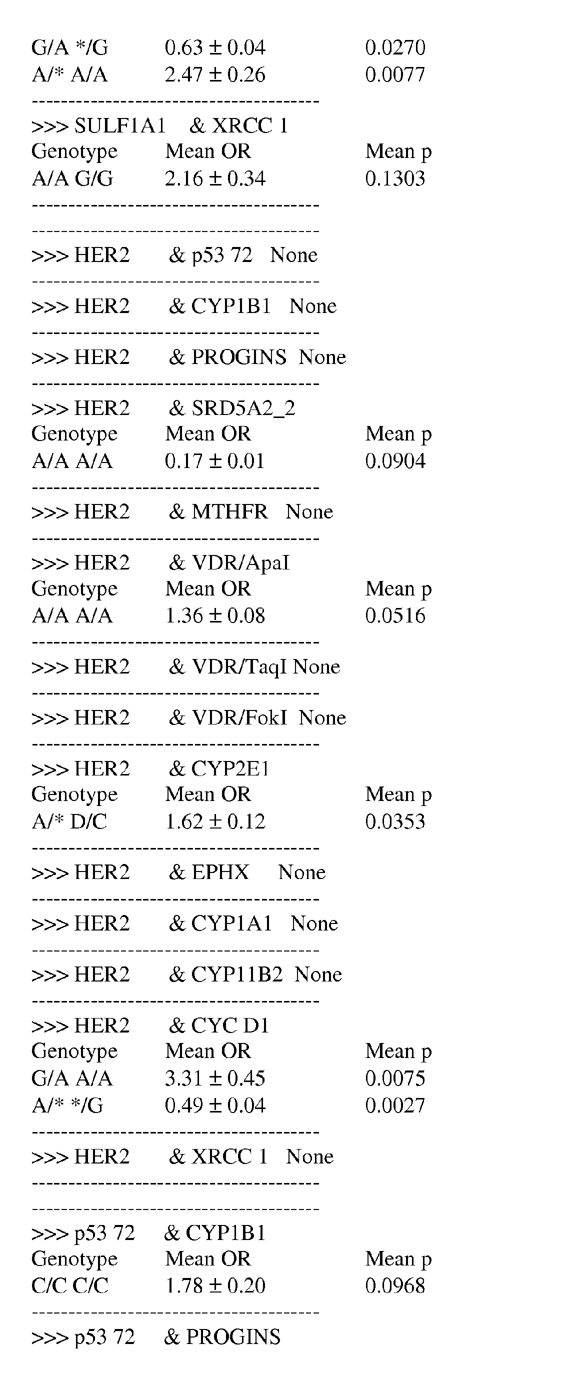

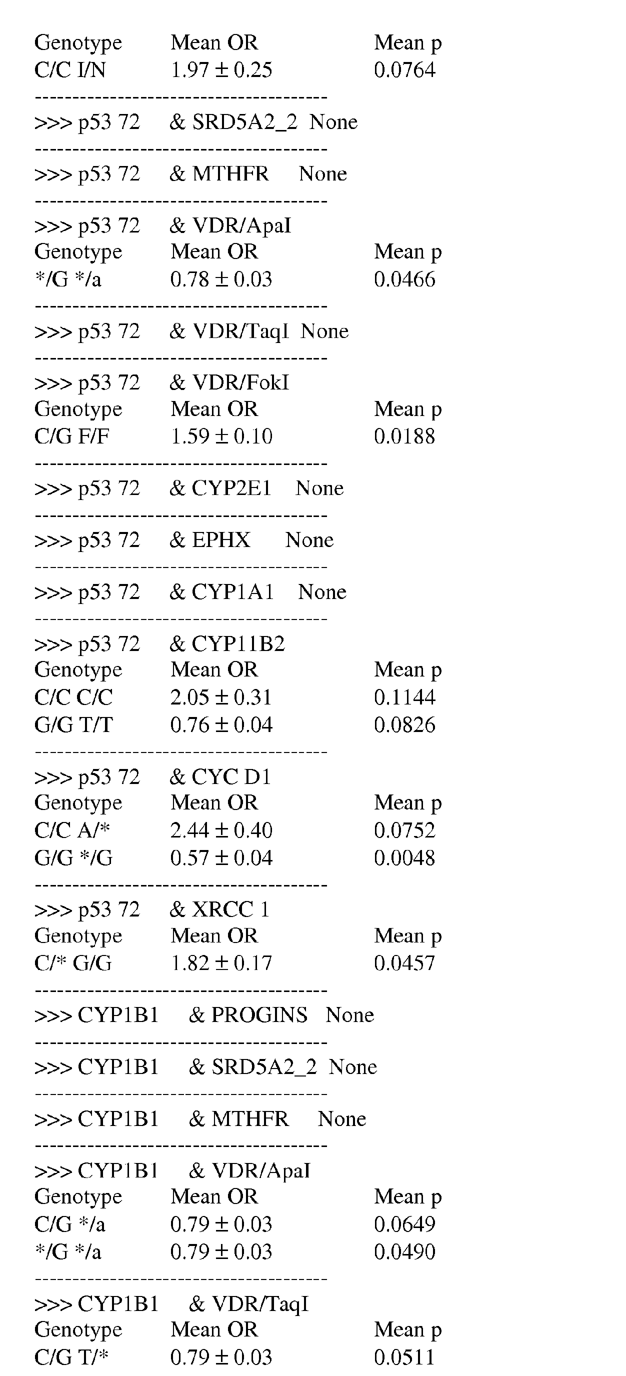

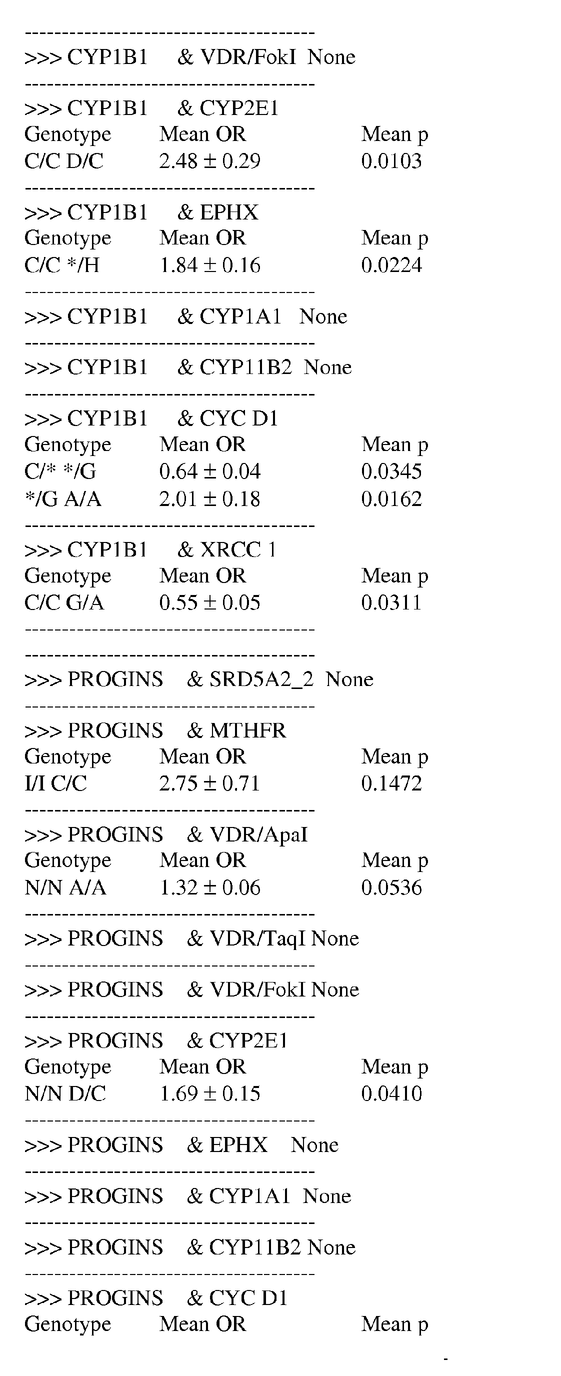

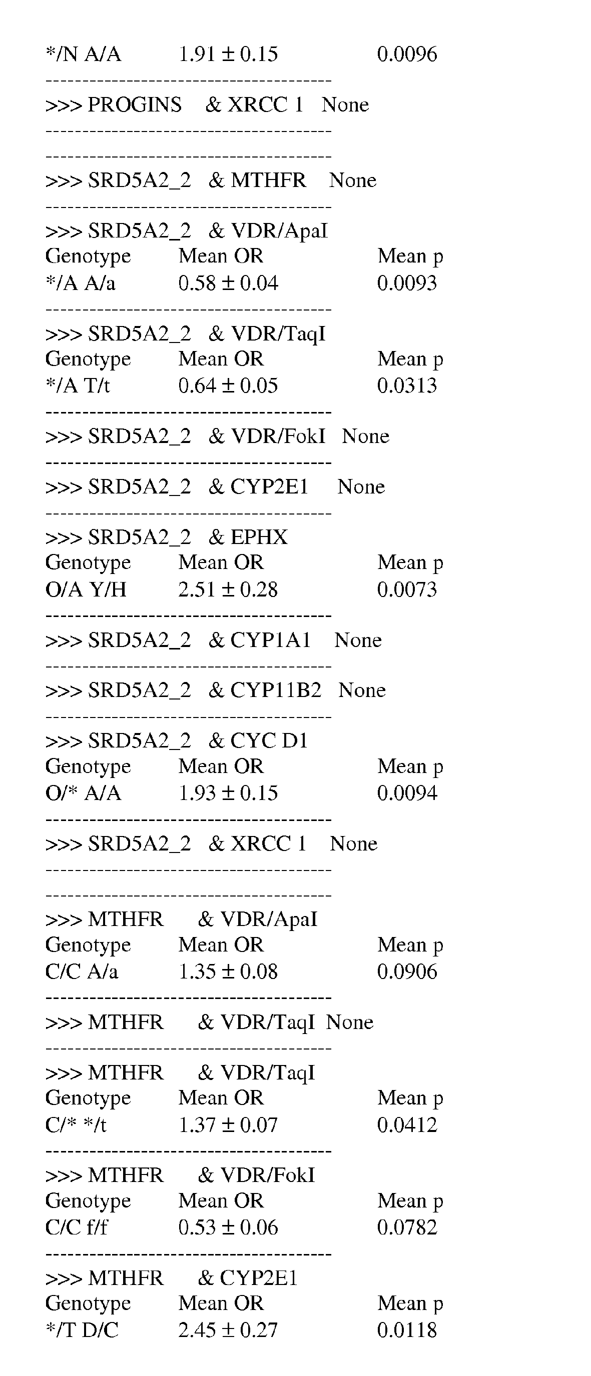

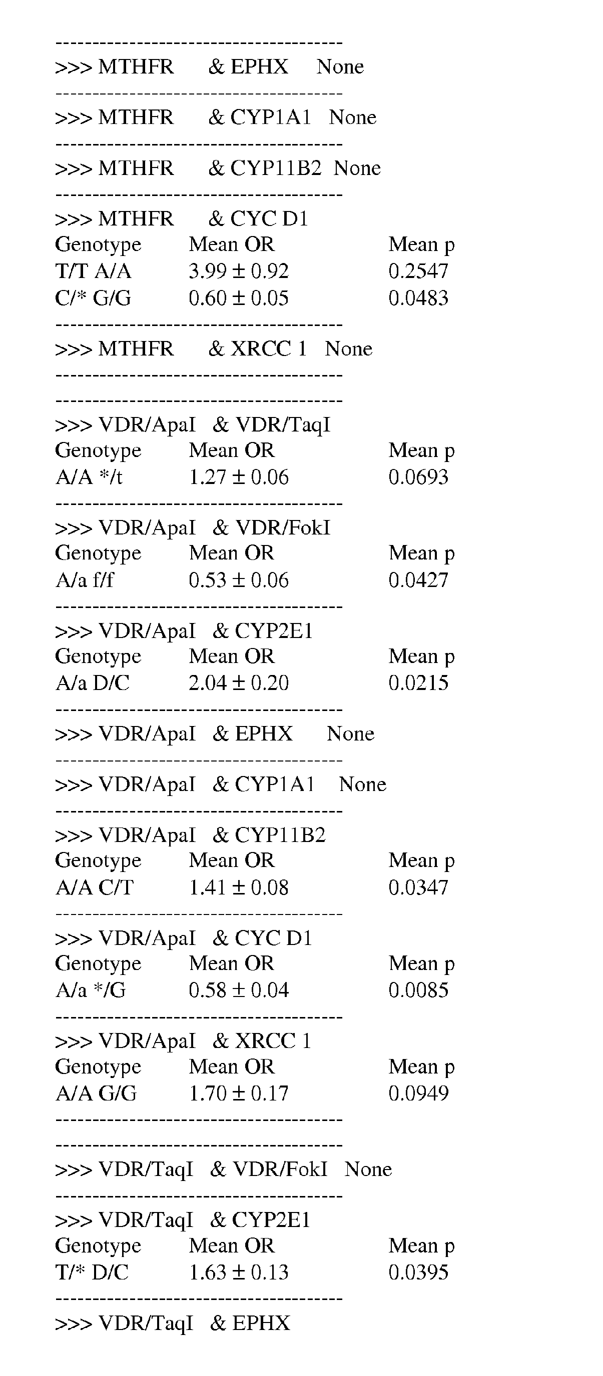

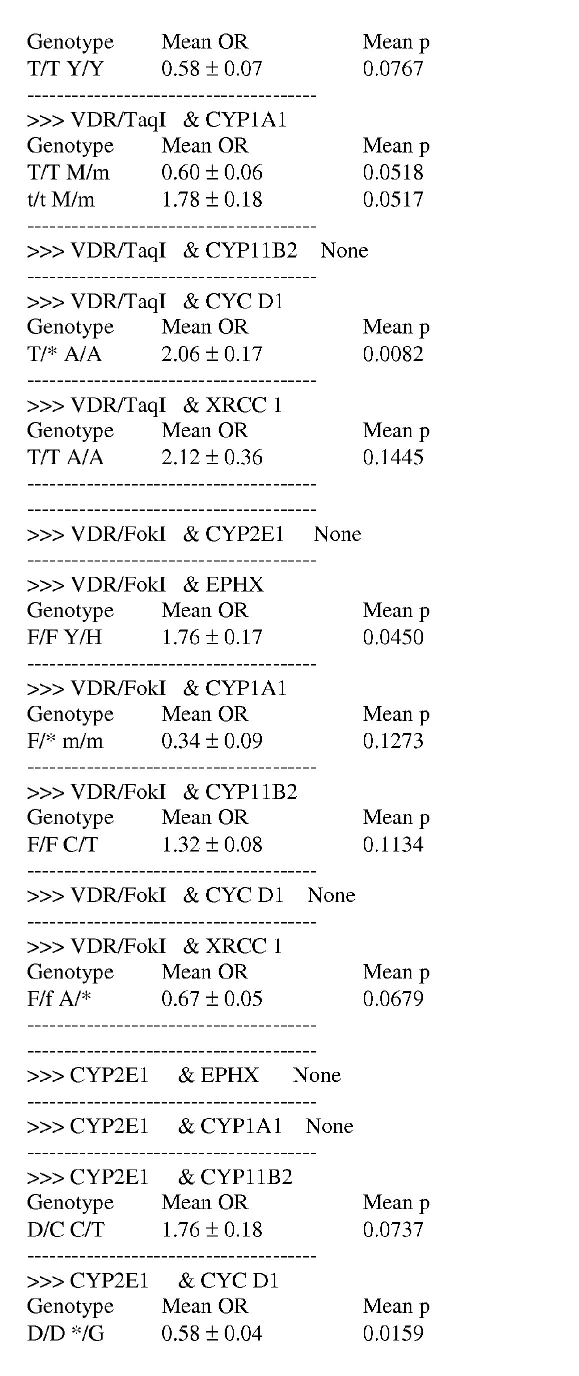

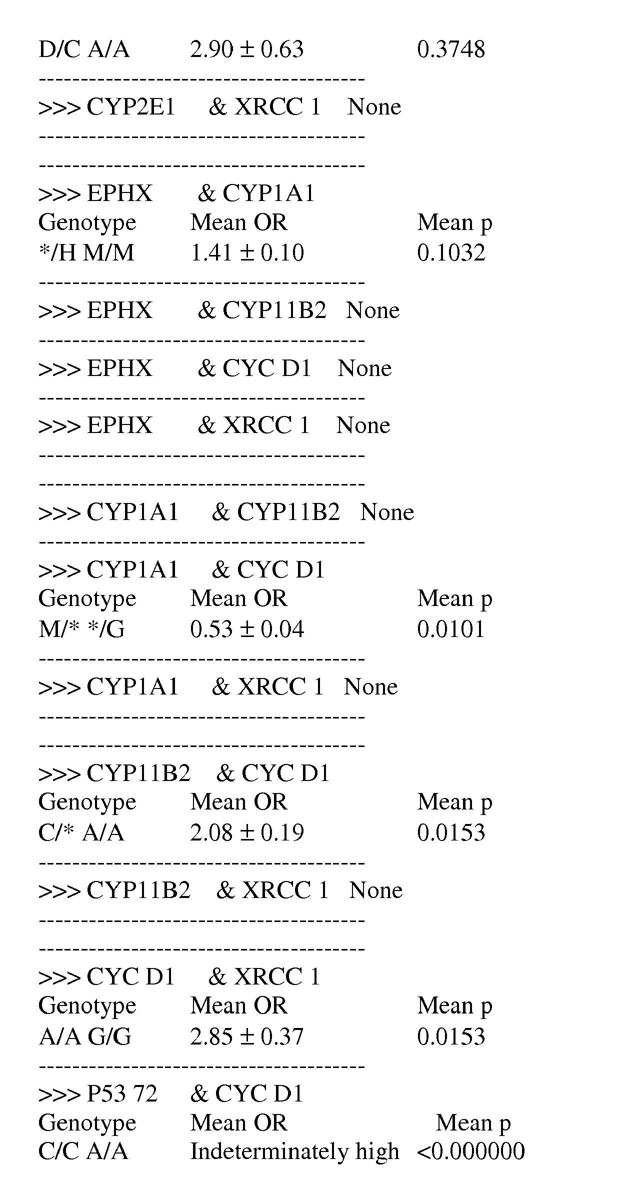

An allelic designation of "*" indicates that the allele can be either of the two possible alleles. For example, */T means T/T and C/T for the Cyp17 polymorphism.

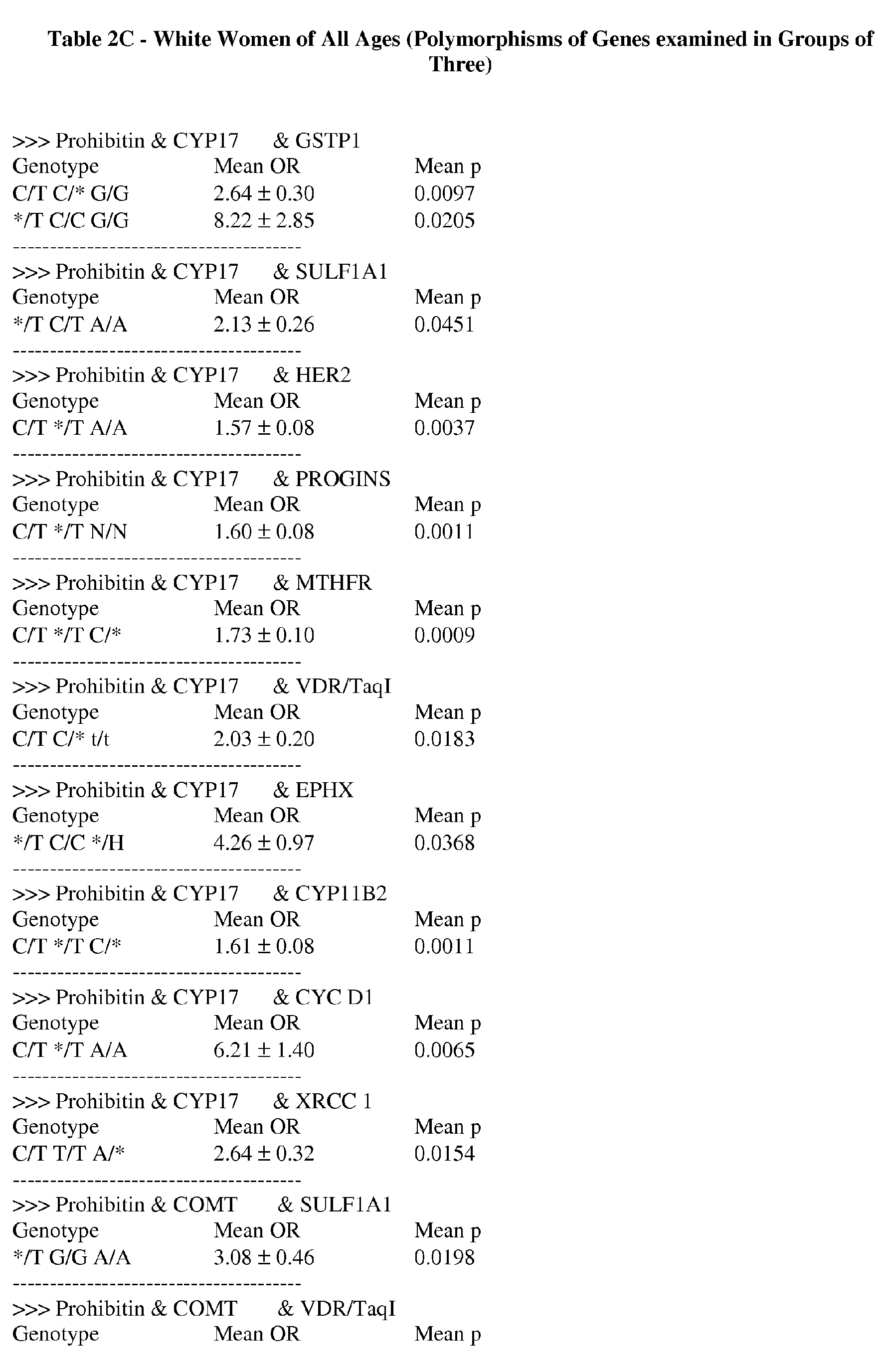

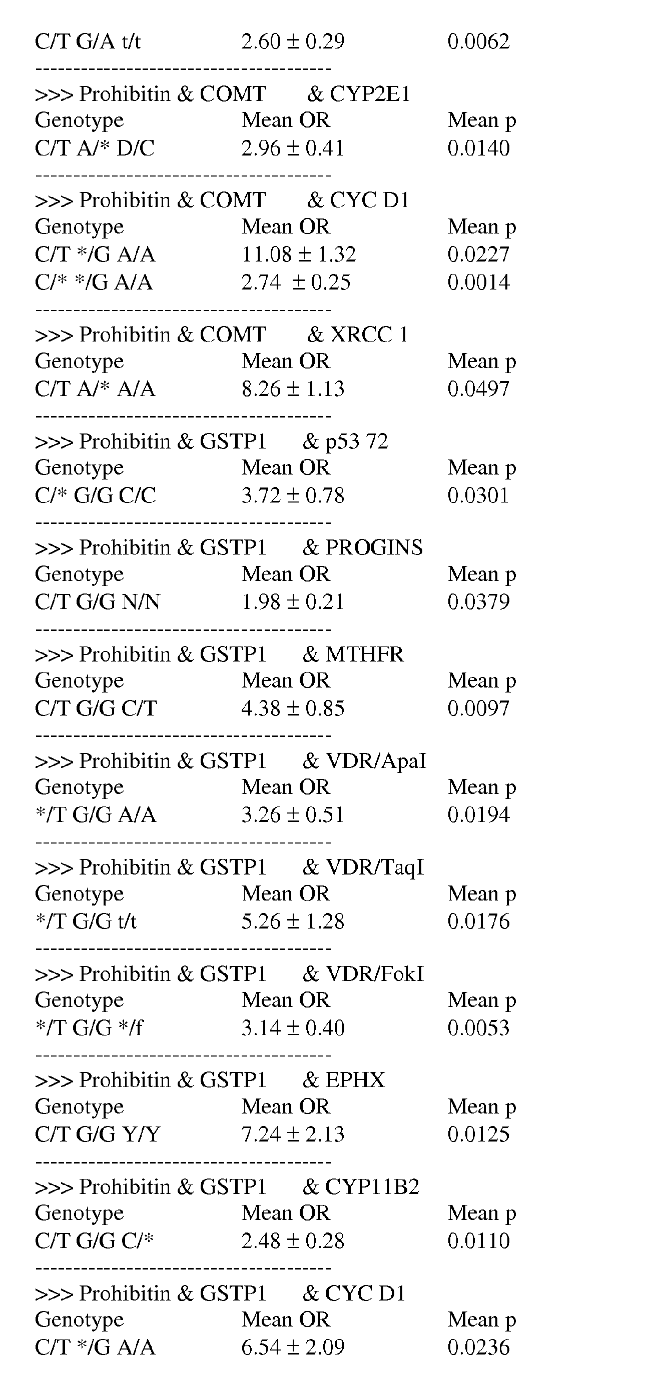

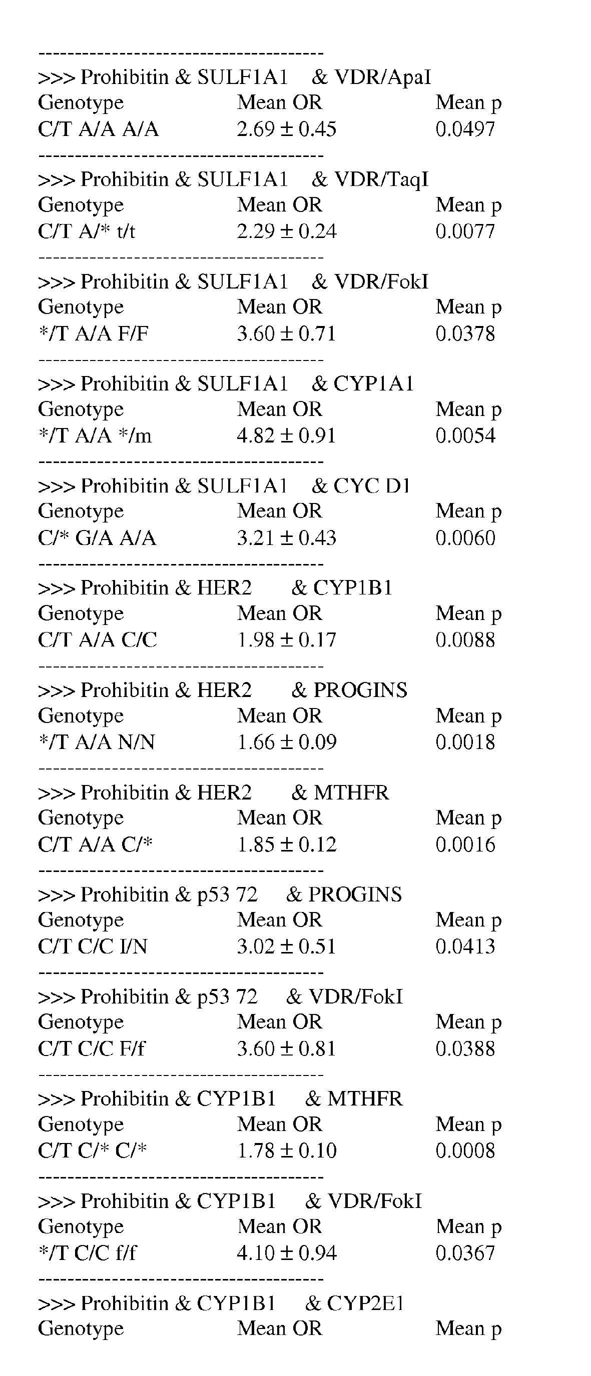

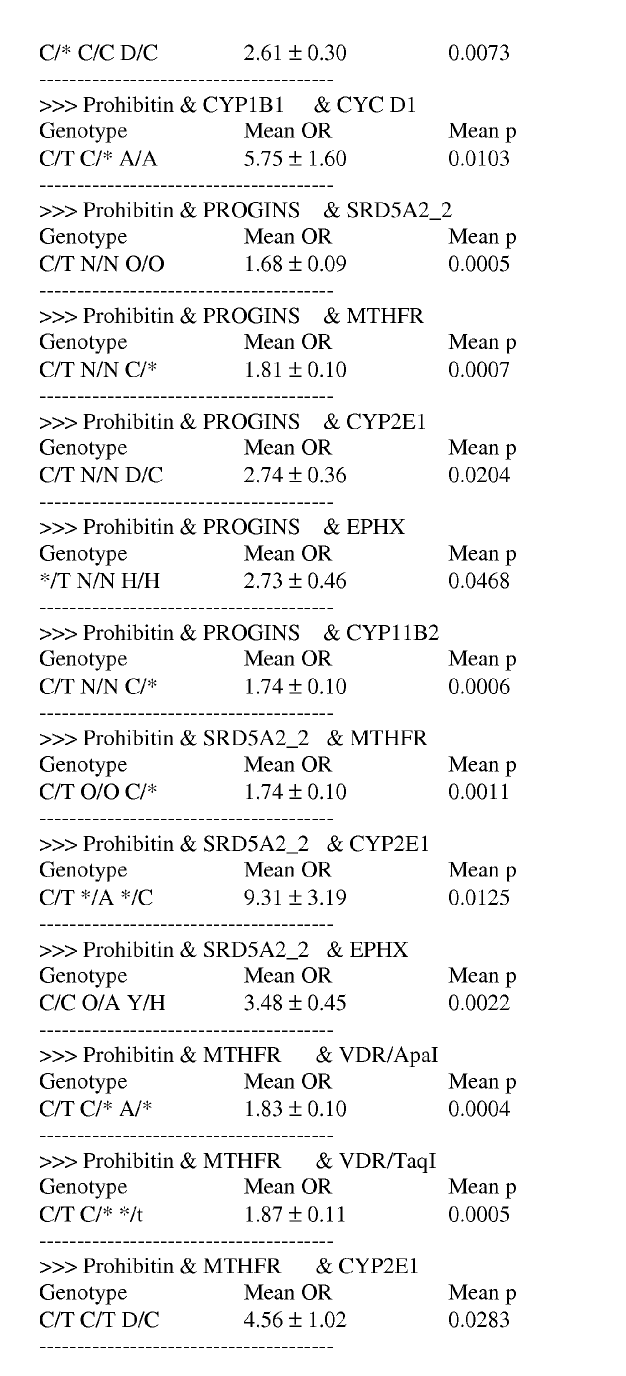

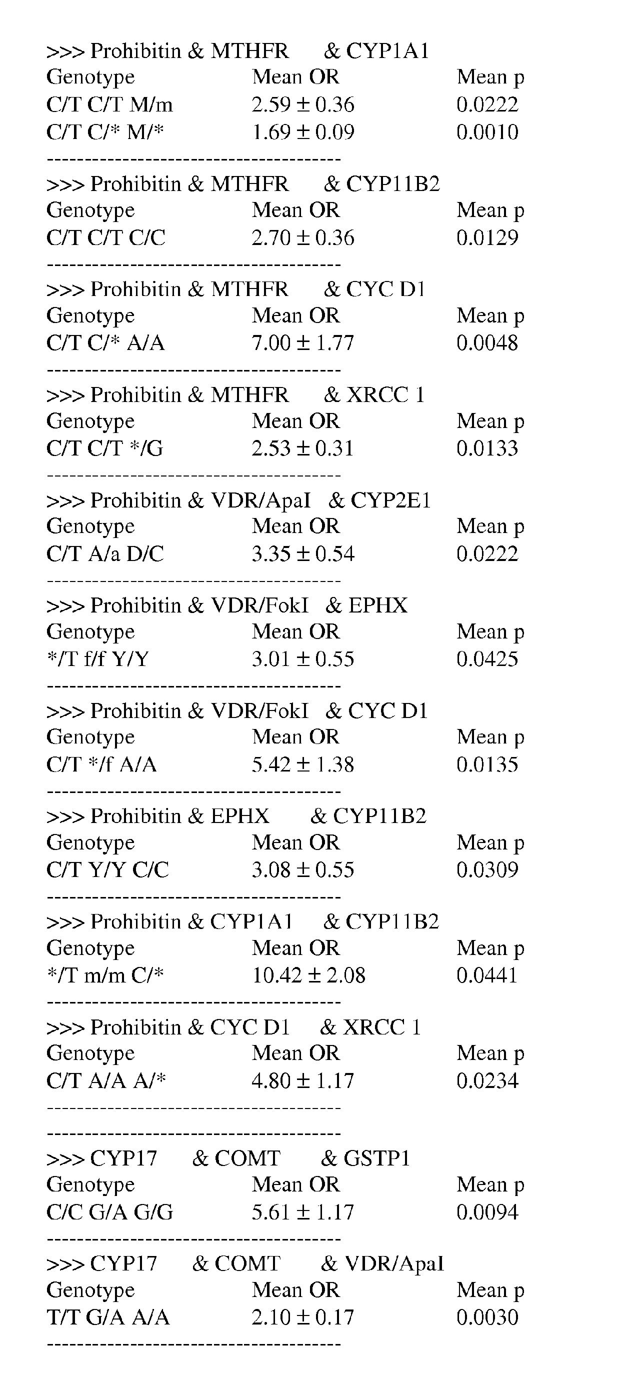

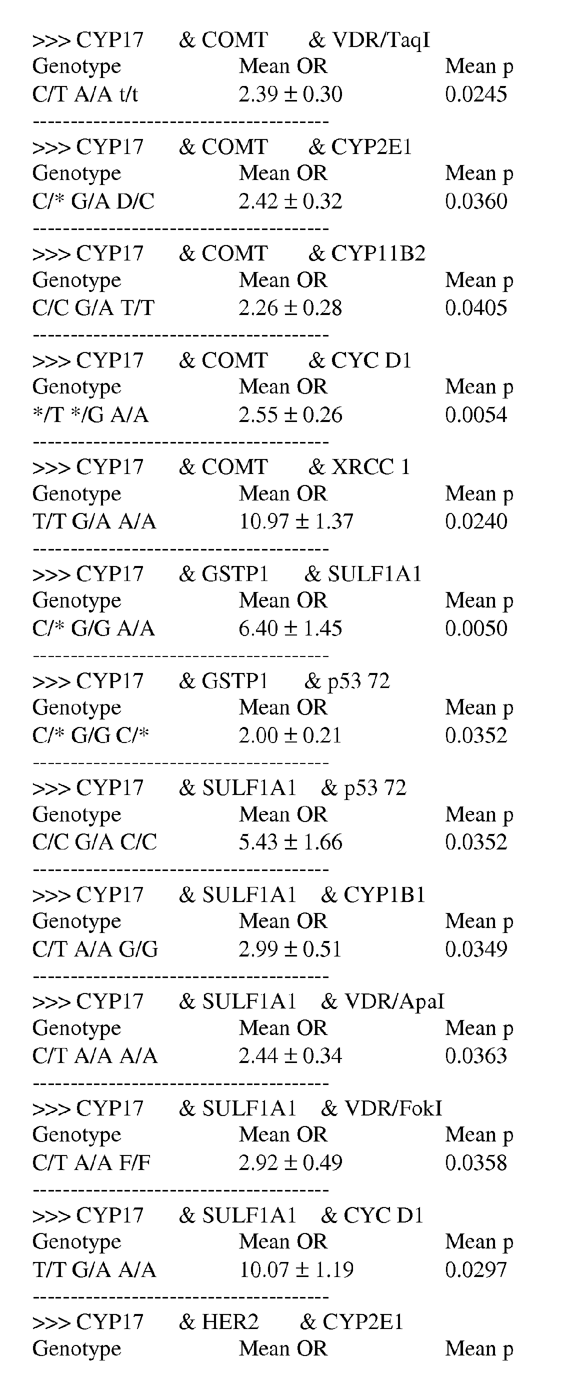

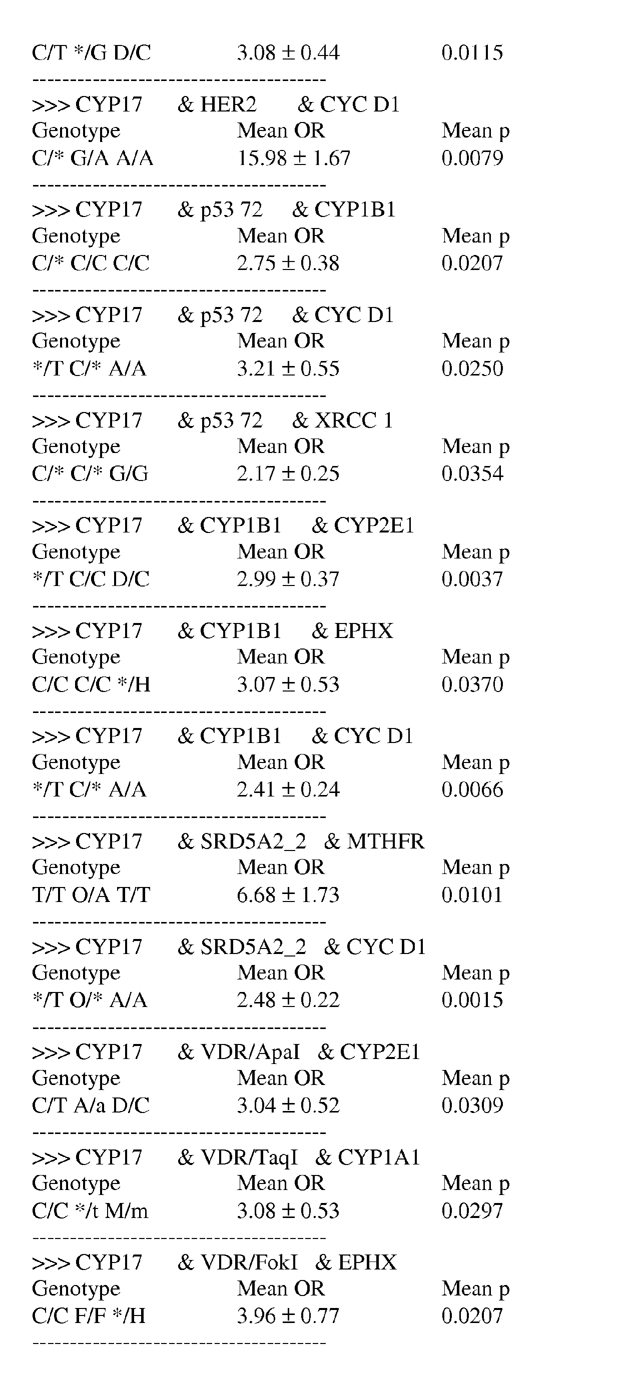

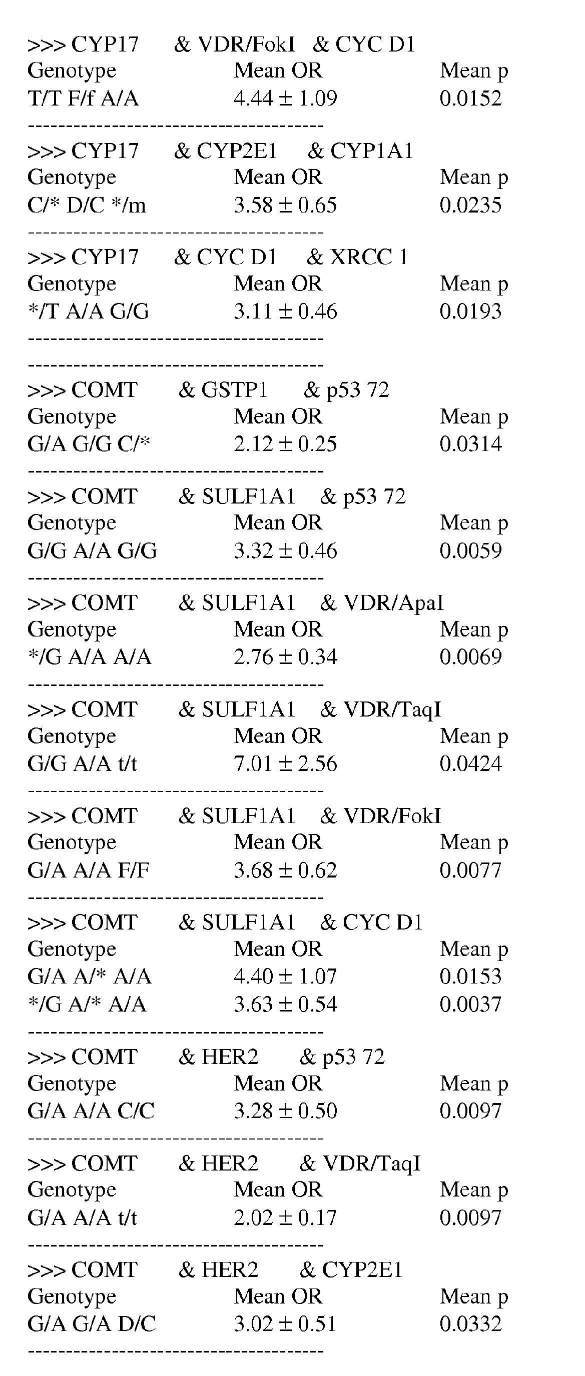

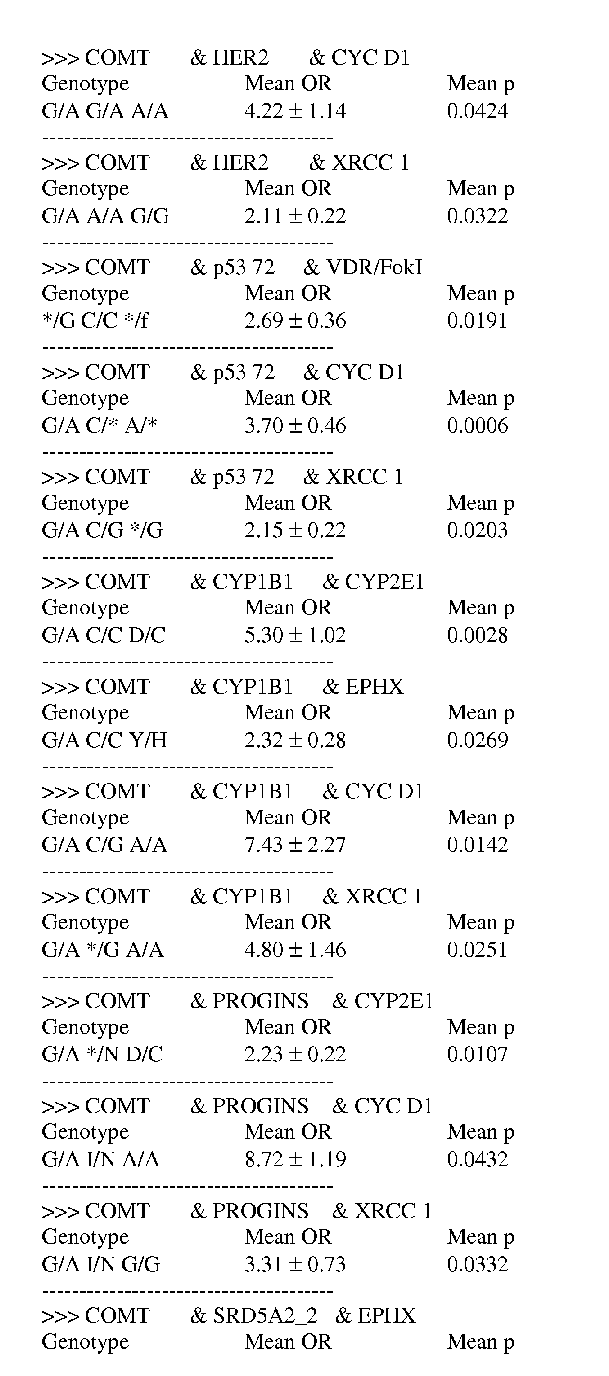

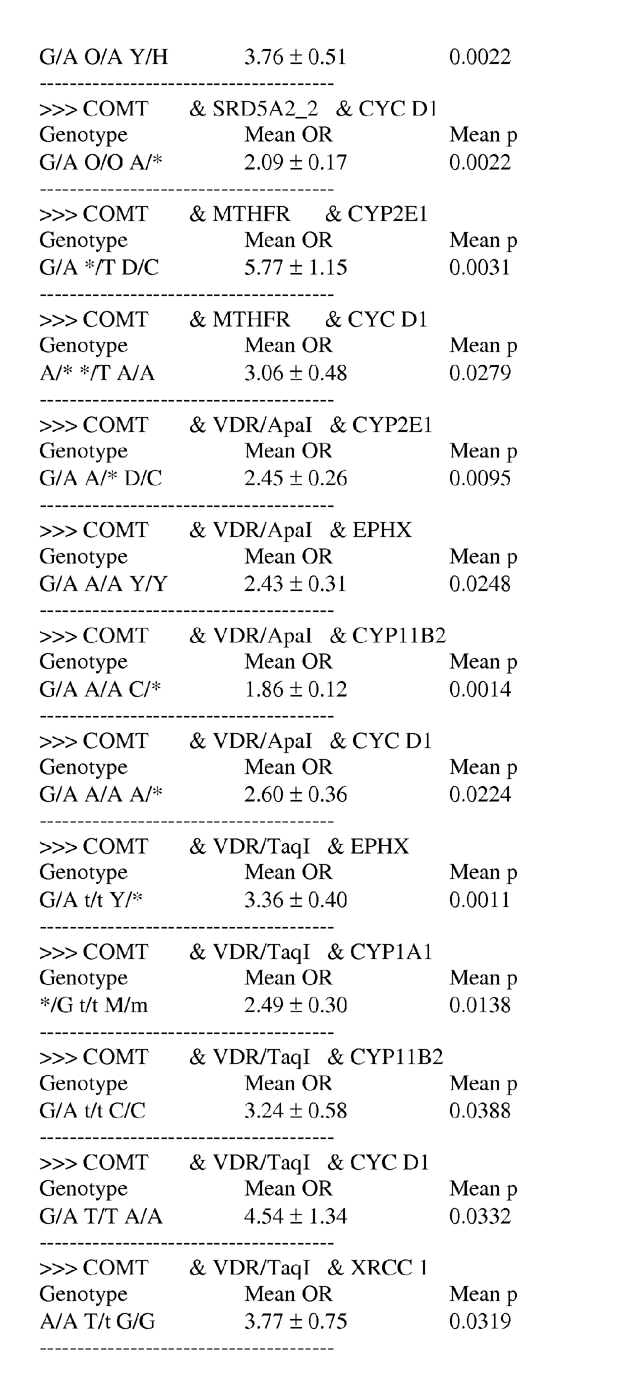

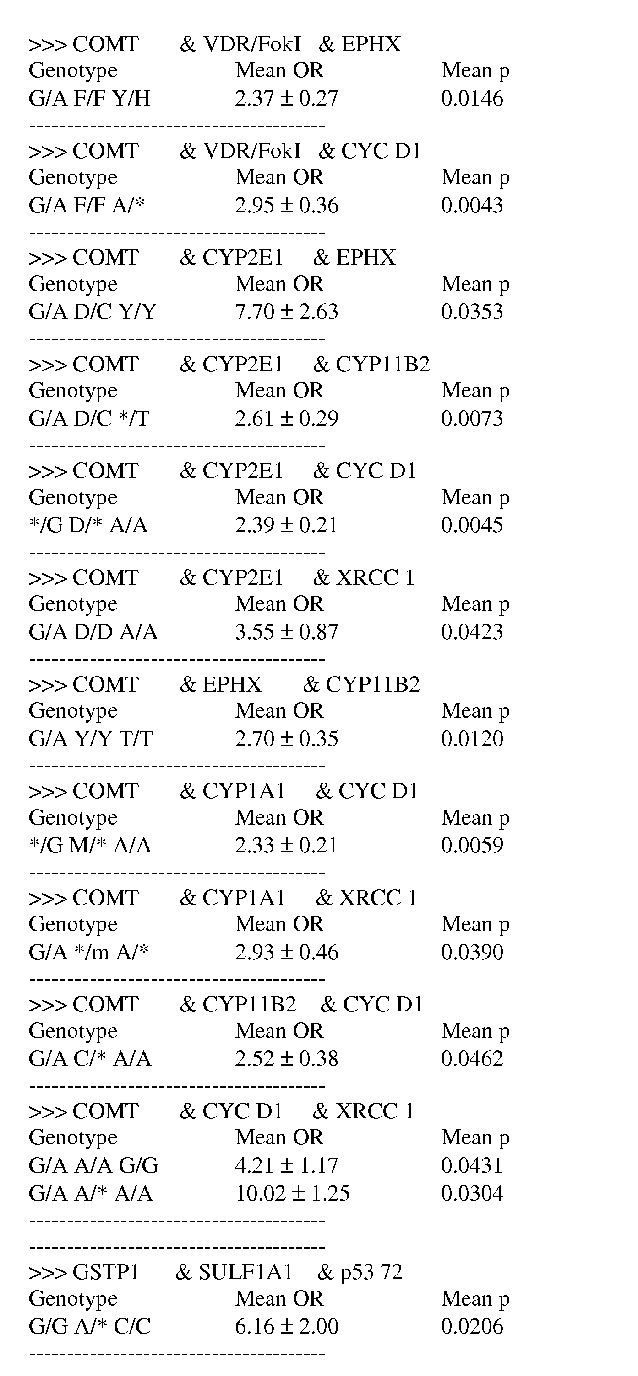

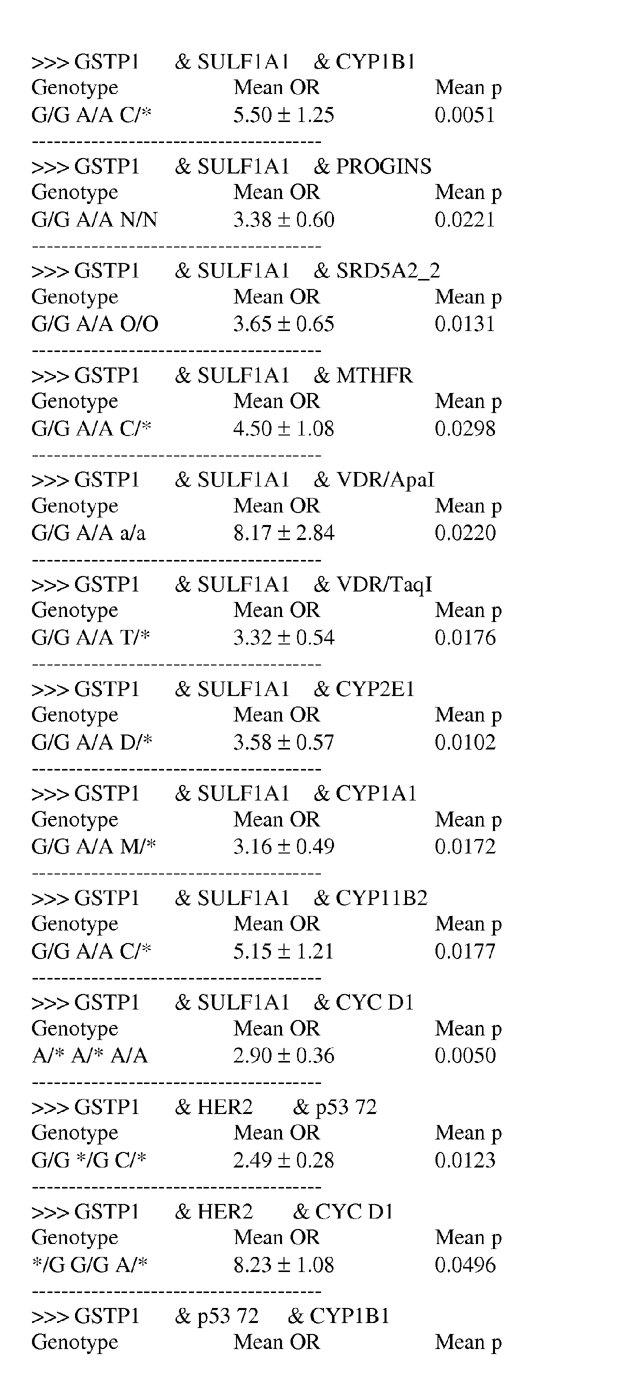

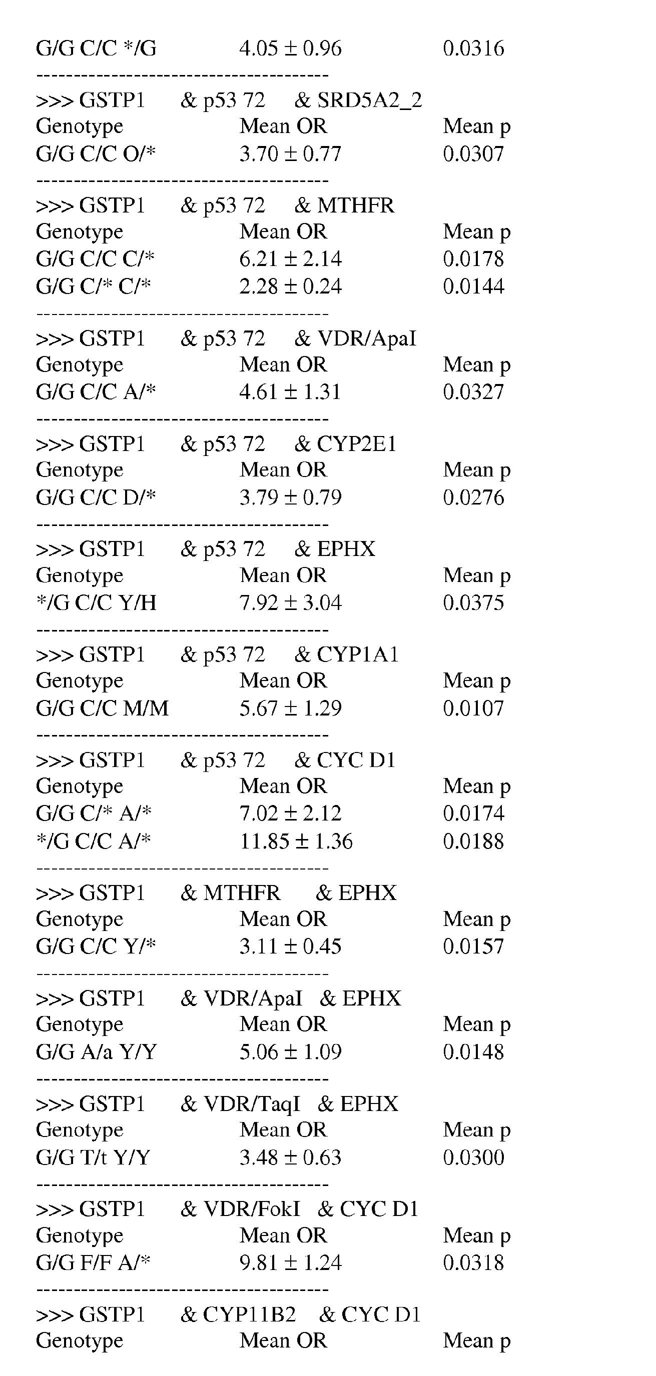

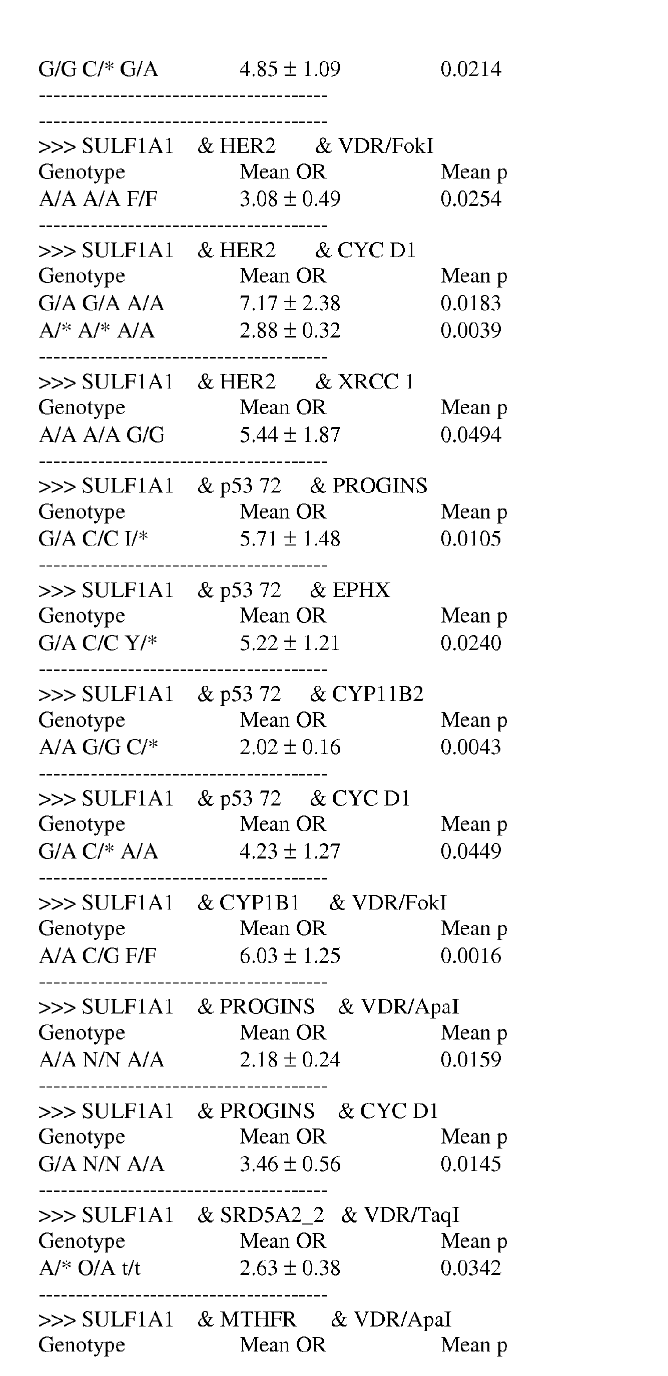

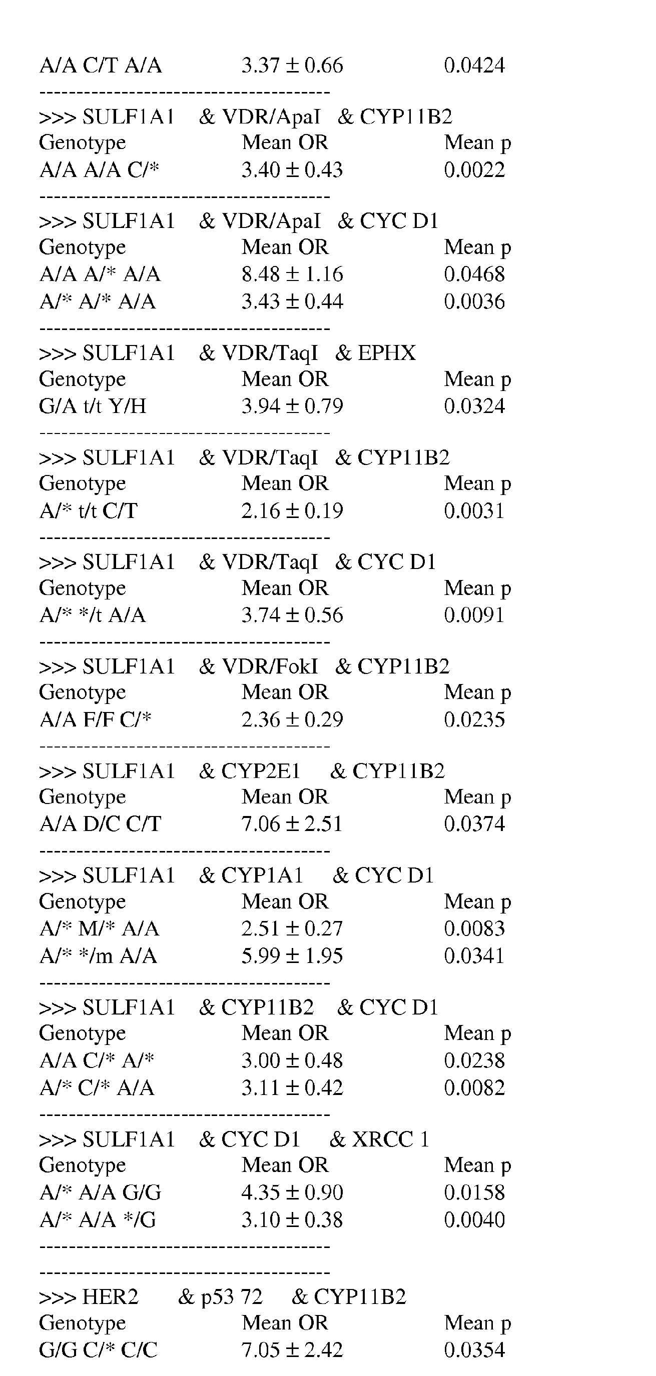

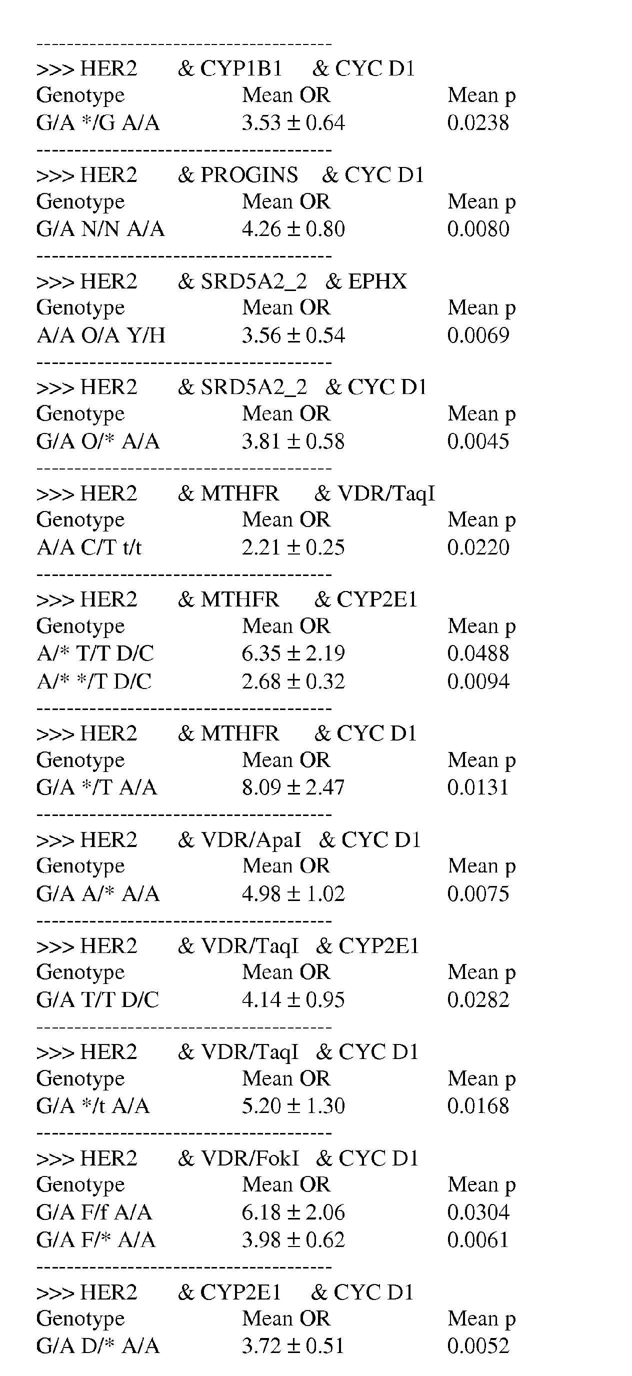

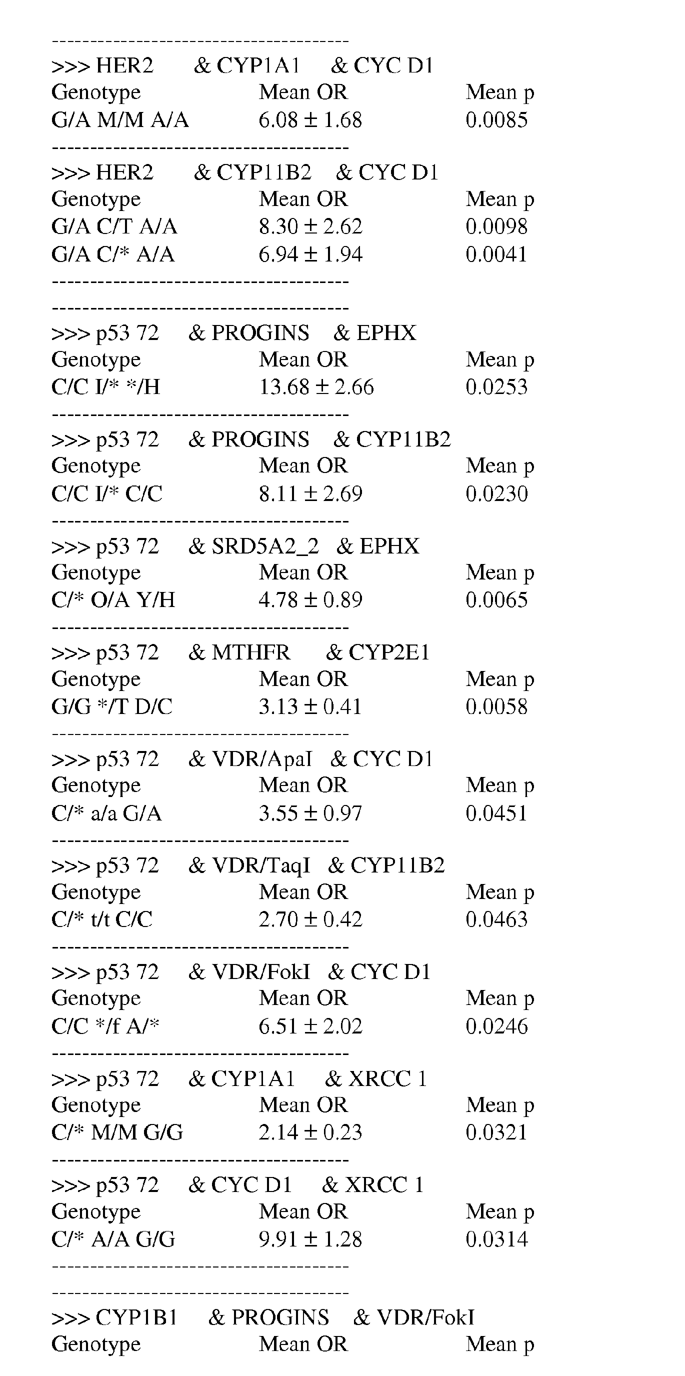

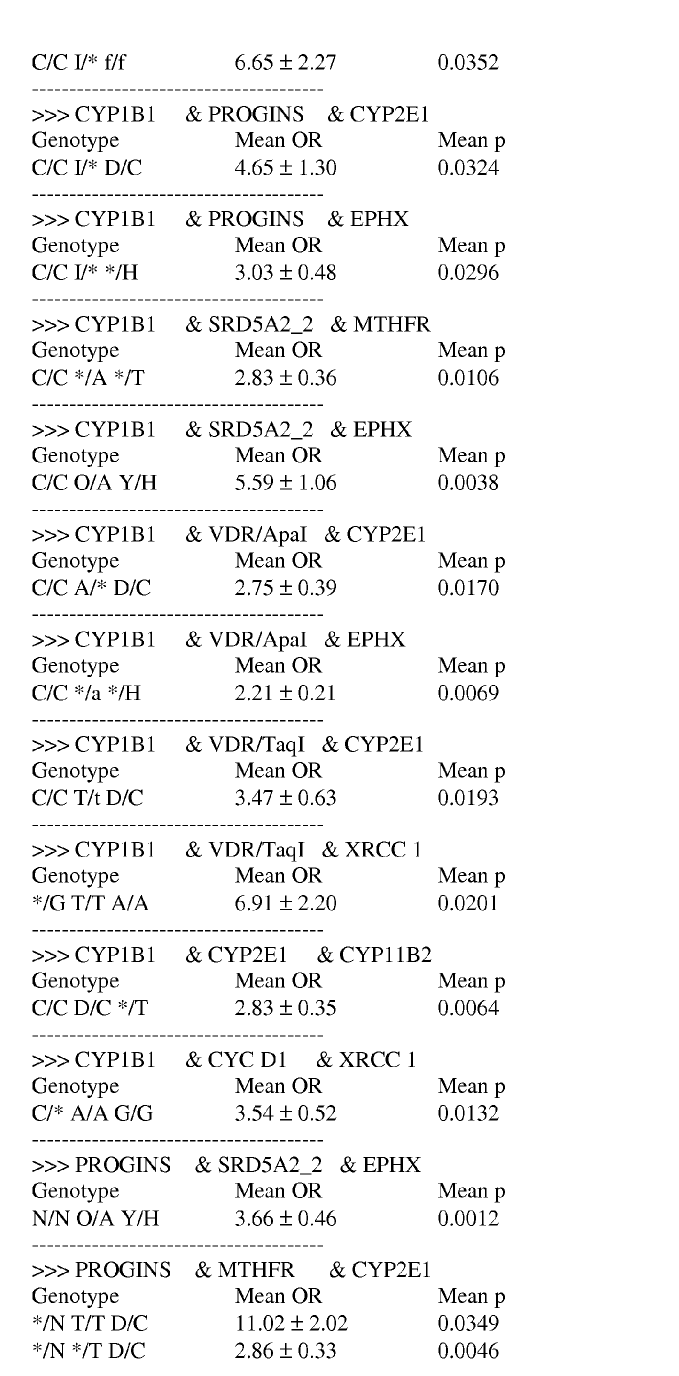

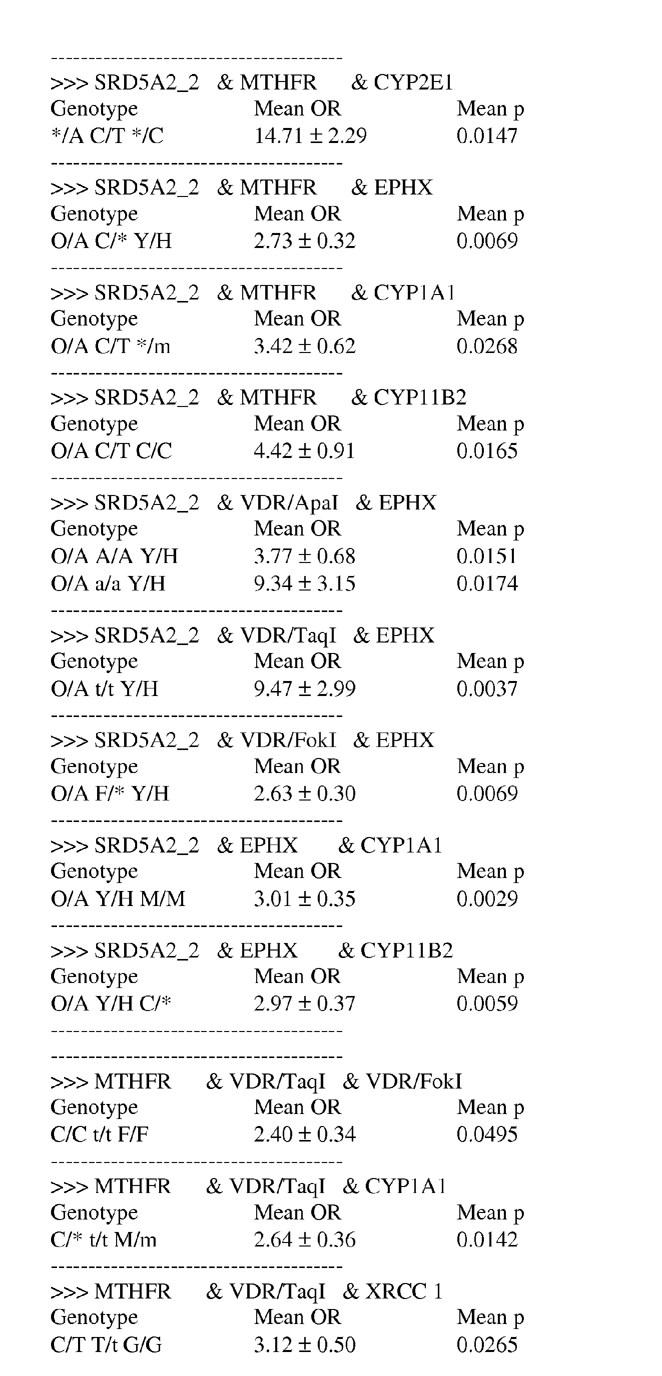

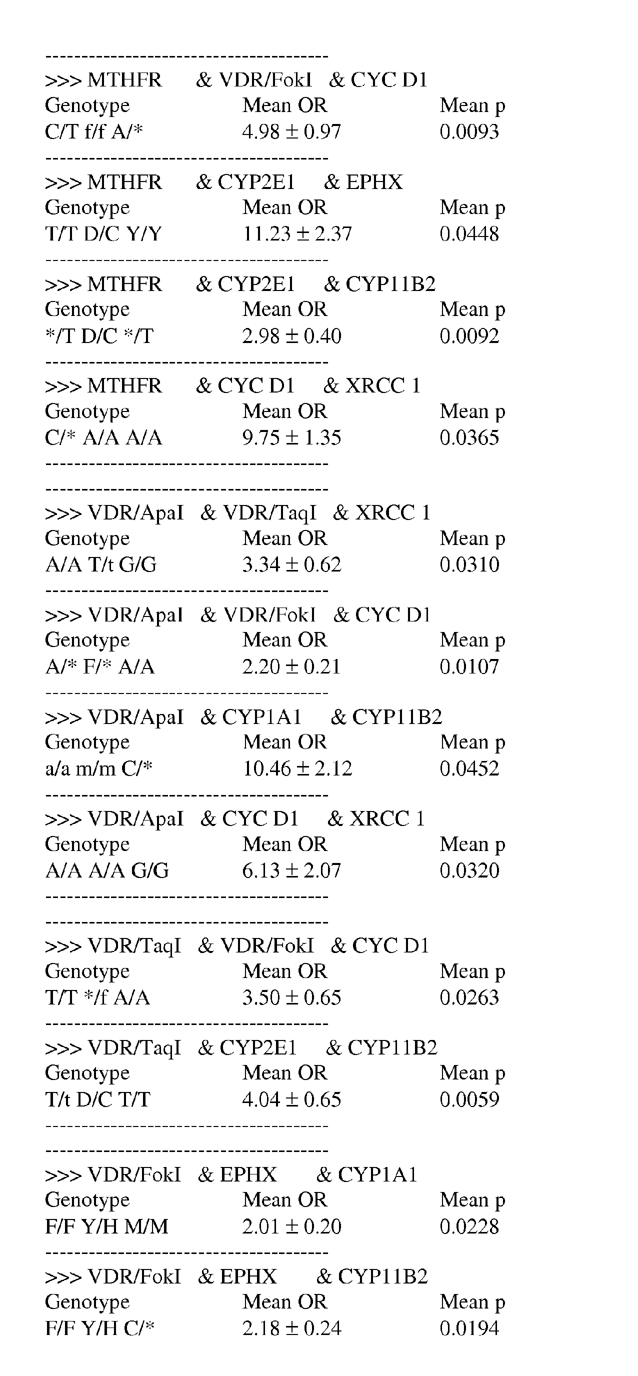

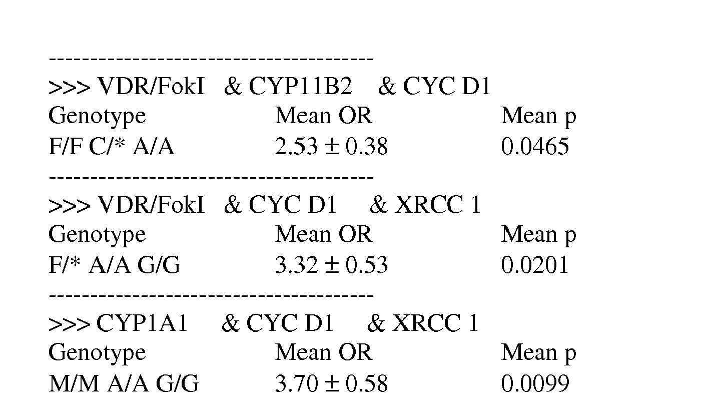

An allelic designation of "*" indicates that the allele can be either of the two possible alleles. For example, */T means T/T and C/T for the Cyp17 polymorphism.

### EXAMPLE 3 - RESULTS: AGE STRATIFIED BELOW 54

In addition to the analyses discussed above, further analyses were performed to stratify the breast cancer cases by age of diagnosis. Stratifying by age is the first example of using a personal history measure with genetic analysis to more accurately estimate an individual's cancer risk. Stratifying by age made an important difference in which combinations of genes were important for estimating risk from breast cancer.

The results presented in Tables 3A-B are a synthesis of a complex bootstrap analysis performed many different ways. The data set used in this analysis consisted of nearly 340 women that have been diagnosed with breast cancer and approximately 900 women who had never been diagnosed with any cancer. All women in this analysis were under the age of 54 when they were diagnosed with breast cancer or, if cancer free, at the time that their DNA was collected for this study. Twenty different genetic polymorphisms were examined. In general, when examined singly (one at a time), these polymorphism were weakly associated with risk of a breast cancer diagnosis. As a group, they may be termed common risk alleles with low penetrance or no penetrance for the breast cancer phenotype. The surprising observation made during this study was that when examined in combination (in pairs or in combinations of three or more), complex genotypes were defined that exhibited higher risk or higher penetrance for a breast cancer diagnosis. Table 3A, presents results for these polymorphisms examined singly. Table 3B presents results for analysis of these same polymorphisms examined in pairs or in combinations of three respectively. Examining these polymorphisms in combinations of two (pairs) is more informative for estimating breast cancer risk than examining polymorphisms alone. These gene pairs can then form the building blocks for identifying complex genotypes involving three or more polymorphic genes with greatly improved discriminatory accuracy for stratifying a woman's individual risk of being diagnosed with breast cancer. The inventors show that by starting with these building blocks of gene pairs and then adding information from other genes, one can stratify an individual's risk of being diagnosed with breast cancer over a wide range of relative risk. This range extends from less than average risk to many times average risk. The tables show the gene or genes being examined and the genotypes of these genes being compared. The tables also show the mean Odds Ratio (OR) or relative risk with the respective genotypes compared to an individual with average risk. The OR is shown as a mean because it is the composite or aggregate result of many analyses and represents the best estimate of the true OR in the general population. An OR less than one represents less than average risk while an OR greater than one represents greater than average risk. The mean p value is a measure statistical significance based on an average of Chi² tests of the ORs in used to determine the mean OR. By convention, p values ≤ 0.05 are considered to be statistically significant. It is a general observation of this analysis that the ORs and associated p values for the genetic polymorphisms examined by themselves have very limited ability to stratify risk and have p values typically greater than 0.05. In pairs, the polymorphisms stratify risk over a greater range and have much lower (more significant) p values. This trend continues as additional genes are added to the pairs with even greater stratification of risk and much smaller p values.

**Table 3A - White Women Under 54 Years of Age (Polymorphisms of Genes examined Singly)**

| >>> Prohibitin | | |
|---|---|---|
| Genotype | Mean OR | Mean p |
| C/C | 0.66 ± .03 | 0.0055 |
| C/T | 1.57 ± .08 | 0.0026 |
| T/T | 0.78 ± .12 | 0.5826 |
| | | |

| >>> CYP17 | | |
|---|---|---|
| Genotype | Mean OR | Mean p |
| C/C | 1.02 ±.07 | 0.7768 |
| C/T | 1.01 ±.05 | 0.7712 |
| T/T | 0.98 ± .05 | 0.7675 |
| | | |

| >>> COMT | | |
|---|---|---|
| Genotype | Mean OR | Mean p |
| A/A | 0.73 ± .04 | 0.0646 |
| G/A | 1.26 ±.06 | 0.1114 |
| G/G | 1.01 ± .06 | 0.7830 |
| | | |

| >>> GSTP1 | | |
|---|---|---|
| Genotype | Mean OR | Mean p |
| A/A | 0.97 ± .05 | 0.7468 |
| G/A | 0.98 ± .05 | 0.7567 |
| G/G | 1.14 ± .08 | 0.5468 |
| | | |

| >>> SULF1A1 | | |
|---|---|---|
| Genotype | Mean OR | Mean p |
| A/A | 1.24 ± .09 | 0.3113 |
| G/A | 0.74 ± .04 | 0.0419 |
| G/G | 1.21 ± .06 | 0.1911 |
| | | |

| >>> HER2 | | |
|---|---|---|
| Genotype | Mean OR | Mean p |
| A/A | 1.03 ± .05 | 0.7691 |
| G/A | 0.93 ± .05 | 0.6095 |
| G/G | 1.20 ± .12 | 0.5315 |
| | | |

| >>> p53 72 | | |
|---|---|---|
| Genotype | Mean OR | Mean p |
| C/C | 1.18 ± .11 | 0.5471 |
| C/G | 1.09 ± .05 | 0.5699 |
| G/G | 0.89 ± .04 | 0.4058 |
| | | |

| >>> CYP1B1 | | |
|---|---|---|
| Genotype | Mean OR | Mean p |
| C/C | 1.01 ±.05 | 0.7825 |
| C/G | 0.91 ±.05 | 0.5167 |
| G/G | 1.14 ± .07 | 0.4677 |
| | | |

| >>> PROGINS | | |
|---|---|---|
| Genotype | Mean OR | Mean p |
| I/I | 1.17 ± .19 | 0.7026 |
| I/N | 0.93 ± .05 | 0.6174 |
| N/N | 1.06 ± .06 | 0.6680 |
| | | |

| >>> SRD5A2_2 | | |
|---|---|---|
| Genotype | Mean OR | Mean p |
| O/O | 1.21 ± .07 | 0.2941 |
| O/A | 0.86 ± .05 | 0.4249 |
| A/A | 0.52 ± .12 | 0.3581 |
| | | |

| >>> MTHFR | | |
|---|---|---|
| Genotype | Mean OR | Mean p |
| C/C | 1.10 ± .06 | 0.6093 |
| C/T | 1.03 ± .06 | 0.7707 |
| T/T | 0.72 ± .07 | 0.2905 |
| | | |

| >>> VDR/ApaI | | |
|---|---|---|
| Genotype | Mean OR | Mean p |
| A/A | 1.25 ± .07 | 0.1672 |
| A/a | 0.82 ± .04 | 0.1773 |
| a/a | 1.01 ± .06 | 0.7935 |
| | | |

| >>> VDR/TaqI | | |
|---|---|---|
| Genotype | Mean OR | Mean p |
| T/T | 1.03 ± .05 | 0.7753 |
| T/t | 0.94 ± .05 | 0.6526 |
| t/t | 1.06 ±.07 | 0.6851 |
| | | |

| >>> VDR/FokI | | |
|---|---|---|
| Genotype | Mean OR | Mean p |
| F/F | 1.08 ± .06 | 0.6391 |
| F/f | 0.91 ± .05 | 0.5788 |
| f/f | 1.05 ± .08 | 0.7567 |
| | | |

| >>> CYP2E1 | | |
|---|---|---|
| Genotype | Mean OR | Mean p |
| D/D | 0.92 ± .08 | 0.6930 |
| D/C | 1.23 ± .12 | 0.4747 |
| C/C | 0.49 ± .13 | 0.3935 |
| | | |

| >>> EPHX | | |
|---|---|---|
| Genotype | Mean OR | Mean p |
| Y/Y | 0.74 ± .07 | 0.2551 |
| Y/H | 1.29 ± .12 | 0.3504 |
| H/H | 1.13 ± .13 | 0.6826 |
| | | |

| >>> CYP1A1 | | |
|---|---|---|
| Genotype | Mean OR | Mean p |
| M/M | 1.04 ± .06 | 0.7474 |
| M/m | 1.00 ± .06 | 0.7824 |
| m/m | 0.62 ± .15 | 0.4939 |
| | | |

| >>> CYP11B2 | | |
|---|---|---|
| Genotype | Mean OR | Mean p |
| C/C | 0.86 ± .05 | 0.4214 |
| C/T | 1.07 ± .05 | 0.6368 |
| T/T | 1.03 ±.06 | 0.7424 |
| | | |

| >>> CYC D 1 | | |
|---|---|---|
| Genotype | Mean OR | Mean p |
| A/A | 2.27 ± .25 | 0.0097 |
| G/A | 0.86 ± .07 | 0.5359 |
| G/G | 0.64 ± .05 | 0.0997 |
| | | |

| >>> XRCC 1 | | |
|---|---|---|
| Genotype | Mean OR | Mean p |
| A/A | 1.32 ± .16 | 0.4476 |
| G/A | 0.69 ± .06 | 0.1335 |
| G/G | 1.29 ± .10 | 0.3131 |
| | | |

**Table 3B - White Women Under 54 Years of Age Polymorphisms of Genes examined in pairs (two at a time)**

| >>> Prohibitin & CYP17 | | |
|---|---|---|
| Genotype | Mean OR | Mean p |
| C/C */T | 0.65 ± .03 | 0.0029 |
| C/T */T | 1.72 ± .09 | 0.0006 |
| | | |

| >>> Prohibitin & COMT | | |
|---|---|---|
| Genotype | Mean OR | Mean p |
| C/T */G | 1.71 ± .09 | 0.0010 |

| >>> Prohibitin & GSTP1 | | |
|---|---|---|
| Genotype | Mean OR | Mean p |
| C/T G/G | 2.19 ± .24 | 0.0229 |
| | | |

| >>> Prohibitin & SULF1A1 | | |
|---|---|---|
| Genotype | Mean OR | Mean p |
| C/C G/A | 0.64 ± .04 | 0.0106 |
| C/T A/A | 1.97 ± .21 | 0.0455 |
| | | |

| >>> Prohibitin & HER2 | | |
|---|---|---|
| Genotype | Mean OR | Mean p |
| C/C A/* | 0.67 ± .03 | 0.0070 |
| C/T A/A | 1.51 ± .09 | 0.0227 |
| | | |

| >>> Prohibitin & p53 72 | | |
|---|---|---|
| Genotype | Mean OR | Mean p |
| C/C G/G | 0.64 ± .03 | 0.0031 |
| C/T C/C | 1.77 ± .24 | 0.1637 |
| C/T G/G | 1.62 ± .09 | 0.0051 |
| | | |

| >>> Prohibitin & CYP1B1 | | |
|---|---|---|
| Genotype | Mean OR | Mean p |
| C/T C/* | 1.49 ± .08 | 0.0131 |
| | | |

| >>> Prohibitin & PROGINS | | |
|---|---|---|
| Genotype | Mean OR | Mean p |
| C/C */N | 0.66 ± .03 | 0.0042 |
| | | |

| >>> Prohibitin & SRD5A2_2 | | |
|---|---|---|
| Genotype | Mean OR | Mean p |
| C/T O/O | 1.80 ± .10 | 0.0003 |
| | | |

| >>> Prohibitin & MTHFR | | |
|---|---|---|
| Genotype | Mean OR | Mean p |
| C/C */T | 0.66 ± .04 | 0.0317 |
| C/T C/* | 2.05 ± .13 | 0.0003 |
| | | |

| >>> Prohibitin & VDR/ApaI | | |
|---|---|---|
| Genotype | Mean OR | Mean p |
| C/C A/a | 0.67 ± .04 | 0.0182 |
| C/T A/A | 1.66 ± .12 | 0.0224 |
| | | |

| >>> Prohibitin & VDR/TaqI | | |
|---|---|---|
| Genotype | Mean OR | Mean p |
| C/C T/* | 0.73 ± .04 | 0.0317 |
| C/T t/t | 1.55 ± .14 | 0.1120 |
| | | |

| >>> Prohibitin & VDR/FokI | | |
|---|---|---|
| Genotype | Mean OR | Mean p |
| C/T F/* | 1.78 ± .10 | 0.0017 |
| | | |
| >>> Prohibitin & CYP2E1 None | | |
| | | |

| >>> Prohibitin & EPHX | | |
|---|---|---|
| Genotype | Mean OR | Mean p |
| C/C Y/Y | 0.53 ± .05 | 0.0343 |
| C/T H/H | 2.30 ± .45 | 0.1550 |
| */T H/H | 2.43 ±.45 | 0.1107 |
| | | |

| >>> Prohibitin & CYP1A1 | | |
|---|---|---|
| Genotype | Mean OR | Mean p |
| C/C M/* | 0.69 ± .03 | 0.0126 |
| | | |
| >>> Prohibitin & CYP11B2 None | | |
| | | |

| >>> Prohibitin & CYC D 1 | | |
|---|---|---|
| Genotype | Mean OR | Mean p |
| C/C */G | 0.47 ± .04 | 0.0026 |
| C/T A/A | 3.73 ± .95 | 0.0474 |
| | | |

| >>> Prohibitin & XRCC 1 | | |
|---|---|---|
| Genotype | Mean OR | Mean p |
| C/C G/A | 0.54 ± .05 | 0.0227 |
| C/T A/A | 3.48 ± .94 | 0.0660 |
| | | |
| >>> CYP17 & COMT | | |
| Genotype | Mean OR | Mean p |
| T/T A/A | 0.60 ± .05 | 0.0455 |
| | | |
| >>> CYP17 & GSTP1 None | | |
| | | |
| >>> CYP17 & SULF1A1 | | |
| Genotype | Mean OR | Mean p |
| C/T A/A | 1.81± .17 | 0.0339 |
| | | |
| >>> CYP17 & HER2 None | | |
| | | |
| >>> CYP17 & | p53 72 None | |
| | | |
| >>> CYP17 | & CYP1B1 | None |
| | | |
| >>> CYP17 | & PROGINS | None |
| | | |
| >>> CYP17 | & SRD5A2_2 | None |
| | | |
| >>> CYP17 | & MTHFR | None |
| | | |
| >>> CYP17 | & VDR/ApaI | None |
| | | |
| >>> CYP17 | & VDR/TaqI | None |
| | | |
| >>> CYP17 | & VDR/FokI | None |
| | | |
| >>> CYP17 | & CYP2E1 | None |
| | | |
| >>> CYP17 | & EPHX | None |
| | | |
| >>> CYP17 | & CYP1A1 | None |
| | | |
| >>> CYP17 | & CYP11B2 | None |
| | | |
| >>> CYP17 & CYC D1 | | |
| Genotype | Mean OR | Mean p |
| C/T A/A | 3.37 ± .59 | 0.0126 |
| | | |
| >>> CYP 17 & XRCC 1 | | |
| Genotype | Mean OR | Mean p |
| T/T A/A | 2.20 ± .43 | 0.1570 |
| | | |
| >>> COMT & GSTP1 | | |
| Genotype | Mean OR | Mean p |
| A/A A/* | 0.71 ± .04 | 0.0546 |
| | | |
| >>> COMT & SULF1A1 | | |
| Genotype | Mean OR | Mean p |
| G/G A/A | 2.58 ± .35 | 0.0175 |
| | | |
| >>> COMT & HER2 | | |
| Genotype | Mean OR | Mean p |
| A/A A/* | 0.67 ± .04 | 0.0236 |
| | | |
| >>> COMT & p53 72 | | |
| Genotype | Mean OR | Mean p |
| G/A C/G | 1.49 ± .09 | 0.0377 |
| | | |
| >>> COMT & CYP1BB1 | | |
| Genotype | Mean OR | Mean p |
| A/A */G | 0.69 ± .04 | 0.0493 |
| >>> COMT & PROGINS None | | |
| | | |
| >>> COMT & SRD5A2_2 | | |
| Genotype | Mean OR | Mean p |
| G/A O/O | 1.37 ± .07 | 0.0337 |
| | | |
| >>> COMT & MTHFR | | |
| Genotype | Mean OR | Mean p |
| G/A C/C | 1.49 ± .11 | 0.0671 |
| | | |
| >>> COMT & VDR/ApaI | | |
| Genotype | Mean OR | Mean p |
| A/A A/a | 0.57 ± .05 | 0.0263 |
| G/A A/A | 1.55 ± .10 | 0.0294 |
| | | |
| >>> COMT & VDR/TaqI | | |
| Genotype | Mean OR | Mean p |
| G/A t/t | 1.43 ± .12 | 0.1629 |
| | | |
| >>> COMT & VDR/FokI | | |
| Genotype | Mean OR | Mean p |
| G/A F/F | 1.46 ± .10 | 0.0776 |
| | | |
| >>> COMT & CYP2E1 | | |
| Genotype | Mean OR | Mean p |
| G/A D/C | 2.41 ± .30 | 0.0246 |
| | | |
| >>> COMT & EPHX | | |
| Genotype | Mean OR | Mean p |
| G/A Y/* | 1.61 ± .15 | 0.0779 |
| | | |
| >>> COMT & CYP1A1 | | |
| Genotype | Mean OR | Mean p |
| A/A M/* | 0.74 ± .04 | 0.0812 |
| | | |
| >>> COMT & CYP11B2 | | |
| Genotype | Mean OR | Mean p |
| A/A T/T | 0.57 ± .06 | 0.0603 |
| | | |
| >>> COMT & CYC D 1 | | |
| Genotype | Mean OR | Mean p |
| G/A A/A | 3.02 ± .51 | 0.0177 |
| | | |
| >>> COMT & XRCC 1 | | |
| Genotype | Mean OR | Mean p |
| G/A A/A | 2.73 ± .69 | 0.1089 |
| | | |
| >>> GSTP1 & SULF1A1 | | |
| Genotype | Mean OR | Mean p |
| G/G A/A | 3.14 ± .73 | 0.0589 |
| >>> GSTP1 | & HER2 | None |
| | | |
| >>> GSTP1 | & p53 72 | None |
| | | |
| >>> GSTP1 | & CYP1B1 | None |
| | | |
| >>> GSTP1 & PROGINS | | |
| Genotype | Mean OR | Mean p |
| G/A I/I | 4.75 ± 1.87 | 0.0759 |
| | | |
| >>> GSTP1 | & SRD5A2_2 | None |
| | | |
| >>> GSTP1 | & MTHFR | None |
| | | |
| >>> GSTP1 & VDR/ApaI | | |
| Genotype | Mean OR | Mean p |
| A/* A/A | 1.27 ± .07 | 0.1504 |
| | | |
| >>> GSTP1 | & VDR/TaqI | None |
| | | |
| >>> GSTP1 | & VDR/FokI | None |
| | | |
| >>> GSTP1 | & CYP2E1 | None |
| | | |
| >>> GSTP1 | & EPHX | None |
| | | |
| >>> GSTP1 | & CYP1A1 | None |
| | | |
| >>> GSTP1 & CYP11B2 | | |
| Genotype | Mean OR | Mean p |
| G/G C/* | 1.45 ± .13 | 0.1567 |
| | | |
| >>> GSTP1 & CYC D1 | | |
| Genotype | Mean OR | Mean p |
| A/A A/A | 2.58 ± .55 | 0.0757 |
| G/A A/A | 2.03 ± .27 | 0.0795 |
| | | |
| >>> GSTP1 | & XRCC 1 | None |
| | | |
| >>> SULF1A1 | & HER2 | None |
| | | |
| >>> SULF1A1 & p53 72 | | |
| Genotype | Mean OR | Mean p |
| A/A G/G | 1.54 ± .14 | 0.0997 |
| G/A G/G | 0.70 ± .04 | 0.0393 |
| | | |
| >>> SULF1A1 & CYP1B1 | | |
| Genotype | Mean OR | Mean p |
| G/A C/* | 0.65 ± .04 | 0.0094 |
| >>> SULF1A1 & PROGINS | | |
| Genotype | Mean OR | Mean p |
| A/A I/I | 7.09 ± 1.54 | 0.1173 |
| G/G I/I | 3.74 ± 1.30 | 0.1481 |
| | | |
| >>> SULF1A1 & SRD5A2_2 None | | |
| | | |
| >>> SULF1A1 | & MTHFR | |
| Genotype | Mean OR | Mean p |
| A/A C/T | 1.95 ± .25 | 0.1038 |
| | | |
| >>> SULF1A1 & VDR/ApaI | | |
| Genotype | Mean OR | Mean p |
| A/A a/a | 1.76 ± .22 | 0.1468 |
| | | |
| >>> SULF1A1 & VDR/TaqI | | |
| Genotype | Mean OR | Mean p |
| A/A T/T | 1.72 ± .18 | .0920 |
| | | |
| >>> SULF1A1 & VDR/FokI | | |
| Genotype | Mean OR | Mean p |
| A/A F/F | 2.12 ± .28 | 0.0633 |
| G/G f/f | 1.73 ± .18 | 0.0788 |
| | | |
| >>> SULF1A1 & CYP2E1 None | | |
| | | |
| >>> SULF1A1 & EPHX None | | |
| | | |
| >>> SULF1A1 & CYP1A1 None | | |
| | | |
| >>> SULF1A1 & CYP11B2 | | |
| Genotype | Mean OR | Mean p |
| A/A C/* | 1.45 ± .12 | 0.1480 |
| | | |
| >>> SULF1A1 & CYC D1 | | |
| Genotype | Mean OR | Mean p |
| A/A A/A | 3.49 ± 1.32 | 0.2022 |
| G/G A/A | 2.25 ± 0.34 | 0.0581 |
| | | |
| >>> SULF1A1 & XRCC 1 | | |
| Genotype | Mean OR | Mean p |
| A/A G/G | 3.75 ± 1.43 | 0.1011 |
| | | |
| >>> HER2 & p53 72 | | |
| Genotype | Mean OR | Mean p |
| A/A C/* | 1.30 ± .07 | 0.1268 |
| | | |
| >>> HER2 & CYP1B1 | | |
| Genotype | Mean OR | Mean p |
| G/G G/G | 2.76 ± .62 | 0.0875 |
| >>> HER2 & PROGINS | | |
| Genotype | Mean OR | Mean p |
| G/A I/I | 4.25 ± 1.27 | 0.0572 |
| | | |
| >>> HER2 & SRD5A2_2 None | | |
| | | |
| >>> HER2 & MTHFR None | | |
| | | |
| >>> HER2 & VDR/ApaI | | |
| Genotype | Mean OR | Mean p |
| A/A A/A | 1.40 ±.09 | 0.0774 |
| | | |
| >>> HER2 & VDR/TaqI None | | |
| | | |
| >>> HER2 & VDR/FokI None | | |
| | | |
| >>> HER2 & CYP2E1 None | | |
| | | |
| >>> HER2 & EPHX None | | |
| | | |
| >>> HER2 & CYP1A1 None | | |
| | | |
| >>> HER2 & CYP11B2 | | |
| Genotype | Mean OR | Mean p |
| G/G C/C | 2.31 ± .50 | 0.2033 |
| | | |
| >>> HER2 & CYC D 1 | | |
| Genotype | Mean OR | Mean p |
| G/A A/A | 5.35 ± 1.86 | 0.0099 |
| A/* A/A | 2.39 ± .27 | 0.0089 |
| | | |
| >>> HER2 & XRCC 1 None | | |
| | | |
| >>> p5372 & CYP1B1 None | | |
| | | |
| >>> p53 72 & PROGINS None | | |
| | | |
| >>> p53 72 & SRD5A2_2 None | | |
| | | |
| >>> p53 72 & MTHFR None | | |
| | | |
| >>> p53 72 & VDR/ApaI | | |
| Genotype | Mean OR | Mean p |
| C/C A/A | 2.31 ± .38 | 0.0764 |
| | | |
| >>> p53 72 & VDR/TaqI None | | |
| | | |
| >>> p53 72 & VDR/FokI | | |
| Genotype | Mean OR | Mean p |
| C/C F/f | 2.09 ± .31 | 0.0986 |
| C/G F/F | 1.72 ± .14 | 0.0373 |
| | | |
| >>> p53 72 & CYP2E1 None | | |
| | | |
| >>> p53 72 & EPHX None | | |
| | | |
| >>> p53 72 & CYP1A1 None | | |
| | | |
| >>> p5372 & CYP11B2 | | |
| Genotype | Mean OR | Mean p |
| C/C C/C | 2.31 ± .48 | 0.1728 |
| G/G C/C | 0.66 ± .05 | 0.0847 |
| | | |
| >>> p53 72 & CYC D1 | | |
| Genotype | Mean OR | Mean p |
| C/C A/* | 3.27± .76 | 0.0531 |
| */G A/A | 1.95 ± .22 | 0.0412 |
| | | |
| >>> p53 72 & XRCC 1 None | | |
| | | |
| >>> CYP1B1 & PROGINS | | |
| Genotype | Mean OR | Mean p |
| G/G I/I | 7.01 ± 1.51 | 0.1186 |
| | | |
| >>> CYP1B1 &SRD5A2_2 None | | |
| | | |
| >>> CYP1B1 & MTHFR None | | |
| | | |
| >>> CYP1B1 & VDR/ApaI | | |
| Genotype | Mean OR | Mean p |
| G/G A/A | 1.56 ± .14 | 0.1189 |
| | | |
| >>> CYP1B1 & VDR/TaqI None | | |
| | | |
| >>> CYP1B1 & VDR/FokI None | | |
| | | |
| >>> CYP1B1 & CYP2E1 | | |
| Genotype | Mean OR | Mean p |
| C/C D/C | 2.77 ± .45 | 0.0274 |
| | | |
| >>> CYP1B1 & EPHX | | |
| Genotype | Mean OR | Mean p |
| C/C */H | 1.88 ± .22 | 0.0722 |
| | | |
| >>> CYP1B1 & CYP1A1 None | | |
| >>> CYP1B1 & CYP11B2 None | | |
| | | |
| >>> CYP1B1 & CYC D1 | | |
| Genotype | Mean OR | Mean p |
| C/G A/A | 2.33 ± .39 | 0.0566 |
| G/G A/A | 2.75 ± .75 | 0.1076 |
| | | |
| >>> CYP1B1 & XRCC 1 None | | |
| | | |
| >>> PROGINS & SRD5A2_2 None | | |
| | | |
| >>> PROGINS & MTHFR | | |
| Genotype | Mean OR | Mean p |
| I/I C/C | 3.48 ± 1.34 | 0.1778 |
| | | |
| >>> PROGINS & VDR/ApaI | | |
| Genotype | Mean OR | Mean p |
| N/N A/A | 1.28 ± .07 | 0.1613 |
| | | |
| >>> PROGINS & VDR/TaqI None | | |
| | | |
| >>> PROGINS & VDR/FokI None | | |
| | | |
| >>> PROGINS & CYP2E1 None | | |
| | | |
| >>> PROGINS & EPHX | | |
| Genotype | Mean OR | Mean p |
| N/N */H | 1.54 ± .14 | 0.1128 |
| | | |
| >>> PROGINS & CYP1A1 None | | |
| | | |
| >>> PROGINS & CYP11B2 None | | |
| | | |
| >>> PROGINS & CYC D 1 | | |
| Genotype | Mean OR | Mean p |
| I/N G/A | 2.03 ± .26 | 0.0501 |
| I/N A/* | 2.24 ± .26 | 0.0145 |
| N/N A/A | 1.97 ± .23 | 0.0482 |
| | | |
| >>> PROGINS & XRCC 1 None | | |
| | | |
| >>> SRD5A2-2 & MTHFR None | | |
| | | |
| >>> SRD5A2_2 & VDR/ApaI | | |
| Genotype | Mean OR | Mean p |
| O/* A/A | 1.26 ± .07 | 0.1562 |
| | | |
| >>> SRD5A2_2 & VDR/TaqI None | | |
| | | |
| >>> SRD5A2_2 & VDR/FokI None | | |
| | | |
| >>> SRD5A2_2 & CYP2E1 | | |
| Genotype | Mean OR | Mean p |
| O/A D/C | 2.37 ± .43 | 0.1318 |
| | | |
| >>> SRD5A2_2 & EPHX | | |
| Genotype | Mean OR | Mean p |
| O/A Y/H | 2.49 ± .36 | 0.0343 |
| | | |
| >>> SRD5A2-2 & CYP1A1 None | | |
| | | |
| >>> SRD5A2_2 & CYP11B2 | | |
| Genotype | Mean OR | Mean p |
| O/O */T | 1.33 ± .07 | 0.0683 |
| | | |
| >>> SRD5A2_2 & CYC D 1 | | |
| Genotype | Mean OR | Mean p |
| O/O A/A | 2.41 ± .28 | 0.0103 |
| | | |
| >>> SRD5A2_2 & XRCC 1 None | | |
| | | |
| >>> MTHFR & VDR/ApaI | | |
| Genotype | Mean OR | Mean p |
| C/C A/a | 1.36 ± .10 | 0.1603 |
| C/C A/* | 1.31 ± .08 | 0.1530 |
| C/T A/A | 1.39 ± .12 | 0.1976 |
| | | |
| >>> MTHFR & VDR/TaqI | | |
| Genotype | Mean OR | Mean p |
| C/C T/t | 1.36 ± .09 | 0.1522 |
| | | |
| >>> MTHFR & VDR/FokI | | |
| Genotype | Mean OR | Mean p |
| C/C F/F | 1.43 ± .11 | 0.1461 |
| | | |
| >>> MTHFR & CYP2E1 | | |
| Genotype | Mean OR | Mean p |
| C/T D/C | 2.50 ± .40 | 0.0583 |
| | | |
| >>> MTHFR & EPHX None | | |
| | | |
| >>> MTHFR & CYP1A1 None | | |
| | | |
| >>> MTHFR & CYP11B2 | | |
| Genotype | Mean OR | Mean p |
| C/T C/C | 1.57 ± .15 | 0.1408 |
| | | |
| >>> MTHFR & CYC D1 | | |
| Genotype | Mean OR | Mean p |
| C/T A/A | 3.24 ± .71 | 0.0342 |
| | | |
| >>> MTHFR & XRCC 1 None | | |
| | | |
| >>> VDR/ApaI & VDR/TaqI | | |
| Genotype | Mean OR | Mean p |
| A/A T/t | 1.61 ± .12 | 0.0366 |
| >>> VDR/ApaI & VDR/FokI | | |
| Genotype | Mean OR | Mean p |
| A/A f/f | 2.02 ± .24 | 0.0507 |
| | | |
| >>> VDR/ApaI & CYP2E1 | | |
| Genotype | Mean OR | Mean p |
| A/A D/D | 1.29± .07 | 0.1246 |
| A/a D/C | 2.05 ± .29 | 0.0973 |
| | | |
| >>> VDR/ApaI & EPHX | | |
| Genotype | Mean OR | Mean p |
| Ala H/H | 1.82 ± .28 | 0.2079 |
| | | |
| >>> VDR/ApaI & CYP1A1 | | |
| Genotype | Mean OR | Mean p |
| A/A M/* | 1.28 ± .07 | 0.1221 |
| | | |
| >>>VDR/ApaI & CYP11B2 | | |
| Genotype | Mean OR | Mean p |
| A/A C/T | 1.37 ± .09 | 0.1160 |
| | | |
| >>> VDR/ApaI & CYC D 1 | | |
| Genotype | Mean OR | Mean p |
| a/a A/A | 4.68 ± 1.57 | 0.0382 |
| a/a A/* | 2.01± .23 | 0.0445 |
| A/* A/A | 2.05 ± .25 | 0.0435 |
| | | |
| >>> VDR/ApaI & XRCC 1 | | |
| Genotype | Mean OR | Mean p |
| A/A G/G | 1.64 ± .21 | 0.1914 |
| a/a G/G | 1.87 ± .25 | 0.1238 |
| | | |
| >>> VDR/TaqI & VDR/FokI None | | |
| | | |
| >>> VDR/TaqI & CYP2E1 None | | |
| | | |
| >>> VDR/TaqI & EPHX | | |
| Genotype | Mean OR | Mean p |
| T/T H/H | 2.07 ± .36 | 0.1765 |
| t/t Y/H | 2.00 ± .32 | 0.1528 |
| | | |
| >>> VDR/TaqI & CYP1A1 | | |
| Genotype | Mean OR | Mean p |
| t/t M/m | 1.84 ± .22 | 0.0838 |
| | | |
| >>>VDR/TaqI & CYP11B2 | | |
| Genotype | Mean OR | Mean p |
| T/t T/T | 1.29 ± .08 | 0.1988 |
| | | |
| >>> VDR/TaqI & CYC D 1 | | |
| Genotype | Mean OR | Mean p |
| T/T A/A | 2.81 ± .52 | 0.0508 |
| T/t A/A | 2.83 ± .45 | 0.0200 |
| | | |
| >>> VDR/TaqI & XRCC 1 None | | |
| | | |
| >>> VDR/FokI & CYP2E1 None | | |
| | | |
| >>> VDR/FokI & EPHX | | |
| Genotype | Mean OR | Mean p |
| F/F Y/H | 2.10 ± .24 | 0.0324 |
| | | |
| >>> VDR/FokI & CYP1A1 None | | |
| | | |
| >>>VDR/FokI & CYP11B2 None | | |
| | | |
| >>> VDR/FokI & CYC D 1 | | |
| Genotype | Mean OR | Mean p |
| F/F A/A | 2.58 ± .60 | 0.1082 |
| F/f A/A | 2.15 ± .32 | 0.0668 |
| | | |
| >>> VDR/FokI & XRCC 1 None | | |
| | | |
| >>> CYP2E1 & EPHX None | | |
| | | |
| >>> CYP2E1 & CYP1A1 | | |
| Genotype | Mean OR | Mean p |
| D/* M/* | 2.32 ± .70 | 0.1915 |
| | | |
| >>> CYP2E1 & CYP11B2 | | |
| Genotype | Mean OR | Mean p |
| D/C T/T | 1.85 ± .27 | 0.1813 |
| | | |
| >>>CYP2E1 & CYCD1 | | |
| Genotype | Mean OR | Mean p |
| D/D A/A | 2.27 ± .25 | 0.0120 |
| D/* A/A | 2.42 ± .27 | 0.0064 |
| | | |
| >>> CYP2E1 & XRCC 1 None | | |
| | | |
| >>> EPHX & CYP1A1 None | | |
| | | |
| >>> EPHX & CYP11B2 | | |
| Genotype | Mean OR | Mean p |
| Y/H C/T | 1.72 ± .19 | 0.1020 |
| | | |
| >>> EPHX & CYC D1 None | | |
| | | |
| >>> EPHX & XRCC 1 None | | |
| | | |
| >>> CYP1A1 & CYP11B2 | | |
| Genotype | Mean OR | Mean p |
| M/m T/T | 1.56 ± .16 | 0.1703 |
| | | |
| >>> CYP1A1 & CYC D1 | | |
| Genotype | Mean OR | Mean p |
| M/M A/A | 2.45 ± .30 | 0.0097 |
| M/* A/A | 2.29 ± .25 | 0.0098 |
| | | |
| >>> CYP1A1 & XRCC 1 None | | |
| | | |
| >>>CYP11B2 & CYC D1 | | |
| Genotype | Mean OR | Mean p |
| C/C A/A | 2.51 ± .71 | 0.2023 |
| T/T A/A | 2.43 ± .45 | 0.0653 |
| | | |
| >>> CYP11B2 & XRCC 1 None | | |
| | | |
| >>> CYC D 1 & XRCC 1 | | |
| Genotype | Mean OR | Mean p |
| A/A A/A | 9.11 ± 1.22 | 0.0421 |
| A/A G/G | 4.10 ± .92 | 0.0167 |
| A/A */G | 2.04 ± .25 | 0.0396 |

An allelic designation of "*" indicates that the allele can be either of the two possible alleles. For example, */T means T/T and C/T for the Cyp17 polymorphism.

### EXAMPLE 4 - RESULTS: AGE STRATIFIED ABOVE 54

The inventors have examined the association of various genetic polymorphisms with breast cancer. The results presented in Tables 4A-B are a synthesis of a complex bootstrap analysis performed many different ways. The data set used in this analysis consisted of nearly 340 women that have been diagnosed with breast cancer and approximately 900 women who had never been diagnosed with any cancer. All women in this analysis were over the age of 54 when they were diagnosed with breast cancer or, if cancer free, at the time that their DNA was collected for this study. Twenty different genetic polymorphisms were examined. In general, when examined singly (one at a time), these polymorphism were weakly associated with risk of a breast cancer diagnosis. As a group, they may be termed common risk alleles with low penetrance or no penetrance for the breast cancer phenotype. The surprising observation made during this study was that when examined in combination (in pairs or in combinations of three or more), complex genotypes were defined that exhibited higher risk or higher penetrance for a breast cancer diagnosis. Table 4A, presents results for these polymorphisms examined singly. Table 4B presents results for analysis of these same polymorphisms examined in pairs or in combinations of three respectively. Examining these polymorphisms in combinations of two (pairs) is more informative for estimating breast cancer risk than examining polymorphisms alone. These gene pairs can then form the building blocks for identifying complex genotypes involving three or more polymorphic genes with greatly improved discriminatory accuracy for stratifying a woman's individual risk of being diagnosed with breast cancer. The inventors show that by starting with these building blocks of gene pairs and then adding information from other genes, one can stratify an individual's risk of being diagnosed with breast cancer over a wide range of relative risk. This range extends from less than average risk to many times average risk. The tables show the gene or genes being examined and the genotypes of these genes being compared. The tables also show the mean Odds Ratio (OR) or relative risk with the respective genotypes compared to an individual with average risk. The OR is shown as a mean because it is the composite or aggregate result of many analyses and represents the best estimate of the true OR in the general population. An OR less than one represents less than average risk while an OR greater than one represents greater than average risk. The mean p value is a measure statistical significance based on an average of Chi² tests of the ORs in used to determine the mean OR. By convention, p values ≤ 0.05 are considered to be statistically significant. It is a general observation of this analysis that the ORs and associated p values for the genetic polymorphisms examined by themselves have very limited ability to stratify risk and have p values typically greater than 0.05. In pairs, the polymorphisms stratify risk over a greater range and have much lower (more significant) p values. This trend continues as additional genes are added to the pairs with even greater stratification of risk and much smaller p values.

**Table 4A - White Women Over 54 Years of Age (Polymorphisms of Genes examined Singly)**

| >>> Prohibitin | | |
|---|---|---|
| Genotype | Mean OR | Mean p |
| C/C | 1.15 ± .08 | .4462 |
| C/T | .93 ± .07 | .6317 |
| T/T | .52 ± .14 | .2995 |
| | | |

| >>> CYP17 | | |
|---|---|---|
| Genotype | Mean OR | Mean p |
| C/C | 1.05 ± .10 | .7112 |
| C/T | .83 ± .06 | .2831 |
| T/T | 1.18 ± .08 | .3393 |
| | | |

| >>> COMT | | |
|---|---|---|
| Genotype | Mean OR | Mean p |
| A/A | 1.03 ±.08 | .7139 |
| G/A | 1.16 ± .08 | .3933 |
| G/G | .78 ± .07 | .2430 |
| | | |

| >>> GSTP1 | | |
|---|---|---|
| Genotype | Mean OR | Mean p |
| A/A | .91 ± .06 | .5572 |
| G/A | 1.02 ±.07 | .7434 |
| G/G | 1.19 ± .12 | .4753 |
| | | |

| >>> SULF1A1 | | |
|---|---|---|
| Genotype | Mean OR | Mean p |
| A/A | 1.25 ± .12 | .3592 |
| G/A | 1.00 ±.07 | .7514 |
| G/G | .91 ± .06 | .5237 |
| | | |

| >>> HER2 | | |
|---|---|---|
| Genotype | Mean OR | Mean p |
| A/A | 1.03 ±.08 | .7163 |
| G/A | 1.08 ± .08 | .6227 |
| G/G | .61 ± .11 | .2487 |
| | | |

| >>> p53 72 | | |
|---|---|---|
| Genotype | Mean OR | Mean p |
| C/C | 1.51 ± .17 | .1614 |
| C/G | 1.07 ± .08 | .6329 |
| G/G | .83 ± .06 | .2573 |
| | | |

| >>> CYP1B1 | | |
|---|---|---|
| Genotype | Mean OR | Mean p |
| C/C | 1.27 ± .09 | .1795 |
| C/G | .86 ± .06 | .3559 |
| G/G | .92 ± .08 | .6390 |

| >>> PROGINS | | |
|---|---|---|
| Genotype | Mean OR | Mean p |
| I/I | 1.15 ± .25 | .6765 |
| I/N | .99 ± .08 | .7287 |
| N/N | 1.00 ±.08 | .7344 |
| | | |

| >>> SRD5A2_2 | | |
|---|---|---|
| Genotype | Mean OR | Mean p |
| O/O | .99 ± .08 | .7305 |
| O/A | 1.07 ± .09 | .6705 |
| A/A | .53 ± .15 | .4551 |
| | | |

| >>> MTHFR | | |
|---|---|---|
| Genotype | Mean OR | Mean p |
| C/C | 1.03 ± .09 | .7177 |
| C/T | .93 ± .08 | .6571 |
| T/T | 1.12 ± .14 | .6585 |
| | | |

| >>> VDR/ApaI | | |
|---|---|---|
| Genotype | Mean OR | Mean p |
| A/A | .99 ± .08 | .7457 |
| A/a | 1.03 ± .07 | .7305 |
| a/a | .98 ± .09 | .7121 |
| | | |

| >>> VDR/TaqI | | |
|---|---|---|
| Genotype | Mean OR | Mean p |
| T/T | .99 ± .08 | .7210 |
| T/t | 1.00 ± .07 | .7247 |
| t/t | 1.03 ± .10 | .7224 |
| | | |

| >>> VDR/FokI | | |
|---|---|---|
| Genotype | Mean OR | Mean p |
| F/F | 1.31 ± .10 | .1655 |
| F/f | .89 ± .07 | .5129 |
| f/f | .74 ± .09 | .2966 |
| | | |

| >>> CYP2E1 | | |
|---|---|---|
| Genotype | Mean OR | Mean p |
| D/D | .58 ± .06 | .0487 |
| D/C | 2.00 ± .22 | .0204 |
| C/C | .65 ± .20 | .6166 |
| | | |

| >>> EPHX | | |
|---|---|---|
| Genotype | Mean OR | Mean p |
| Y/Y | .95 ± .11 | .7349 |
| Y/H | 1.20 ± .15 | .5473 |
| H/H | .81 ± .14 | .5966 |
| | | |

| >>> CYP1A1 | | |
|---|---|---|
| Genotype | Mean OR | Mean p |
| M/M | .97 ± .08 | .7177 |
| M/m | 1.02 ±.09 | .7235 |
| m/m | 1.21 ± .33 | .6484 |
| | | |

| >>> CYP11B2 | | |
|---|---|---|
| Genotype | Mean OR | Mean p |
| C/C | .99 ± .08 | .7498 |
| C/T | 1.21 ± .08 | .2808 |
| T/T | .82 ± .06 | .2738 |
| | | |

| >>> CYC D 1 | | |
|---|---|---|
| Genotype | Mean OR | Mean p |
| A/A | 1.91 ± .21 | .0402 |
| G/A | .75 ± .08 | .2717 |
| G/G | .84 ± .09 | .5137 |
| | | |

| >>> XRCC 1 | | |
|---|---|---|
| Genotype | Mean OR | Mean p |
| A/A | .82 ± .15 | .6249 |
| G/A | .78 ± .08 | .3577 |
| G/G | 1.41 ± .15 | .2249 |
| | | |

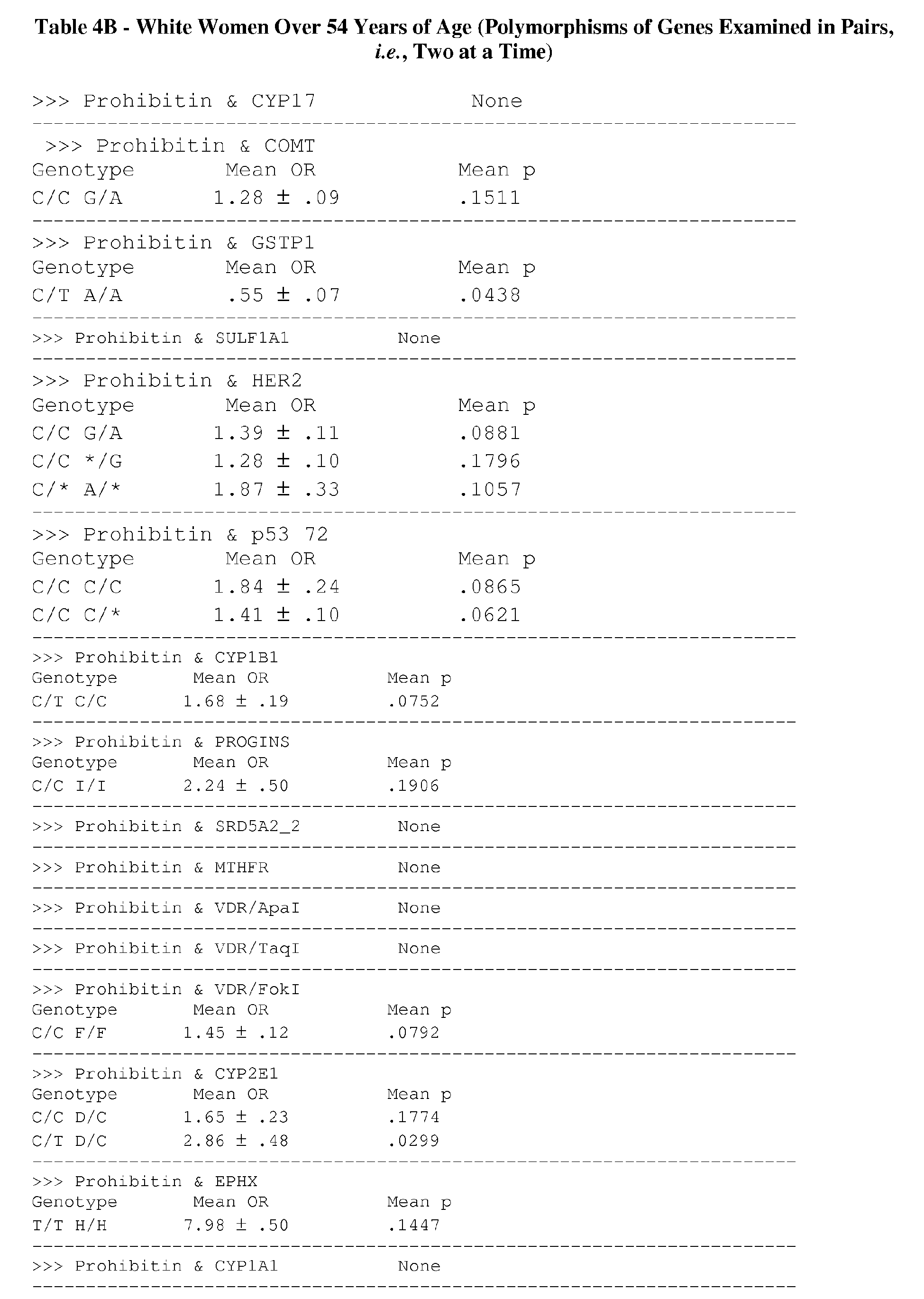

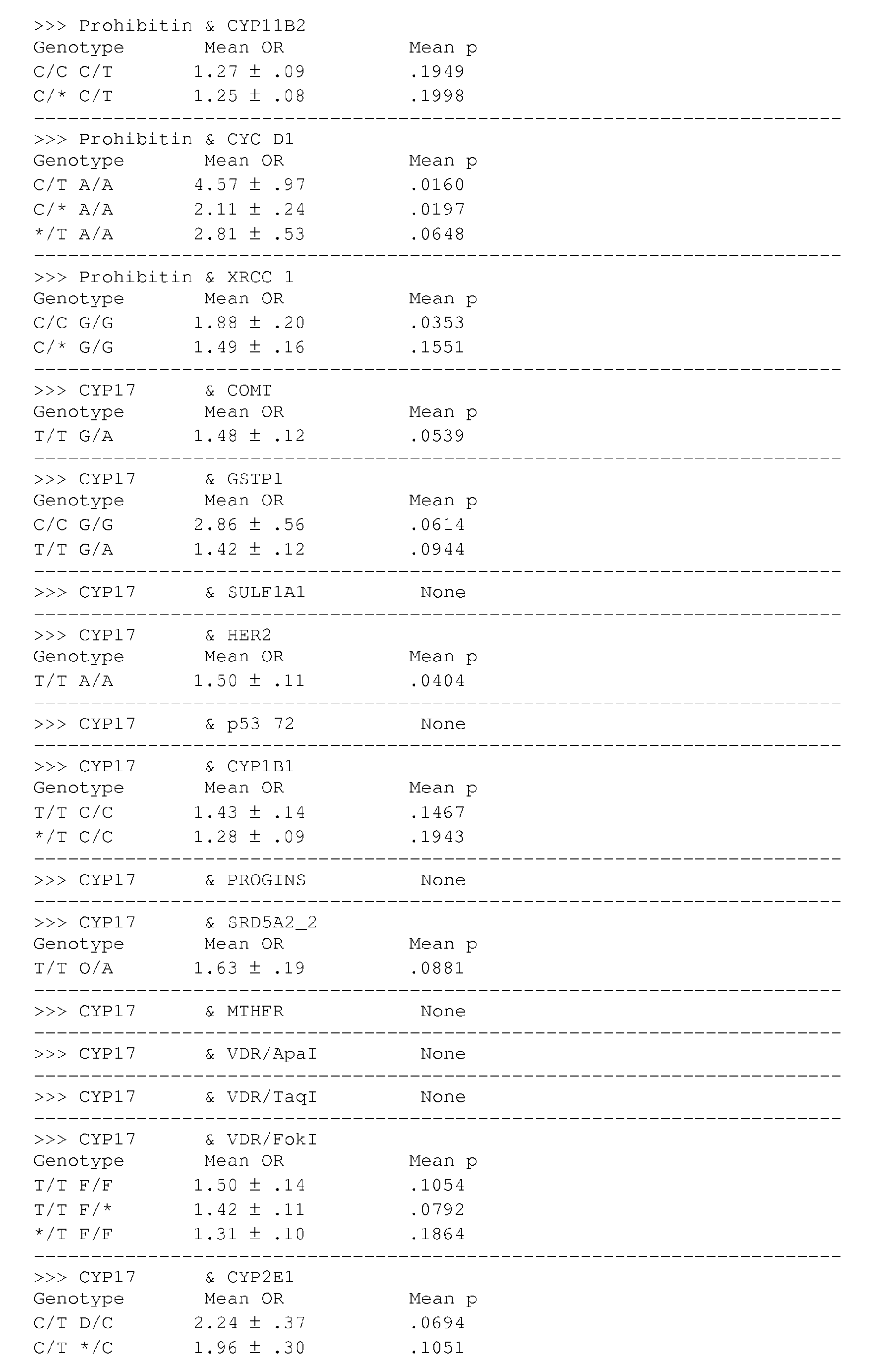

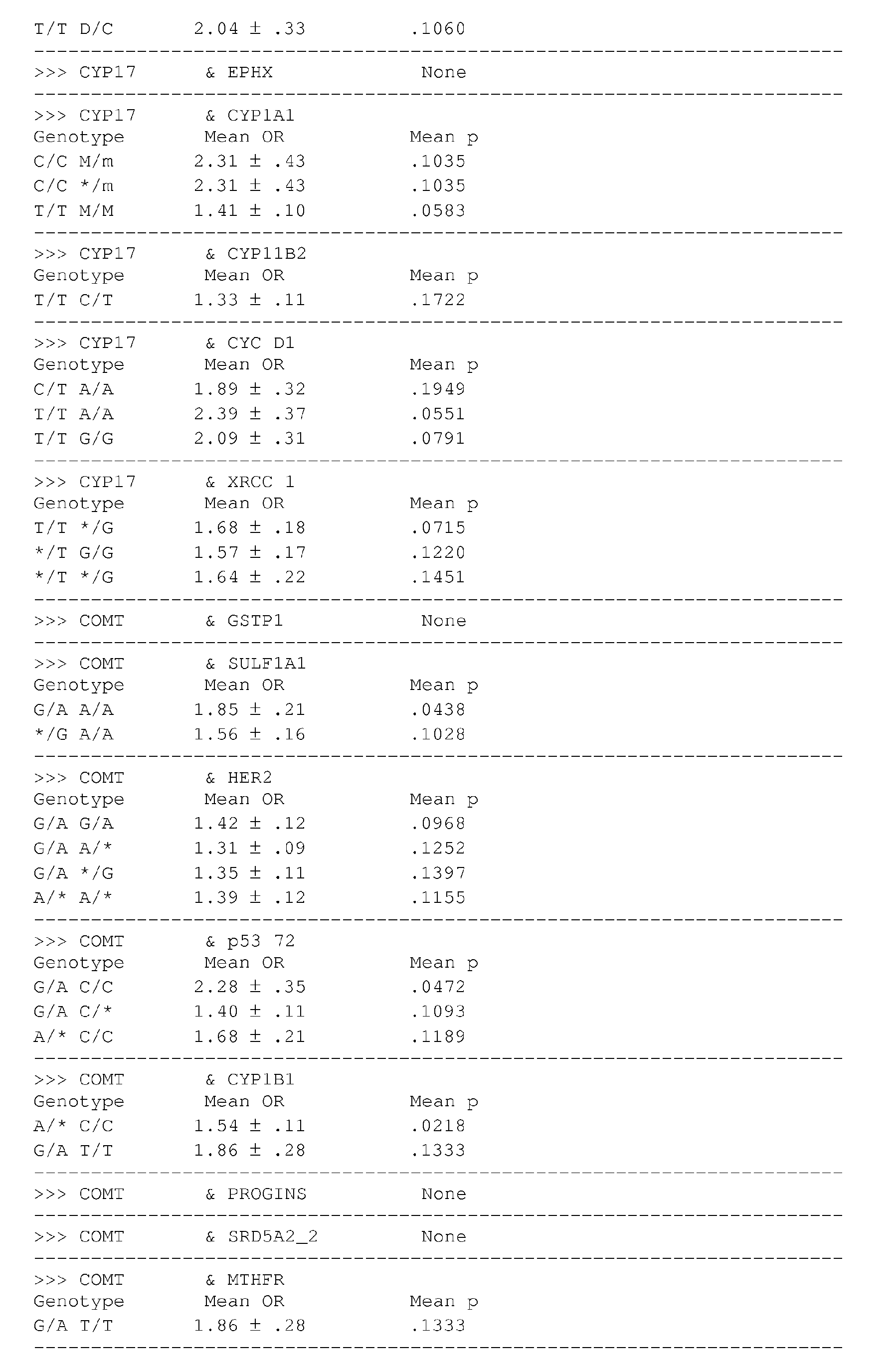

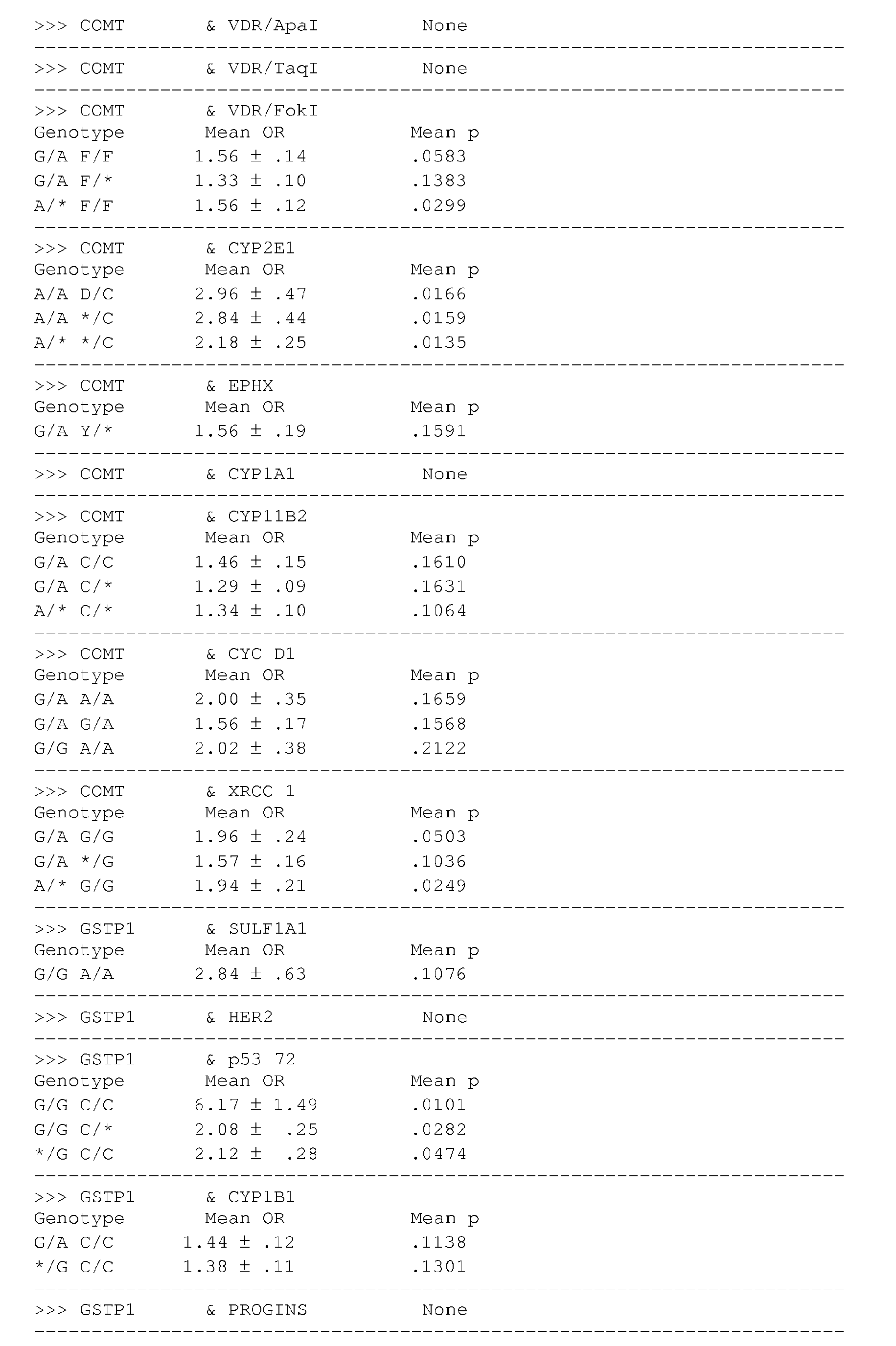

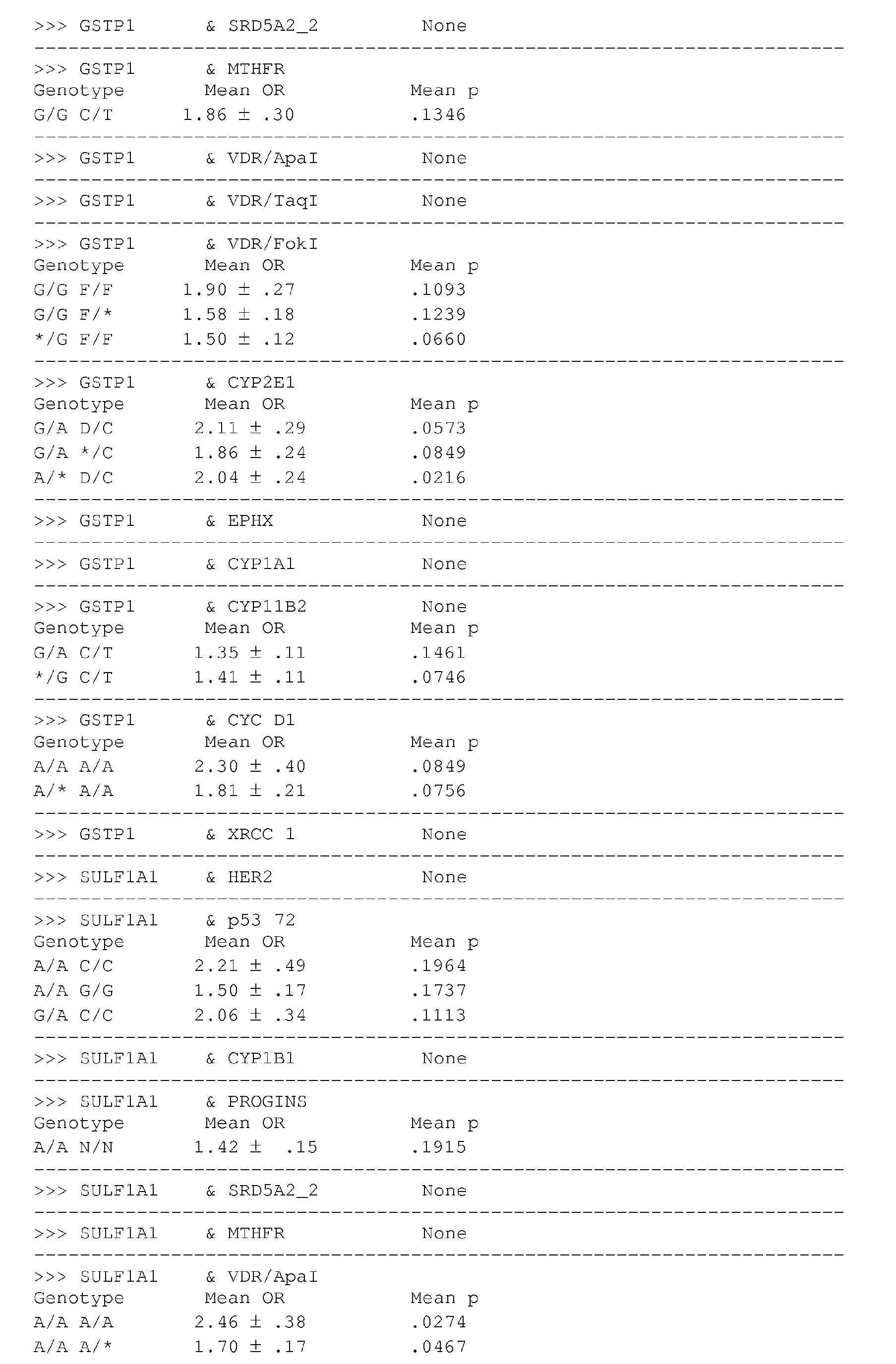

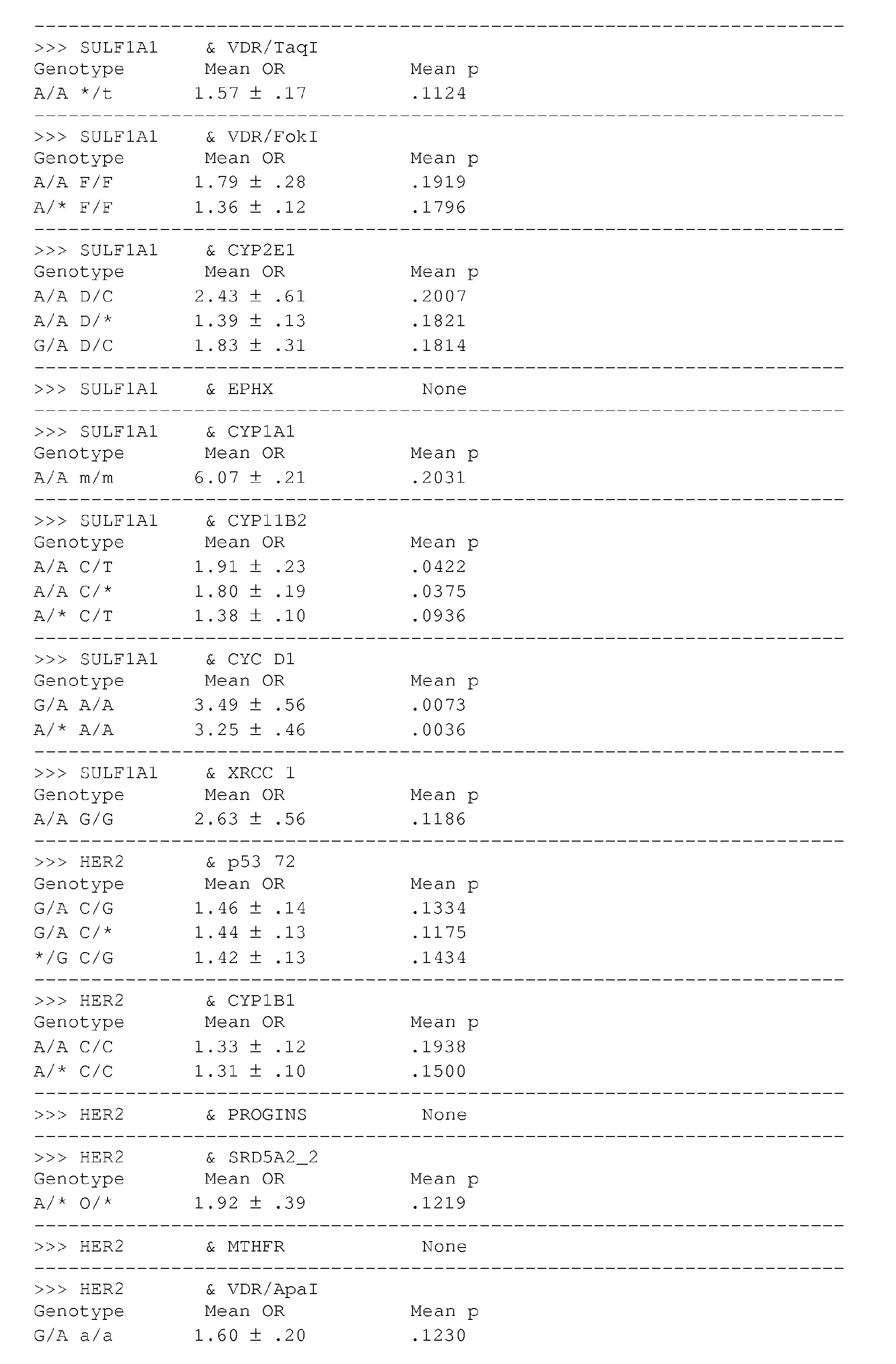

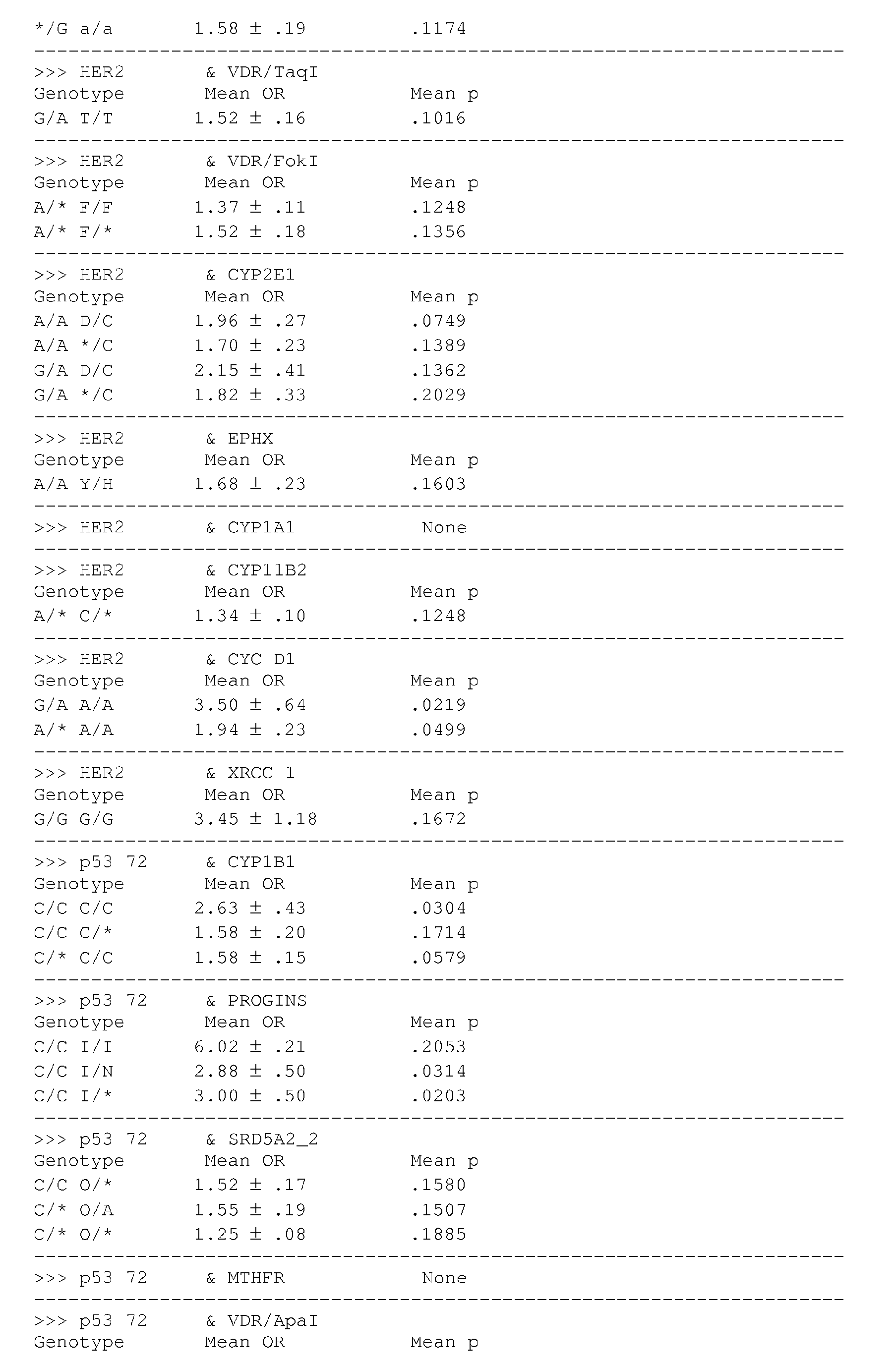

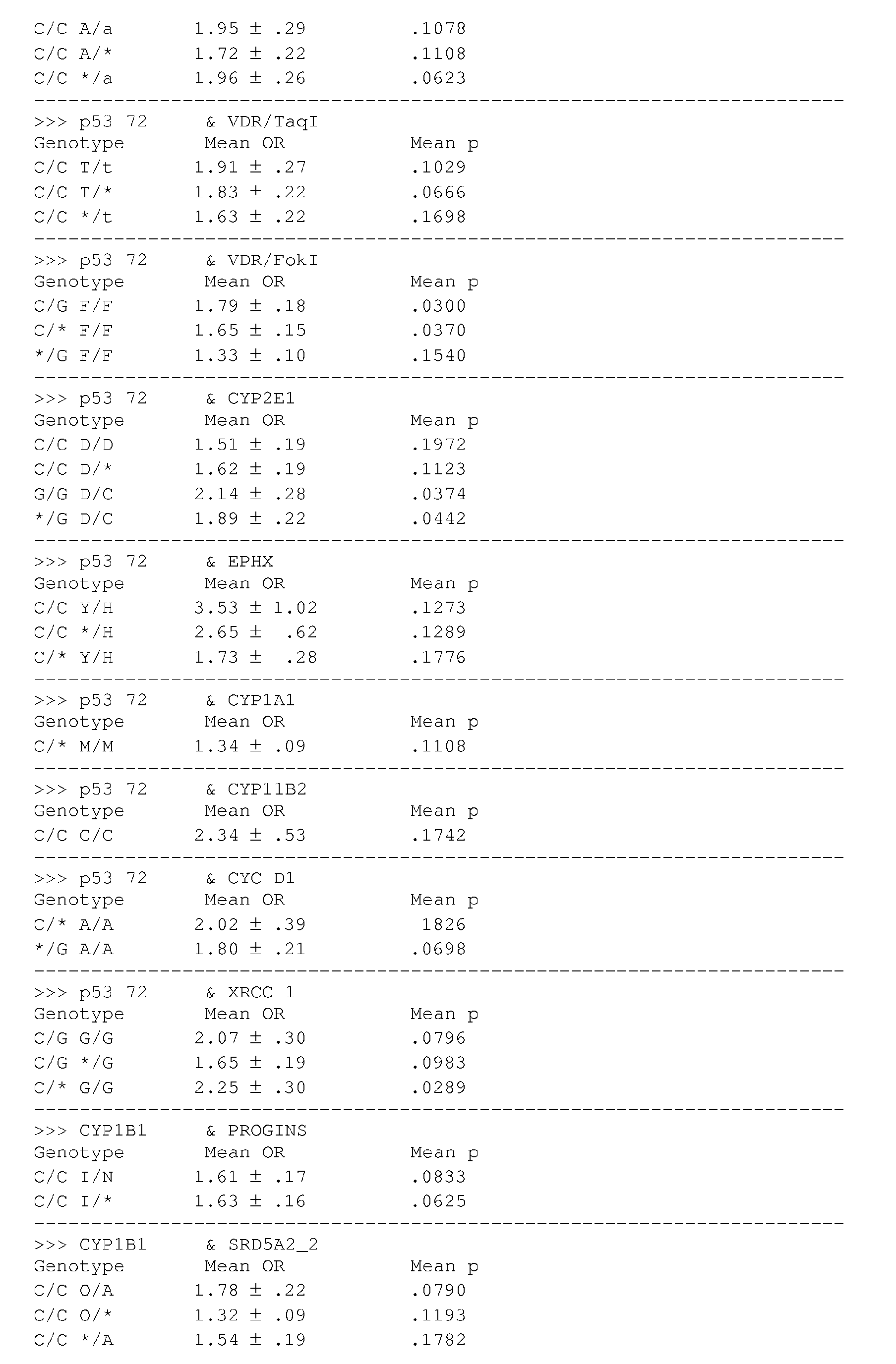

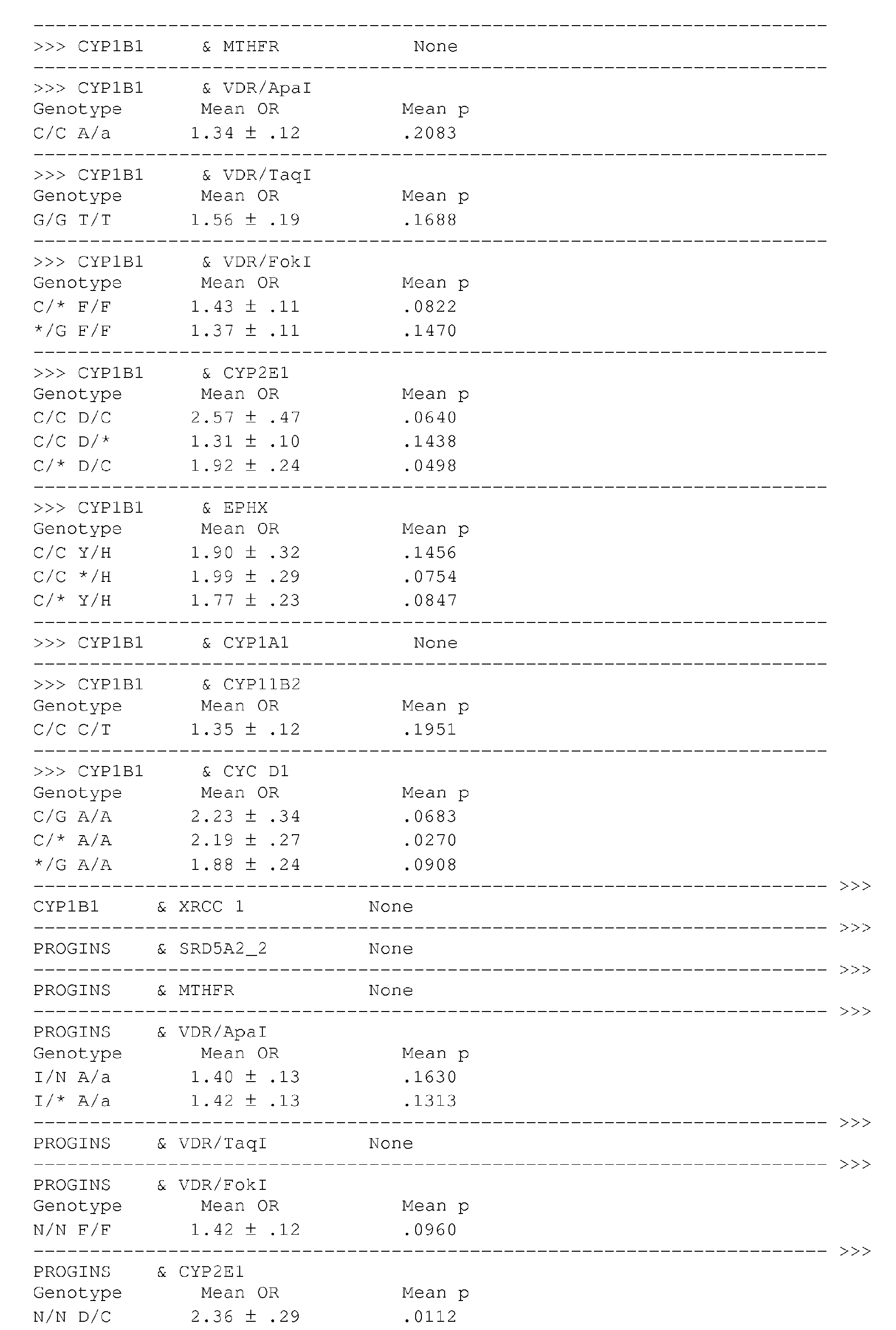

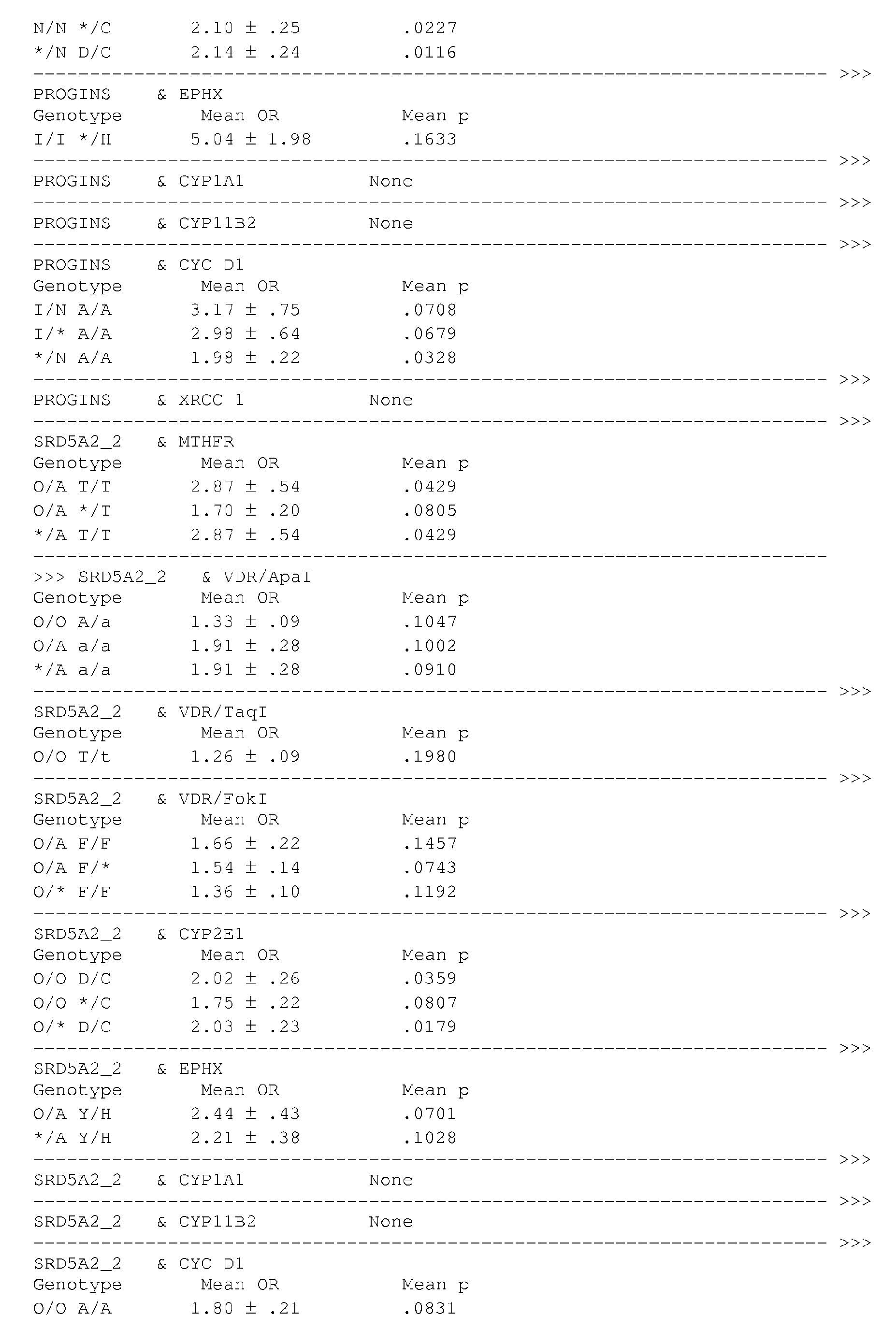

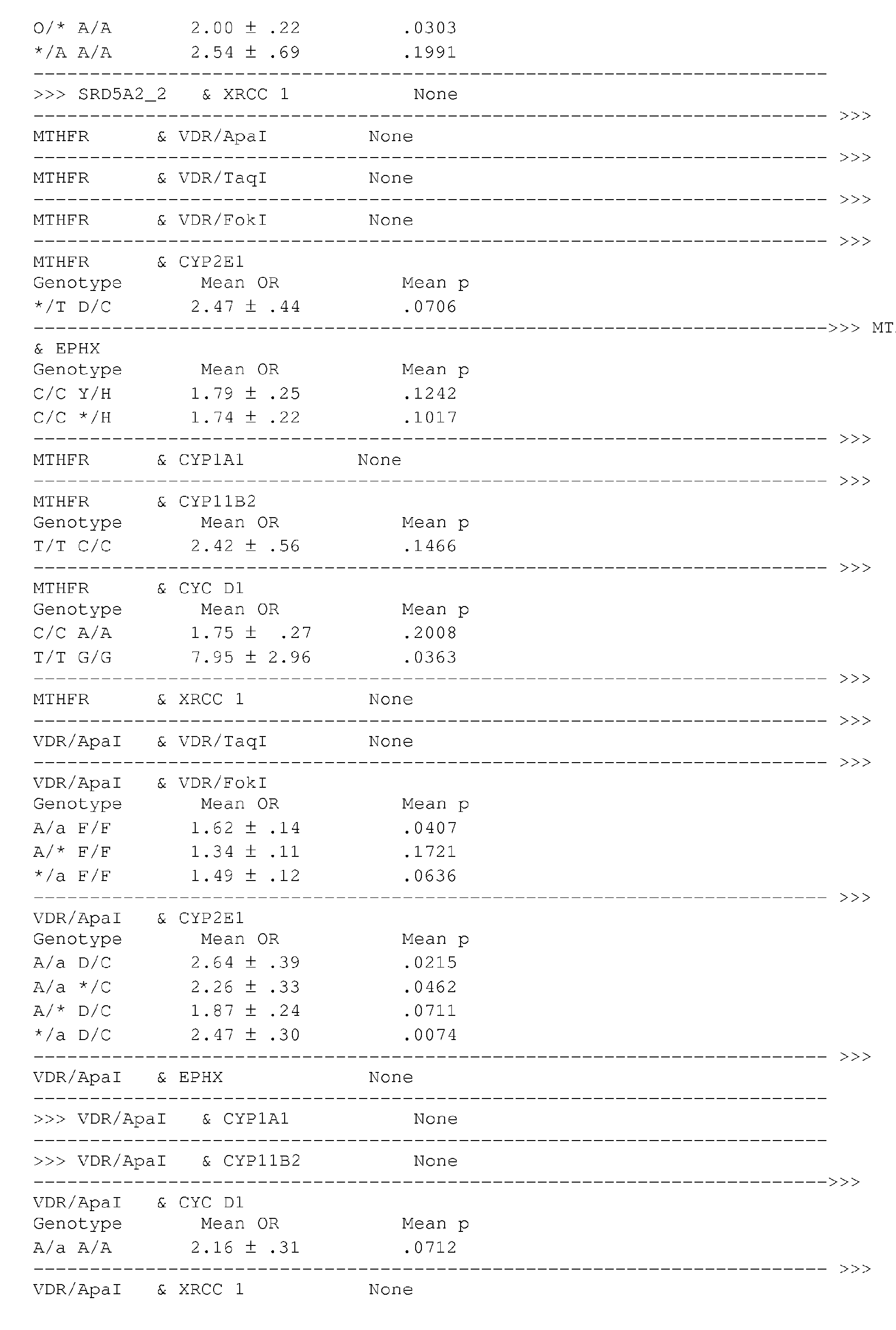

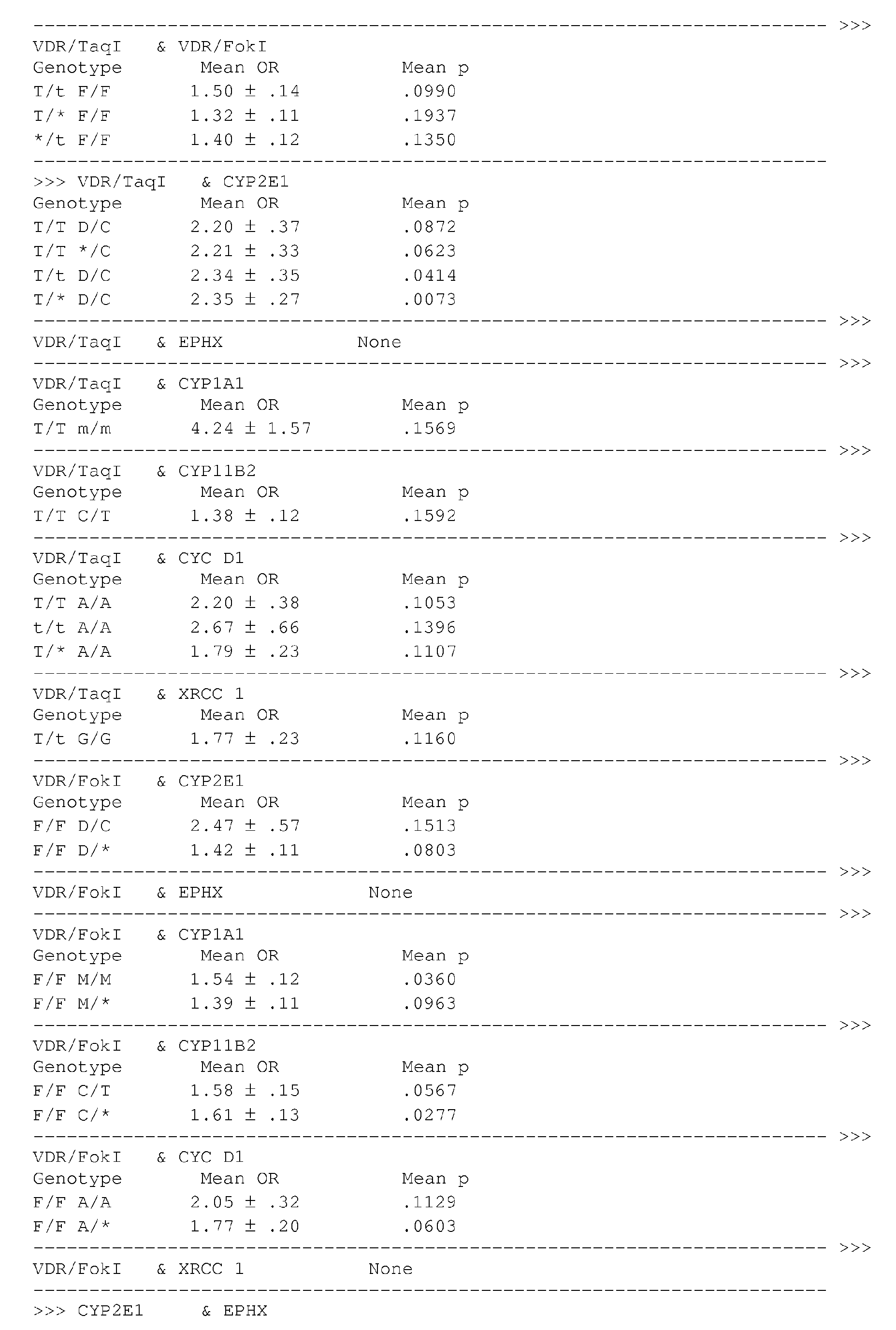

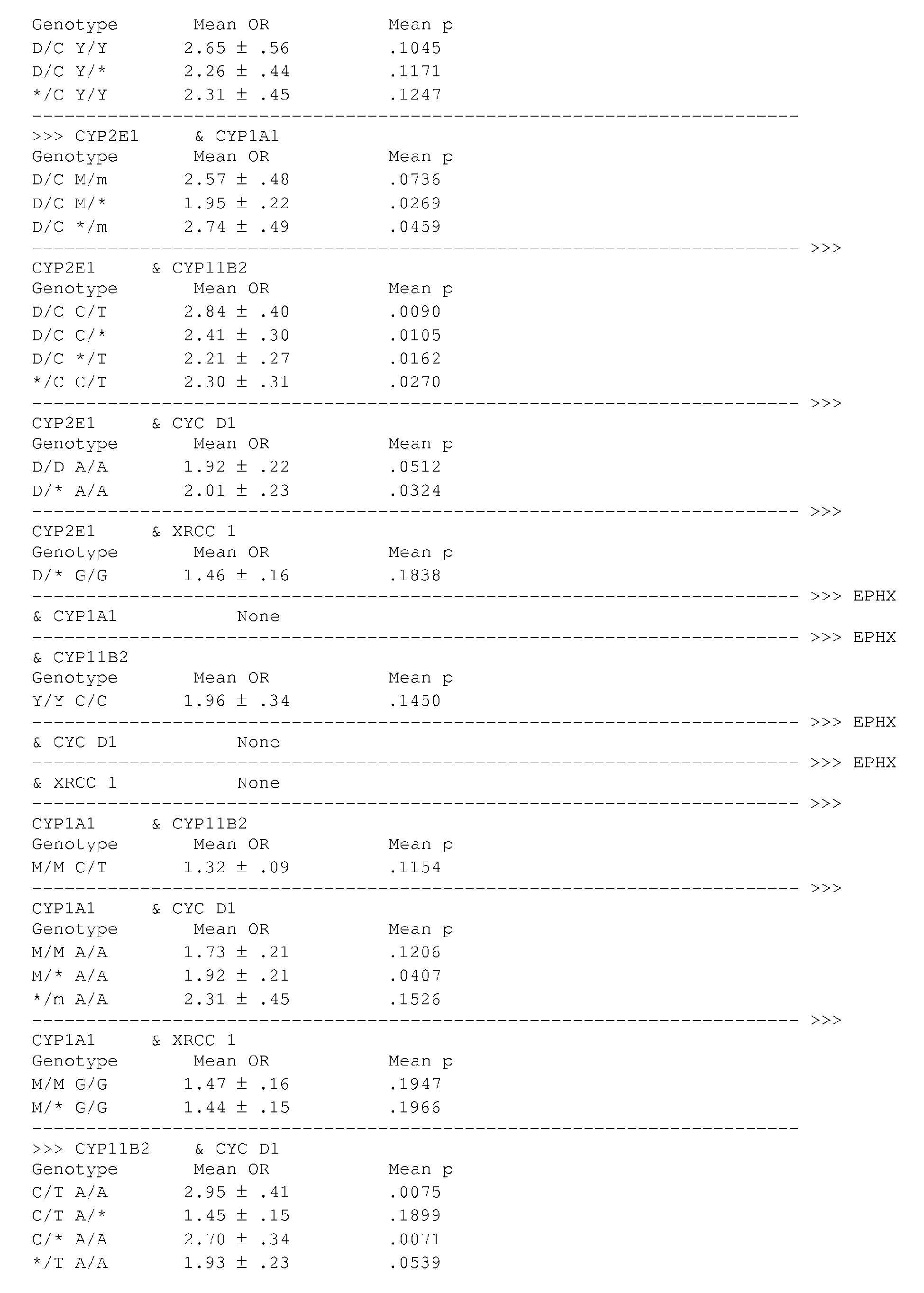

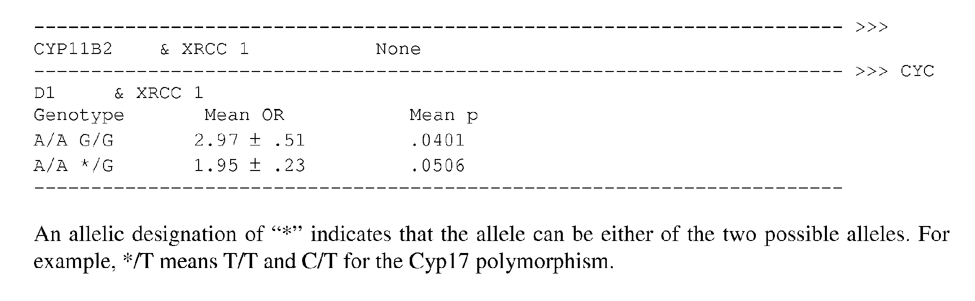

### EXAMPLE 5 - CONCLUSION

In summary, the inventors have examined genetic polymorphisms in a number of genes and have determined their association, alone and in combination, with breast cancer risk. The unexpected results of these experiments were that, considered individually, the examined genes and their polymorphisms were only modestly associated with breast cancer risk. However, when examined in combination of two, three or more, complex genotypes with wide variation in breast cancer risk were identified. This information has great utility in facilitating the most effective and most appropriate application of cancer screening and chemoprevention protocols, with resulting improvements in patient outcomes.

The following embodiments will provide further illustration of the present invention.
1. A method for assessing a female subject's risk for developing breast cancer comprising determining, in a sample from said subject, the allelic profile of two or more genes selected from the group consisting of prohibitin (PRO), progesterone receptor (PROGINS), steroid 17,20 lyase (CYP17), catechol o-methyltransferase (COMT), epidermal growth factor receptor 2 (HER2), 5α-reductase (SRD5α), glutathione S-transferase P1 (GSTP1), phenol sulphotransferase (SULF1A1), cytochrome p450-1B1 (CYP1B1), tumor suppressor p53 (p53 72), methylenetetrahydrofolate reductase (MTHFR), vitamin D receptor Apa I polymorphism (VDR/ApaI), vitamin D receptor Taq^{α}I polymorphism (VDR/TaqI), vitamin D receptor Fok I polymorphism (VDR/FokI), cytochrome P450 1A1 (CYP1A1), human aldosterone synthase or steroid 18-hydroxylase (CYP11B2), cyclin D1 (CYC D1), homo sapiens DNA repair protein (XRCC 1), human cytochrome P450IIE1 (ethanol-inducable) (CYP2E1), and microsomal epoxide hydrolase (EPHX).
2. The method of embodiment 1, wherein a gene pair selected from the group consisting of Pro and CYP17; Pro and COMT; Pro and GSTP1; Pro and SULF1A1; Pro and HER2; Pro and p53 72; Pro and CYP1B1; Pro and PROGINS; Pro and SRD5A2_2; Pro and MTHFR; Pro and VDR/ApaI; Pro and VDR/TaqI; Pro and VDR/FokI; Pro and CYP2E1; Pro and EPHX; Pro and CYP11B2; Pro and CYC D1; Pro and XRCC1; CYP17 and COMT; CYP17 and GSTP1; CYP17 and SULF1A1; CYP17 and HER2; CYP17 and p53 72; CYP17 and CYP1B1; CYP17 and PROGINS; CYP17 and SRD5A2_2; CYP17 and MTHFR; CYP17 and VDR/ApaI; CYP17 and VDR/TaqI; CYP17 and VDR/FokI; CYP17 and CYP2E1; CYP17 and EPHX; CYP17 and CYP1A1; CYP17 and CYP11B2; CYP17 and CYC D1; CYP17 and XRCC1; COMT and GSTP1; COMT and SULF1A1; COMT and HER2; COMT and p53 72; COMT and CYP1B1; COMT and PROGINS; COMT and SRD5A2_2; COMT and MTHFR; COMT and VDR/ApaI; COMT and VDR/TaqI; COMT and VDR/FokI; COMT and CYP2E1; COMT and EPHX; COMT and CYP1A1; COMT and CYP11B2; COMT and CYC D1; COMT and XRCC 1; GSTP1 and SULF1A1; GSTP1 and HER2; GSTP1 and p53 72; GSTP1 and CYP1B1; GSTP1 and PROGINS; GSTP1 and SRD5A2_2; GSTP1 and MTHFR; GSTP1 and VDR/ApaI; GSTP1 and VDR/TaqI; GSTP1 and VDR/FokI; GSTP1 and CYP2E1; GSTP1 and EPHX; GSTP1 and CYP1A1; GSTP1 and CYP11B2; GSTP1 and CYC D1; GSTP1 and XRCC1; SULF1A1 and HER2; SULF1A1 and p53 72; SULF1A1 and CYP1B1; SULF1A1 and PROGINS; SULF1A1 and SRD5A2_2; SULF1A1 and MTHFR; SULF1A1 and VDR/ApaI; SULF1A1 and VDR/TaqI; SULF1A1 and VDR/FokI; SULF1A1 and CYP2E1; SULF1A1 and EPHX; SULF1A1 and CYP1A1; SULF1A1 and CYP11B2; SULF1A1 and CYC D1; SULF1A1 and XRCC 1; HER2 and p53 72; HER2 and CYP1B1; HER2 and PROGINS; HER2 and SRD5A2_2; HER2 and MTHFR; HER2 and VDR/ApaI; HER2 and VDR/TaqI; HER2 and VDR/FokI; HER2 and CYP2E1; HER2 and EPHX; HER2 and CYP1A1; HER2 and CYP11B2; HER2 and CYC D1; HER2 and XRCC 1; p53 72 and CYP1B1; p53 72 and PROGINS; p53 72 and SRDA2_2; p53 72 and MTHFR; p53 72 and VDR/ApaI; p53 72 and VDR/TaqI; p53 72 and VDR/FokI; p53 72 and CYP2E1; p53 72 and EPHX; p53 72 and CYP1A1; p 53 72 and CYP11B2; p53 72 and CYC D1; p53 72 and XRCC1; CYP1B1 and PROGINS; CYP1B1 and SRD5A2_2; CYP1B1 and MTHFR; CYP1B1 and VDR/ApaI; CYP1B1 and VDR/TaqI; CYP1B1 and VDR/FokI; CYP1B1 and CYP2E1; CYP1B1 and EPHX; CYP1B1 and CYP1A1; CYP1B1 and CYP11B2; CYP1B1 and CYC D1; CYP1B1 and XRCC 1; PROGINS and SRD5A2_2; PROGINS and MTHFR; PROGINS and VDR/ApaI; PROGINS and VDR/TaqI; PROGINS and VDR/FokI; PROGINS and CYP2E1; PROGINS and EPHX; PROGINS and CYP1A1; PROGINS and CYP11B2; PROGINS and CYC D1; PROGINS and XRCC 1; SRD5A2_2 and MTHFR; SRD5A2_2 and VDR/ApaI; SRD5A2_2 and VDR/TaqI; SRD5A2_2 and VDR/FokI; SRD5A2_2 and CYP2E1; SRD5A2_2 and EPHX; SRD5A2_2 and CYP1A1; SRD5A2_2 and CYP11B2; SRD5A2_2 and CYC D1;SRD5A2_2 and XRCC 1; MTHFR and VDR/ApaI; MTHFR and VDR/TaqI; MTHFR and VDR/FokI; MTHFR and CYP2E1; MTHFR and EPHX; MTHFR and CYP1A1; MTHFR and CYP11B2; MTHFR and CYC D1; MTHFR and XRCC1; VDR/ApaI and VDR/TaqI; VDR/ApaI and VDR/FokI; VDR/ApaI and CYP2E1; VDR/ApaI and EPHX; VDR/ApaI and CYP1A1; VDR/ApaI and CYP11B2; VDR/ApaI and CYC D1; VDR/ApaI and XRCC 1; VDR/TaqI and VDR/FokI; VDR/TaqI and CYP2E1; VDR/TaqI and EPHX; VDR/TaqI and CYP1A1; VDR/TaqI and CYP11B2; VDR/TaqI and CYC D1; VDR/Taq1 and XRCC 1; VDR/FokI and CYP2E1; VDR/FokI and EPHX; VDR/FokI and CYP1A1; VDR/FokI and CYP11B2; VDR/FokI and CYC D1; VDR/FokI and XRCC 1; CYP2E1 and EPHX; CYP2E1 and CYP1A1; CYP2E1 and CYP11B2; CYP2E1 and CYC D1; CYP2E1 and XRCC 1; EPHX and CYP1A1; EPHX and CYP11B2; EPHX and CYC D1; EPHX and XRCC 1; CYP1A1 and CYP11B2; CYP1A1 and CYC D1; CYP1A1 and XRCC 1; CYP11B2 and CYC D1; CYP11B2 and XRCC 1; CYC D1 and XRCC 1; and p52 72 and CYC D1 is examined.
3. The method of embodiment 1, further comprising determining the allelic profile of at least a third gene.
4. The method of embodiment 1, further comprising determining the allelic profile of at least a fourth gene.
5. The method of embodiment 1, further comprising assessing one or more aspects of the subject's personal history.
6. The method of embodiment 1, wherein said one or more aspects are selected from the group consisting of age, ethnicity, reproductive history, menstruation history, use of oral contraceptives, body mass index, alcohol consumption history, smoking history, exercise history, diet, family history of breast cancer or other cancer including the age of the reltive at the time of their cancer diagnosis, and a personal history of breast cancer, breast biopsy or DCIS, LCIS, or atypical hyperplasia.
7. The method of embodiment 6, wherein one or more aspects comprises age.
8. The method of embodiment 7, wherein said subject is less than 54 years of age.
9. The method of embodiment 1, wherein determining said allelic profile is achieved by amplification of nucleic acid from said sample.
10. The method of embodiment 9, wherein amplification comprises PCR.
11. The method of embodiment 9, wherein primers for amplification are located on a chip.
12. The method of embodiment 9, wherein primers for amplification are specific for alleles of said genes.
13. The method of embodiment 9, further comprising cleaving amplified nucleic acid.
14. The method of embodiment 9, wherein said sample is derived from oral tissue or blood.
15. The method of embodiment 1, further comprising making a decision on the timing and/or frequency of cancer diagnostic testing for said subject.
16. The method of embodiment 1, further comprising making a decision on the timing and/or frequency of prophylactic cancer treatment for said subject.
17. The method of embodiment 1, wherein the alleles examined are either a C or T at base 729 (GB# U49725) for Pro, either plus or minus an Alu insert at base 956 (GB# Z49816) for PROGINS, either a T or C at base 1805 (GB# M19489) for CYP17, either a G or A at position 1947 (GB# Z26491) for COMT, either a G or A at base 77,829 (GB# AC040933) for HER2, either an 18 or 38 insert at base 2333 (GB# L03843) for SRD5α, either a G or A at base 2628 (GB# M24485) for GSTP1, either a G or A at base 4742 (GB# U54701) for SULF1A1, either a G or C at base 1294 (GB# U56438)for CYP1B1, either a G or C at base 640 (GB# AF136270) for p53, either a C or T at base 1024 (GB# AH007464) for MTHFR, either a C or T at base 46,083 (GB# AC004466) for VDR/ApaI, a polymorphism at base 46,360-46,363 (GB# AC004466) for VDR/TaqI, a polymorphism at base 12,019-12024 (GB# AC004466) for VDR/FokI, either a C or T at base 133 (GB# D12525) for CYP1A1, either a C or T at the Hae III Site (GGCC) at base 335-338 (GB# D13752) for CYP11B2, either a G or A at codon 330 in exon 4 at base 8429 (GB# AF511593) for CYC D1, either a G or A at codon 399, exon 10 at base 28152 (GB# L34079) for XRCC 1, Intron 6 Dra I polymorphism at base 10454-10459 for CYP2E1, and a Tyr/His change in exon 3 (GB# AF253417) for EPHX.
18. A nucleic acid microarray comprising nucleic acid sequences corresponding to genes for prohibitin (Pro), progesterone receptor (PROGINS), steroid 17,20 lyase (CYP17), catechol o-methyltransferase (COMT), epidermal growth factor receptor 2 (HER2), 5α-reductase (SRD5α), glutathione S-transferase P1 (GSTP1), phenol sulphotransferase (SULF1A1), cytochrome p450-1B1 (CYP1B1), tumor suppressor p53 (p53 72), methylenetetrahydrofolate reductase (MTHFR), vitamin D receptor Apa I polymorphism (VDR/ApaI), vitamin D receptor Taq^{α}I polymorphism (VDR/TaqI), vitamin D receptor Fok I polymorphism (VDR/FokI), cytochrome P450 1A1 (CYP1A1), human aldosterone synthase or steroid 18-hydroxylase (CYP11B2), cyclin D1 (CYC D1), homo sapiens DNA repair protein (XRCC 1), human cytochrome P450IIE1 (ethanol-inducable) (CYP2E1), and microsomal epoxide hydrolase (EPHX).
19. The nucleic acid microarray of embodiment 17, wherein said nucleic acid sequences comprise sequences for at least two different alleles for each of said genes.
20. A method for determining the need for routine diagnostic testing of a female subject for breast cancer comprising determining, in a sample from said subject, the allelic profile of two or more genes selected from the group consisting of prohibitin (Pro), progesterone receptor (PROGINS), steroid 17,20 lyase (CYP17), catechol o-methyltransferase (COMT), epidermal growth factor receptor 2 (HER2), 5α-reductase (SRD5α), glutathione S-transferase P1 (GSTP1), phenol sulphotransferase (SULF1A1), cytochrome p450-1B1 (CYP1B1), tumor suppressor p53 (p53 72), methylenetetrahydrofolate reductase (MTHFR), vitamin D receptor Apa I polymorphism (VDR/ApaI), vitamin D receptor Taq^{α}I polymorphism (VDR/TaqI), vitamin D receptor Fok I polymorphism (VDR/FokI), cytochrome P450 1A1 (CYP1A1), human aldosterone synthase or steroid 18-hydroxylase (CYP11B2), cyclin D1 (CYC D1), homo sapiens DNA repair protein (XRCC 1), human cytochrome P450IIE1 (ethanol-inducable) (CYP2E1), and microsomal epoxide hydrolase (EPHX).
21. A method for determining the need of a female subject for prophylactic anti-breast cancer therapy comprising determining, in a sample from said subject, the allelic profile of two or more genes selected from the group consisting of prohibitin (Pro), progesterone receptor (PROGINS), steroid 17,20 lyase (CYP17), catechol o-methyltransferase (COMT), epidermal growth factor receptor 2 (HER2), 5α-reductase (SRD5α), glutathione S-transferase P1 (GSTP1), phenol sulphotransferase (SULF1A1), cytochrome p450-1B1 (CYP1B1), tumor suppressor p53 (p53 72), methylenetetrahydrofolate reductase (MTHFR), vitamin D receptor Apa I polymorphism (VDR/ApaI), vitamin D receptor Taq^{α}I polymorphism (VDR/TaqI), vitamin D receptor Fok I polymorphism (VDR/FokI), cytochrome P450 1A1 (CYP1A1), human aldosterone synthase or steroid 18-hydroxylase (CYP11B2), cyclin D1 (CYC D1), homo sapiens DNA repair protein (XRCC 1), human cytochrome P450IIE1 (ethanol-inducable) (CYP2E1), and microsomal epoxide hydrolase (EPHX).

### IX. References

US 4,683,195, US 4,683,202, US 4,800,159, US 4,883,750, US 5,578,832, US 5,837,832, US 5,837,860, US 5,861,242, US 6,159,693, EP-A 0 329,822, PCT/US87/00880, PCT/US89/01025, WO 88/10315, WO 89/06700, and WO 90/07641
Bharaj, Scorilas, Giai, Diamandis, "TA repeat polymorphism of the 5alpha-reductase gene and breast cancer," Cancer Epidemiol Biomarkers Prev., 9:387-393, 2000.
British Patent Application GB 2,202,328
Costantino, Gail, Pee, Anderson, Redmond, Benichou, Wieand, "Validation studies for models projecting the risk of invasive and total breast cancer incidence," J Natl Cancer Inst., 91:1541-1548, 1999.
Coughtrie, Gilissen, Shek, Strange, Fryer, Jones, Bamber, "Phenol sulphotransferase SULT1A1 polymorphism: molecular diagnosis and allele frequencies in caucasian and african populations," Biochem. J., 337:45-49, 1999.
Curran, Vaughn, Lea, Weinstein, Morrison, Griffiths, "Association of a vitamin D receptor polymorphism with sporadic breast cancer development," Int. J. Cancer, 83:723-726.
Dunning, Healey, Pharoah, Teare, Ponder, Easton, "A systematic review of genetic polymorphisms and breast cancer risk," Cancer Epidemiol Biomarkers Prev., 8:843-854, 1999.
Feigelson, McKean-Cowdin, Coetzee, Stram, Kolonel, Henderson, "Building a multigenic model of breast cancer susceptibility: CYP17 and HSD17B1 are two important candidates," Cancer Res., 61:785-789, 2001.
Fodor, Weinrich, Meister, Mermod, Rutter, "A pancreatic exocrine cell factor and AP4 bind overlapping sites in the amylase 2A enhancer," Biochemistry, 30(33):8102-8108, 1991.
Frohman, In: PCR Protocols: A Guide To Methods And Applications, Academic Press, N.Y., 1990.
Hacia, Brody, Chee, Fodor, Collins, "Detection of heterozygous mutations in BRCA1 using high density oligonucleotide arrays and two-colour fluorescence analysis," Nature Genet., 14:441-449, 1996.
Hassett, Aicher, Sidhu, Omiecinski, Environmental Health, University of Washington, 1994a.
Hassett, Robinson, Beck, Omiecinski, "The human microsomal epoxide hydrolase gene (EPHX1): complete nucleotide sequence and structural characterization," Genomics, 23(2):433-442, 1994b.
Hassett, Smith, Omiecinski, Environmental Health, Univeristy of Washington, 2000.
Hirvonen, Husgafvel-Pursiainen, Anttila, Karjalainen, Vainio, "The human CYP2E1 gene and lung cancer: DraI and RsaI restriction fragment length polymorphisms in a Finnish study population," Carcinogenesis, 14(1):85-88, 1993.
Innis, Myambo, Gelfand, Brow, "DNA sequencing with Thermus aquaticus DNA polymerase and direct sequencing of polymerase chain reaction-amplified DNA," Proc Natl Acad Sci USA, 85(24):9436-9440, 1988.
Jupe, Badgett, Neas, Craft, Mitchell, Resta, Mulvihill, Aston, Thompson, "Single nucleotide polymorphism in prohibitin 3' untranslated region and breast-cancer susceptibility," Lancet., 357:1588-1589, 2001.
Kong, Wei, Amos, Lynch, Levin, Zong, Frazier, "Cyclin D1 polymorphism and increased risk of colorectal cancer at young age," J. Natl. Cancer Inst., 93:1106-1108, 2001. Kupari, Hautanen, Lankinen, Koskinen, Virolainen, Nikkila, White, Circulation, 97:565-575, 1998.
Kwoh, Davis, Whitfield, Chappelle, DiMichele, Gingeras, "Transcription-based amplification system and detection of amplified human immunodeficiency virus type 1 with a bead-based sandwich hybridization format," Proc. Nat'l Acad. Sci. USA, 86: 1173, 1989
Lander and Schork, "Genetic dissection of complex traits," Science, 30;265:2037-2048, 1994.
McTiernan, Kuniyuki, Yasui, Bowen, Burke, Culver, Anderson, Durfy, "Comparisons of two breast cancer risk estimates in women with a family history of breast cancer," Cancer Epidemiol Biomarkers Prev., 10:333-338, 2001.
Newton, Holland, Heptinstall, Hodgson, Edge, Markham, McLean, "The production of PCR products with 5' single-stranded tails using primers that incorporate novel phosphoramidite intermediates," Nucl. Acids Res. 21:1155-1162, 1993.
Ohara, Dorit, Gilbert, "One-sided polymerase chain reaction: the amplification of cDNA," Proc. Nat'l Acad. Sci. USA, 86: 5673-5677, 1989. Pease, Solas, Sullivan, Cronin, Holmes, Fodor, "Light-generated oligonucleotide arrays for rapid DNA sequence analysis," Proc. Natl. Acad. Sci. USA, 91:5022-5026, 1994.
Pierce, Sivaraman, Chang, Lum, Donlon, Seifried, Wilkens, Lau, Le Marchand, "Relationships of TP53 codon 72 and HRAS1 polymorphisms with lung cancer risk in an ethnically diverse population," Cancer Epidemiol Biomarkers Prev., 9:1199-1204, 2000.
Rasmussen, Larsen and Rasmussen, "Covalent immobilization of DNA onto polystyrene microwells: the molecules are only bound at the 5' end," Anal. Biochem, 198:138-142, 1991.
Reddy and Chow, "Safety and efficacy of antiestrogens for prevention of breast cancer," Am J Health Syst Pharm., 57:1315-2132, 2000.
Sambrook et. al., In: Molecular Cloning: A Laboratory Manual, 2d Ed., Cold Spring Harbor Laboratory Press, Cold Spring Harbor, NY, 1989.
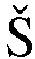 armanová, Bene 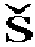 ová, Gut, Nedelcheva-Kristensen, Týnková, Soucek, "Genetic polymorphisms of biotransformation enzymes in patients with Hodkin's and non-Hodkin's lymphomas," Hum. Mol. Gen., 10(12):1265-1273, 2001.
Spurdle, Hopper, Dite, Chen, Cui, McCredie, Giles, Southey, Venter, Easton, Chenevix-Trench, "CYP17 promoter polymorphism and breast cancer in Australian women under the age forty years," J. Nat'l Cancer Inst. 92:1674-1681, 2000. Shoemaker, Lashkari, Morris, Mittmann, Davis, "Quantitative phenotypic analysis of yeast deletion mutants using a highly parallel molecular bar-coding strategy," Nature Genetics, 14:450-456, 1996.
Sjalander, Birgander, Hallmans, Cajander, Lenner, Athlin, Beckman, Beckman, "p53 polymorphisms and haplotypes in breast cancer," Carcinogenesis, 17:1313-1316, 1996.
Stem, Mason, Selhub, Choi, "Genomic DNA hypomethylation, a characteristic of most cancers, is present in peripheral leukocytes of individuals who are homozygous for the C677T polymorphism in the methylenetetrahydrofolate reductase gene," Cancer Epidemiol Biomarkers Prev., 9:849-853, 2000.
Sturgis, Castillo, Li, Zheng, Eicher, Clayman, Strom, Spitz, Wei, "Polymorphisms of DNA repair gene XRCC1 in squamous cell carcinoma of the head and neck," Carcinogenesis, 20(11):2125-2129, 1999.
Sweeney, McClure, Fares, Stone, Coles, Thompson, Korourian, Hutchins, Kadlubar, Ambrosone, "Association between survival after treatment for breast cancer and glutathione S-transferase P1 Ile105Val polymorphism," Cancer Res., 60:5621-5624, 2000.
Thompson, Shields, Freudenheim, Stone, Vena, Marshall, Graham, Laughlin, Nemoto, Kadlubar, Ambrosone, "Genetic polymorphisms in catechol-O-methyltransferase, menopausal status, and breast cancer risk," Cancer Res., 58:2107-2110, 1998.
Walker, Fraiser, Schram, Little, Nadeau, Malinowski, "Strand displacement amplification--an isothermal, in vitro DNA amplification technique," Nucleic Acids Res. 20(7):1691-1696, 1992.
Wang, Habuchi, Takahashi, Kamoto, Zuo, Mitsumori, Tsuchiya, Sato, Ogawa, Kato, Int. J. Cancer, 97:787-790, 2002.
Wang-Gohrke, Chang-Claude, Becher, Kieback, Runnebaum, "Progesterone receptor gene polymorphism is associated with decreased risk for breast cancer by age 50," Cancer Res., 60:2348-2350, 2000.
Wu and Wallace, "The ligation amplification reaction (LAR)--amplification of specific DNA sequences using sequential rounds of template-dependent ligation," Genomics, 4:560-569, 1989.
Xie, Shu, Deng, Wen, Creek, Dai, Gao, Jin, Zheng, "Population-based, case-control study of HER2 genetic polymorphism and breast cancer risk," J Natl Cancer Inst., 92:412-417, 2000.
Zheng, Xie, Cerhan, Sellers, Wen, Folsom, "Sulfotransferase 1A1 polymorphism, endogenous estrogen exposure, well-done meat intake, and breast cancer risk," Cancer Epidemiol Biomarkers Prev., 10:89-94, 2001.
Zheng, Xie, Jin, Cheng, Dai, Wen, Shu, Gao, "Genetic polymorphism of cytochrome P450-1B1 and risk of breast cancer," Cancer Epidemiol Biomarkers Prev., 9:147-150, 2000.

## Claims

1. A method for (i) assessing a female subject's risk for developing breast cancer, (ii) determining the need for routine diagnostic testing of a female subject for breast cancer, or (iii) determining the need of a female subject for prophylactic anti-breast cancer therapy, the method comprising the step of determining, in a sample from said subject, the allelic profile of the VDR-ApaI and CYP11B2 genes.

2. The method of claim 1, the method further comprising the step(s) of determining the allelic profile of at least a third and, optionally, at least a fourth gene.

3. The method of claim 2, wherein the at least third gene is the COMT or the CYP1A1 gene.

4. The method of claim 1, the method further comprising the step of assessing one or more aspects of the subject's personal history.

5. The method of claim 1, wherein the one or more aspects of the subject's personal history is/are age, ethnicity, reproductive history, menstruation history, use of oral contraceptives, body mass index, alcohol consumption history, smoking history, exercise history, diet, family history of breast cancer or other cancer including the age of the relative at the time of their cancer diagnosis, and a personal history of breast cancer, breast biopsy or DCIS, LCIS, or atypical hyperplasia.

6. The method of claim 5, wherein one or more aspects comprise(s) age.

7. The method of claim 6, wherein said subject is less than 54 years of age.

8. The method of claim 1, wherein the step of determining said allelic profile is achieved by amplification of nucleic acid from said sample.

9. The method of claim 8, wherein amplification omprises PCR or wherein the sample is derived from oral tissue or blood.

10. The method of claim 8, wherein the primers for amplification are located on a chip or are specific for alleles of said genes.

11. The method of claim 8, the method further comprising the step of cleaving amplified nucleic acid.

12. The method of claim 1, the method further comprising the step of making a decision on the timing and/or frequency of (i) cancer diagnostic testing or (ii) prophylactic cancer treatment for said subject.

13. The method of claim 3, wherein the alleles examined are
(a) either a C or T at base 46,083 (GB# AC004466) for VDR/ApaI,
(b) either a C or T at the Hae III site (GGCC) at base 335-338 (GB# D13752) for CYP11B2,
(c) either a G or A at position 1947 (GB# Z26491) for COMT, and
(d) either a T or C at base 133 (GB# D12525) for CYP1A1.

14. A nucleic acid microarray, the microarray comprising nucleic acid sequences corresponding to the genes as defined in claim 1 or 3.

15. The nucleic acid microarray of claim 14, wherein said nucleic acid sequences comprise sequences for at least two different alleles for each of said genes.
